# EUROPÄISCHE PATENTSCHRIFT

(11) **EP 2 673 267 B1**
(45) Veröffentlichungstag und Bekanntmachung des Hinweises auf die Patenterteilung: **03.06.2015**
(21) Anmeldenummer: 12700370.5
(22) Anmeldetag: 16.01.2012
(51) Int. Cl.: C07D 319/06, C07D 405/14, C07D 409/14, C09B 23/00, H01L 51/00, A61K 8/49, C09K 11/06, H05B 33/20

(54) **1,3-DIOXAN-5-ON-VERBINDUNGEN**
1,3-DIOXAN-5-ONE COMPOUNDS
COMPOSÉS DE 1,3-DIOXANE-5-ONE

(30) Priorität: 10.02.2011 DE 102011010841
(43) Veröffentlichungstag der Anmeldung: 18.12.2013
(73) Patentinhaber: Merck Patent GmbH, 64293 Darmstadt (DE)
(72) Erfinder: RUDOLPH, Thomas, 64291 Darmstadt (DE); BUEHLE, Philipp, 64673 Zwingenberg (DE); ROSSKOPF, Ralf, 64839 Muenster (DE)
(86) Internationale Anmeldenummer: PCT/EP2012/000155
(87) Internationale Veröffentlichungsnummer: WO 2012/107158

(56) Entgegenhaltungen:
- WO-A2-2007/039110
- DE-A1-102008 033 943
- US-A- 2 848 458

## Beschreibung

Die Erfindung betrifft spezielle [1,3]-Dioxan-5-on-Verbindungen, ein Verfahren zu deren Herstellung und deren Anwendung als Farbstoffe oder die Verwendung von Verbindungen nach Anspruch 1 in einer elektronischen Vorrichtung, beispielsweise als fluoreszierende Emitter für organische Elektrolumineszenzvorrichtungen (OLEDs) oder für organische lichtemittierende elektrochemische Zellen (OLECs), sowie entsprechende elektronische Vorrichtungen.

Derzeit ist zum Färben von Matrices, wie zum Beispiel Haut, Haare, Nägel oder Textilien, eine Vielzahl von Farbstoffen bekannt. Dabei können sich z.B. Direktfarbstoffe an die Matrix assoziieren und/oder kovalente chemische Bindungen zu der Matrix ausbilden. Bei anderen Färbeprozessen kann eine lösliche Vorstufe des Farbstoffes während des Färbeprozesses zum Farbstoff auf der Matrix umgesetzt werden. Des Weiteren können bei z.B. der Dispersionsfärbung schwer lösliche oder unlösliche Farbstoffe bei Behandlung der Matrix mit einer solchen Dispersion in die Matrix hinein diffundieren und ggf. eine kovalente Bindung zu der Matrix ausbilden. Das Färben der Matrix kann somit in unterschiedlicher Art und Weise stattfinden und zu einem unterschiedlichen Ergebnis hinsichtlich des Anbindungscharakters und auch des Farbergebnisses führen.

Zum Färben von Lebensmitteln oder kosmetischen Mitteln sind verschiedene Farbstoffe zugelassen, die zuletzt durch die Verordnung vom 9. August 2010 (BGBI. I S. 1146) geändert worden ist. Die Anzahl der zur Verwendung zugelassenen lipophilen Farbstoffe ist sehr begrenzt. Exemplarisch sind die beiden Farbstoffe C.I. 75300 (E100, Curcumin) und C.I. 40800 (E160a, Beta-Carotin) zu nennen. Beide Farbstoffe weisen den Mangel unbefriedigender Stabilitäten auf und können z. B. durch UV- oder sichtbares Licht, pH-Wertänderung, Wärme oder durch Oxidation zersetzt werden.

Bei Verwendung von, insbesondere synthetischen, Farbstoffen kann, insbesondere im humanen Anwendungsbereich, zudem eine geringe Verträglichkeit vorliegen.

Es besteht demzufolge weiterhin ein Bedarf an Farbstoffen, die unter anderem verträglich und insbesondere hautverträglich sind, eine hohe Substantivität auf das zu färbende Substrat haben und deren Farben sich durch eine hohe Licht-, Wärme-, pH- und Oxidationsstabilität auszeichnen. Demzufolge beschäftigt sich die vorliegende Erfindung mit dem Problem, alternative Farbstoffe mit verbesserten Eigenschaften zum Färben unterschiedlichster Substrate bereitzustellen.

Es besteht weiterhin ein Bedarf an fluoreszierenden Emittern in elektronischen Vorrichtungen.

Erfindungsgemäß werden diese Probleme durch die Gegenstände der unabhängigen Ansprüche gelöst. Vorteilhafte Ausführungsformen sind Gegenstand der abhängigen Ansprüche.

Überraschenderweise wurde gefunden, dass die [1,3]-Dioxan-5-on-Verbindungen der Formel I nach Anspruch 8, Farbstoffe mit dem gewünschten Eigenschaftsprofil sind. Weiterhin wurde gefunden, dass die Verbindungen der Formel I nach Anspruch 1, ebenfalls fluoreszierende Emitter sind, die für den Einsatz in elektronischen Vorrichtungen, insbesondere for organische Elektrolumineszenzvorrichtungen (OLEDs) oder organische lichtemittierende elektrochemische Zellen (OLECs), geeignet sind. Weiterhin wurde gefunden, dass die Verbindungen der Formel I, wie nachfolgend beschrieben, zum Schutz von Haut und Haar vor Photoalterung durch Licht eingesetzt werden können, insbesondere zum Schutz vor Photoalterung, welche durch sichtbares Licht induziert wird.

Gegenstand der Erfindung ist daher die Verwendung der Verbindungen der Formel I nach Anspruch 1, 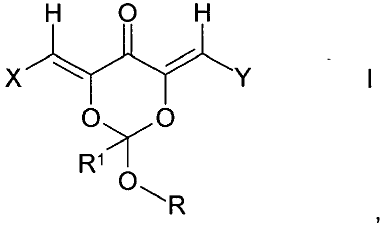 wobei
R eine geradkettige oder verzweigte Alkylgruppe mit 1 bis 20 C-Atomen bedeutet,
R¹ eine geradkettige oder verzweigte Alkylgruppe mit 1 bis 20 C-Atomen bedeutet,
X und Y jeweils unabhängig voneinander
eine unsubstituierte oder einfach- oder mehrfach durch R² substituierte Aryl- oder Heteroarylgruppe mit 5 bis 24 Ringatomen bedeuten oder eine Gruppe von unsubstituierten oder einfach oder mehrfach durch R² substituierten Aryl- und/oder Heteroarylgruppen mit 5 bis 24 Ringatomen bedeuten, wobei die Aryl- und/oder Heteroarylgruppen dieser Gruppe jeweils unabhängig voneinander einfach oder mehrfach durch eine Einfachbindung, eine Doppelbindung, konjugierte Doppelbindungen, ein C-Atom oder durch eine Einheit der Formel (CHR⁴)ₙ-(Het)ₒ-(CHR⁴)ₚ verknüpft sind,
R² jeweils unabhängig voneinander bei jedem Auftreten D, Hal, Alkyl, OH, O-Alkyl, O-Aryl, S-Alkyl, NH₂, NHAlkyl, N(Alkyl)₂, N(Aryl)₂, Cycloalkyl, O-Cycloalkyl, S-Cycloalkyl, NH-Cycloalkyl, N(Cycloalkyl)₂, CN, NO₂, Si(Alkyl)₃, B(OR³)₂, C(O)R³, P(O)(R³)₂, S(O)R³, S(O)₂R³, eine geradkettige oder verzweigte Alkenylgruppe mit 2 bis 20 C-Atomen und einer oder mehreren Doppelbindungen oder eine geradkettige oder verzweigte Alkinylgruppe mit 2 bis 20 C-Atomen und mindestens einer Dreifachbindung und gegebenenfalls einer oder mehrerer Doppelbindungen bedeutet,
R³ jeweils unabhängig voneinander H, D, OH, Alkyl, Aryl, Cycloalkyl, OAlkyl, OAryl oder O-Cycloalkyl bedeutet,
R⁴ jeweils unabhängig voneinander bei jedem Auftreten H, D, Hal, Alkyl, OH, O-Alkyl, O-Aryl, S-Alkyl, NH₂, NHAlkyl, N(Alkyl)₂, N(Aryl)₂, Cycloalkyl, O-Cycloalkyl, S-Cycloalkyl, NH-Cycloalkyl, N(Cycloalkyl)₂, CN, NO₂, Si(Alkyl)₃, B(OR³)₂, C(O)R³, C(O)₂R³, P(O)(R³)₂, S(O)R³, S(O)₂R³, eine geradkettige oder verzweigte Alkenylgruppe mit 2 bis 20 C-Atomen und einer oder mehreren Doppelbindungen oder eine geradkettige oder verzweigte Alkinylgruppe mit 2 bis 20 C-Atomen und mindestens einer Dreifachbindung und gegebenenfalls einer oder mehrerer Doppelbindungen bedeutet,
Alkyl eine geradkettige oder verzweigte Alkylgruppe mit 1 bis 20 C-Atomen bedeutet, die teilweise oder vollständig durch Halogen substituiert sein kann,
Cycloalkyl eine cyclische gesättigte oder teilweise ungesättigte Cycloalkylgruppe mit 3 bis 7 C-Atomen bedeutet,
Aryl eine Arylgruppe mit 6 bis 10 C-Atomen bedeutet, die einfach oder mehrfach durch Alkyl, OAlkyl, N(Alkyl)₂ oder Hal substiituiert sein kann,
Hal F, Cl, Br oder I bedeutet,
Het O, S, -N=N-, NH oder NR² bedeutet,
n eine ganze Zahl von 0 bis 5 bedeutet,
o 0 oder 1 bedeutet,
p eine ganze Zanl von 0 bis 5 bedeutet,
n+o+p mindestens die Zahl 1 bedeutet
und deren Salze, Tautomere, Steroisomere, einschließlich deren Mischungen in allen Verhältnissen und/oder Solvate, wobei die Verbindung 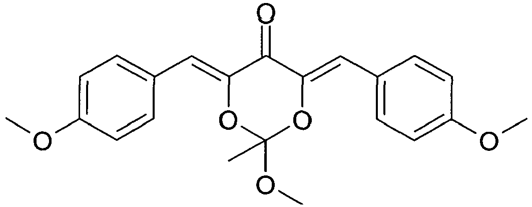 ausgeschlossen wird.

Die per Disclaimer ausgeschlossene Verbindung 2-Methoxy-4,6-bis-[1-(4-methoxy-phenyl)-meth-(Z)-yliden]-2-methyl-[1,3]dioxan-5-on ist aus WO 2007/039110 A2 bekannt. Diese Verbindung ist als Synthesezwischenstufe für die erfindungsgemäßen hautbindenden UV-Filter der WO 2007/039110 A2 beschrieben. Die Verwendung als lipophiler Farbstoff für unterschiedliche Substrate oder als fluoreszierender Emitter wird nicht beschrieben.

Aus US 2,848,458 sind 5-Oxo-1,3-dithiane als Zwischenstoffe und Farbstoffe bekannt.

Gegenstand der Erfindung sind ebenfalls die Verbindungen der Formel I nach Anspruch 8.

Die Verbindungen der Formel I sind so definiert, dass man darunter auch pharmazeutisch oder kosmetisch verwendbare Derivate, Salze, Hydrate, Solvate und Isomere(wie z.B. Stereoisomere, Diastereomere, Enantiomere, Racemate, Tautomere, E-Z-Isomere) versteht. Unter Solvaten der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate. Unter pharmazeutisch oder kosmetisch verwendbaren Derivaten versteht man z.B. die Salze der erfindungsgemäßen Verbindungen.

Eine geradkettige oder verzweigte Alkylgruppe mit 1 bis 8 C-Atomen ist beispielsweise Methyl, Ethyl, Isopropyl, Propyl, Butyl, sec-Butyl or tert-Butyl, weiterhin Pentyl, 1-, 2- or 3-Methylbutyl, 1,1-, 1,2- or 2,2-Dimethylpropyl, 1-Ethylpropyl, n-Hexyl, n-Heptyl oder n-Octyl.

Eine geradkettige oder verzweigte Alkylgruppe mit 1 bis 20 C-Atomen umfasst die zuvor beschriebene Gruppe der geradkettigen oder verzweigten Alkylgruppe mit 1 bis 8 C-Atomen und Nonanyl, Decanyl, Undecanyl, Dodecanyl, Tridecanyl, Tetradecanyl, Pentadecanyl, Hexadecanyl, Heptadecanyl, Oktadecanyl, Nonadecanyl und Eicosanyl.

Der Begriff "Alkyl" bedeutet eine geradkettige oder verzweigte Alkylgruppe mit 1 bis 20 C-Atomen, wie zuvor beschrieben, die teilweise oder vollständig durch Halogen substituiert sein kann, d.h. im Fall einer perfluorierten Alkylgruppe sind alle H-Atome dieser Alkylgruppe durch F ersetzt. Im Fall einer teilfluorierten Alkylgruppe ist mindestens ein H-Atom, jedoch nicht alle H-Atome durch ein F-Atom/F-Atome ersetzt.

Bevorzugte Beispiele für eine teilfluorierte geradkettige oder verzweigte Alkylgruppe sind CF₃-CHF-CF₂-, CF₂H-CF₂-, CF₃-CF₂-CH₂-, CF₃-CF₂-CH₂-CH₂-, oder CF₃-CF₂-CF₂-CF₂-CF₂-CF₂-CH₂-CH₂-.

Eine geradkettige oder verzweigte Perfluoralkylgruppe mit 1 bis 8 C-Atomen ist beispielsweise Trifluormethyl, Pentafluorethyl, Heptafluorpropyl, Heptafluorisopropyl, n-Nonafluorbutyl, sec.-Nonafluorbutyl, tert.-Nonafluorbutyl, Dodecafluorpentyl, 1-, 2- oder 3-Trifluormethyloctafluorbutyl, 1,1-, 1,2- oder 2,2-Bis(trifluormethyl)pentafluorpropyl, 1-Pentafluorethyl-hexafluorpropyl, n-Tridecafluorhexyl, n-Pentadecafluorheptyl oder n-Heptadecafluoroctyl. Bevorzugte Beispiele für die perfluorierte Alkylgruppe R_{f} sind Pentafluorethyl, Heptafluorpropyl, Heptafluorisopropyl, n-Nonafluorbutyl, sec.-Nonafluorbutyl oder tert.-Nonafluorbutyl.

Der Begriff "Cycloalkyl" bedeutet eine cyclische gesättigte oder teilweise ungesättigte Cycloalkylgruppe mit 3 bis 7 C-Atomen. Unsubstituierte gesättigte oder teilweise ungesättigte Cycloalkylgruppen mit 3-7 C-Atomen sind daher Cyclopropyl, Cyclobutyl, Cyclopentyl, Cyclohexyl, Cycloheptyl, Cyclopentenyl, Cyclohexenyl oder Cycloheptenyl.

Der Begriff "Hal" bedeutet F, Cl, Br oder I. Hal ist bevorzugt F, Cl oder Br.

Der Begriff "Aryl" bedeutet eine Arylgruppe mit 6 oder 10 C-Atomen, die einfach oder mehrfach durch Alkyl, O-Alkyl, N(Alkyl)₂ oder Hal substituiert sein kann, beispielsweise einfach oder mehrfach durch Alkyl, O-Alkyl, N(Alkyl)₂ oder Hal substituiertes Phenyl oder Naphthyl, wobei Alkyl und Hal eine der zuvor angegebenen Bedeutungen haben.

Der Begriff "Het" bedeutet O, S, N, -N=N-, NH oder NR², wobei R² eine Bedeutung hat, wie zuvor und nachfolgend beschrieben. Bevorzugt ist Het O, N oder NR², wobei R² Alkyl bedeutet. Besonders bevorzugt ist Het N.

Ein geradkettiges oder verzweigtes Alkenyl mit 2 bis 20 C-Atomen, wobei auch mehrere Doppelbindungen vorhanden sein können, ist beispielsweise Allyl, 2- oder 3-Butenyl, Isobutenyl, sek.-Butenyl, ferner 4-Pentenyl, iso-Pentenyl, Hexenyl, Heptenyl, Octenyl, -C₉H₁₇, -C₁₀H₁₉ bis -C₂₀H₃₉; vorzugsweise Allyl, 2- oder 3-Butenyl, Isobutenyl, sek.-Butenyl, 4-Pentenyl, iso-Pentenyl, Hexenyl oder Decenyl.

Ein geradkettiges oder verzweigtes Alkinyl mit 2 bis 20 C-Atomen, wobei auch mehrere Dreifachbindungen vorhanden sein können, ist beispielsweise Ethinyl, 1- oder 2-Propinyl, 2- oder 3-Butinyl, ferner 4-Pentinyl, 3-Pentinyl, Hexinyl, Heptinyl, Octinyl, -C₉H₁₅, -C₁₀H₁₇ bis -C₂₀H₃₇, vorzugsweise Ethinyl, 1- oder 2-Propinyl, 2- oder 3-Butinyl, 4-Pentinyl, 3-Pentinyl oder Hexinyl, wobei gegebenenfalls eine oder mehrere Doppelbindungen enthalten sein können. Bevorzugt enthält das geradkettige oder verzweigte Alkinyl mit 2 bis 20 C-Atomen eine Dreifachbindung.

In den Verbindungen der Formel I ist R eine geradkettige oder verzweigte Alkylgruppe mit 1 bis 20 C-Atomen, bevorzugt eine geradkettige oder verzweigte Alkylgruppe mit 1 bis 8 C-Atomen, vorzugsweise Methyl, Ethyl, n-Propyl, Isopropyl, n-Butyl, n-Pentyl, n-Hexyl, Ethylhexyl oder n-Octyl, ganz besonders bevorzugt Ethyl.

In den Verbindungen der Formel I ist R¹ eine geradkettige oder verzweigte Alkylgrupppe mit 1 bis 20 C-Atomen, wie zuvor beschrieben, vorzugsweise Methyl, Ethyl, n-Propyl, Isopropyl, n-Butyl, n-Pentyl, n-Hexyl, Ethylhexyl, n-Octyl, n-Decanyl, n-Dodecanyl, n-Tetradecanyl, n-Hexadecanyl, n-Octadecanyl oder n-Eicosanyl. Ganz besonders bevorzugt ist R¹ Methyl.

In den Verbindungen der Formel I stehen X und Y jeweils unabhängig voneinander für eine unsubstituierte oder einfach oder mehrfach durch R² substituierte Arylgruppe oder Heteroarylgruppe mit 5 bis 24 Ringatomen oder eine Gruppe von unsubstituierten oder einfach oder mehrfach durch R² substituierten Aryl- und/oder Heteroarylgruppen mit 5 bis 24 Ringatomen, wobei die Aryl- und/oder Heteroarylgruppen dieser Gruppe jeweils unabhängig voneinander einfach oder mehrfach durch eine Einfachbindung, eine Doppelbindung, konjugierte Doppelbindungen, ein C-Atom oder durch eine Einheit der Formel -(CHR⁴)ₙ-(Het)ₒ-(CHR⁴)ₚ-verknüpft sind, wobei R² und R⁴ eine zuvor oder nachfolgend beschriebene Bedeutung haben, n eine ganze Zahl von 0 bis 5 bedeutet, o 0 oder 1 bedeutet, p eine ganze Zahl von 0 bis 5 bedeutet und die Summe n+o+p mindestens die Zahl 1 bedeutet.

Die Arylgruppe mit 6 bis 24 Ringatomen für die Subsituenten X und/oder Y im Sinne dieser Erfindung ist eine aromatische Gruppe mit einem gemeinsamen aromatischen Elektronensystem mit 6 bis 24 C-Atomen, gegebenenfalls einfach oder mehrfach durch R² substituiert. Die Arylgruppe mit 6 bis 24 C-Atomen ist vorzugsweise durch R² substituiertes oder unsubstituiertes 1-, 2-, 3-, 4-, 5- oder 6-Phenyl, 1-, 2-, 3-, 4-, 6-, 7- oder 8-Naphthyl, 1-, 2-, 3-, 4-, 6-, 7- oder 8-Phenanthrenyl, 1-, 2-, 3-, 4-, 5-, 6-, 7-, 8-, 9- oder 10-Anthracenyl, 1-, 2-, 3-, 4-, 5-, 6-, 7-, 8-, 9-, 10-, 11- oder 12-Tetracenyl, 1-, 2-, 3-, 4-, 5-, 6-, 7-, 8-, 9-, 10-, 11- oder 12-Benzo[a]anthracenyl, 1-, 2-, 3-, 4-, 5-, 6-, 7-, 8-, 9-, 10-, 11-, 12-, 13- oder 15-Pentacenyl, 1-, 2-, 3-, 4-, 5-, 6-, 7-, 8-, 9-, 10-, 11- oder 12-Chrysenyl, 1-, 2-, 3-, 4-, 5-, 6-, 7-, 8-, 9- oder 10-Pyrenyl, 1-, 2-, 3-, 4-, 5-, 6-, 7-, 8-, 9-, , 10-, 11- oder 12-Benzo[a]pyrenyl, 1-, 2-, 3-, 4-, 5-, 6-, 7- oder 8-Azulenyl, 1-, 2-, 3-, 4-, 5-, 6-, 7-, 8-, 9- oder 10-Fluoranthenyl, 1-, 2-, 3-, 4-, 5-, 6-, 7-, 8-, 9-, 10-, 11- oder 12-Perylenyl, 1-, 2-, 3-, 4-, 5-, 6- oder 7-Indenyl oder 1-, 2-, 3-, 4-, 5-, 6-, 7-, 8- oder 9-Fluorenyl.

Die Heteroarylgruppe mit 5 bis 24 Ringatomen für die Substituenten X und/oder Y ist im Sinne dieser Erfindung ist eine heteroaromatische Gruppe mit einem gemeinsamen aromatischen Elektronensystem mit 2 bis 23 C-Atomen und insgesamt mindestens 5 aromatischen Ringatomen, gegebenenfalls einfach oder mehrfach durch R² substituiert. Die Heteroatome sind bevorzugt ausgewählt aus N, O und/oder S. Die Heteroarylgruppe mit 5 bis 24 Ringatomen ist vorzugsweise durch R² substituiertes oder unsubstituiertes 2- oder 3-Furyl, 2- oder 3-Thienyl, 1-, 2-oder 3-Pyrrolyl, 1-, 2-, 4- oder 5-Imidazolyl, 3-, 4- oder 5-Pyrazolyl, 2-, 4-oder 5-Oxazolyi, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -4- oder -5-yl, 1-oder 5-Tetrazolyl, 1,2,3-Oxadiazol-4- oder -5-yl 1,2,4-Oxadiazol-3- oder -5-yl, 1,3,4-Thiadiazol-2- oder -5-yl, 1,2,4-Thiadiazol-3- oder-5-yl, 1,2,3-Thiadiazol-4- oder -5-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-1H-Indolyl, 1-, 2-, 3-, 4-, 5-, 6- oder 7-2H-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 1-, 2-, 3-, 4-, 5-, 6-, 7- oder 8-Chinolinyl, 1-, 3-, 4-, 5-, 6-, 7- oder 8-Isochinolinyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolinyl, 2-, 4-, 5-, 6-, 7-oder 8-Chinazolinyl oder 1-, 2- oder 3-Pyrrolidinyl, 1-, 2-, 3-, 4-, 6-, 7-, 8-oder 9-Dibenzofuranyl, 1-, 2-, 3-, 4-, 6-, 7-, 8- oder 9-Dibenzothienyl, 1-, 2-, 3-, 5-, 6-, 7- oder 8-Indolizinyl, 1-, 2-, 3-, 4-, 6-, 7-, 8- oder 10-Phenanthridinyl7-1H-Indolyl, 1-, 2-, 3-, 4-, 6-, 7-, 8-, 9-, 10-, 11- oder 12-Benzophenanthridinyl oder 1-, 2-, 3-, 4-, 6-, 7-, 8-, 9-, 10-, 11- oder 12-Benzoacridinyl.

Beispiele für die unsubstituierten Grundstrukturen, die die Basis für die Gruppe von unsubstituierten Aryl- und/oder Heteroarylgruppen mit 5 bis 24 Ringatomen bilden, wie zuvor beschrieben, wobei die Aryl- und/oder Heteroarylgruppen dieser Gruppe jeweils unabhängig voneinander einfach oder mehrfach durch eine Einfachbindung, eine Doppelbindung, konjugierte Doppelbindungen, ein C-Atom oder durch eine Einheit der Formel -(CHR⁴)ₙ-(Het)ₒ-(CHR⁴)ₚ- verknüpft sind und R⁴, n, o, p und n+o+p eine zuvor oder nachfolgend beschriebene Bedeutung haben sind Biphenyl, Terphenyl, Bipyridin, 9,9'-Spirobifluoren, 9,9-Diphenylfluoren, Diphenylether, Diphenylthioether, Stilben, 1,2-Diphenylethan, 1,1-Diphenylmethan, Biphenylen, Triphenylen, die gegebenenfalls einfach oder mehrfach durch R² substituiert sein können, wie zuvor oder nachfolgend beschrieben. Bevorzugt werden die Aryl- und/oder Heteroarylgruppen durch eine Einfachbindung verknüpft.

n ist eine ganze Zahl von 0 bis 5 bedeutet, insbesondere 0, 1, 2, 3 oder 4, besonders bevorzugt 0 oder 1.

o bedeutet 0 oder 1. p bedeutet 0 bis 5, insbesondere 0 oder 1 und die Summe n+o+p bedeutet vorzugsweise die Zahl 1.

Weitere komplexere unsubstituierte Grundstrukturen für die zuvor beschriebene Gruppe von unsubstituierten Aryl- und/oder Heteroarylgruppen mit 5 bis 24 Ringatomen, wobei die Aryl- und/oder Heteroarylgruppen miteinander durch die angegebenen Alternativen verknüpft sind, wie zuvor beschrieben, können durch die folgenden Formeln dargestellt werden:

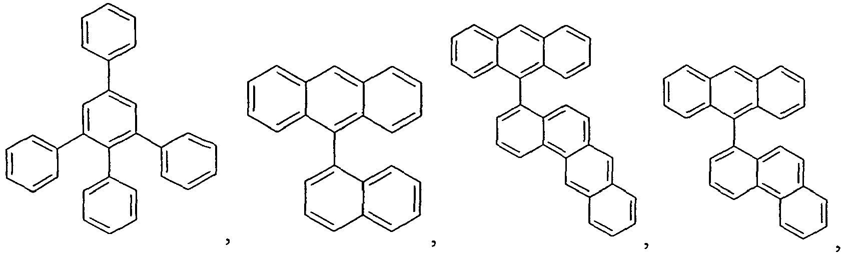

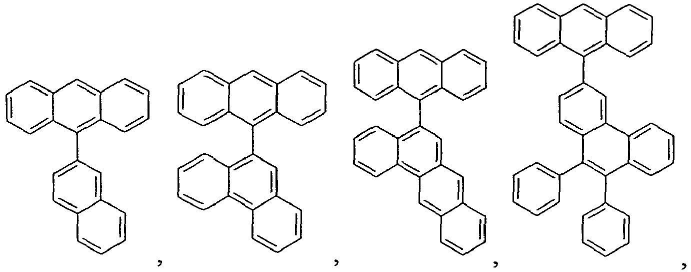

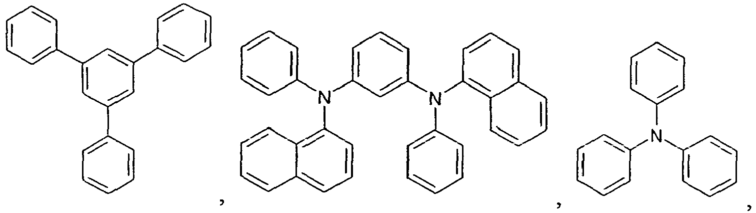

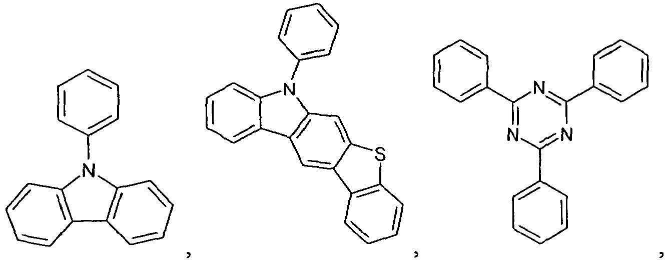

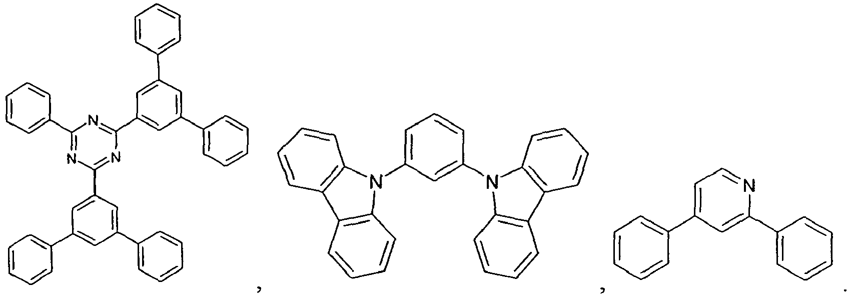

R² bedeutet jeweils unabhängig voneinander bei jedem Auftreten D, Hal, Alkyl, OH, O-Alkyl, O-Aryl, S-Alkyl, NH₂, NHAlkyl, N(Alkyl)₂, N(Aryl)₂, Cycloalkyl, O-Cycloalkyl, S-Cycloalkyl, NH-Cycloalkyl, N(Cycloalkyl)₂, CN, NO₂, Si(Alkyl)₃, B(OR³)₂, C(O)R³, P(O)(R³)₂, S(O)R³, S(O)₂R³, eine geradkettige oder verzweigte Alkenylgruppe mit 2 bis 20 C-Atomen und einer oder mehreren Doppelbindungen oder eine geradkettige oder verzweigte Alkinylgruppe mit 2 bis 20 C-Atomen und mindestens einer Dreifachbindung und gegebenenfalls einer oder mehrerer Doppelbindungen, wobei Hal, Alkyl, Aryl und Cycloalkyl eine zuvor genannte Bedeutung haben und R³ jeweils unabhängig voneinander H, D, OH, Alkyl, Aryl, Cycloalkyl, O-Alkyl, O-Aryl oder O-Cycloalkyl bedeutet.

R³ ist vorzugsweise H oder Alkyl, wobei Alkyl eine Bedeutung hat, wie zuvor beschrieben.

R² ist jeweils unabhängig voneinander bevorzugt Hal, Alkyl, O-Alkyl, O-Aryl, NHAlkyl, N(Alkyl)₂. Verbindungen der Formel I, bei denen X und/oder Y durch diese bevorzugte Gruppe von R² substituiert sind, werden vorzugsweise als Farbstoffe eingesetzt. Alkyl bei der Definition von R² ist vorzugsweise eine geradkettige oder verzweigte Alkylgruppe mit 1 bis 8 C-Atomen, die gegebenenfalls auch teilfluoriert sein kann. Besonders bevorzugte Substituenten R² sind Methyl, Isopropyl, Trifluormethyl, Methoxy, Di-(n-Butyl)amino, Dimethylamino, n-Octyloxy, Phenyloxy, -F oder -Br.

R⁴ bedeutet jeweils unabhängig voneinander bei jedem Auftreten H, D, Hal, Alkyl, OH, O-Alkyl, O-Aryl, S-Alkyl, NH₂, NHAlkyl, N(Alkyl)₂, N(Aryl)₂, Cycloalkyl, O-Cycloalkyl, S-Cycloalkyl, NH-Cycloalkyl, N(Cycloalkyl)₂, CN, NO₂, Si(Alkyl)₃, B(OR³)₂, C(O)R³, P(O)(R³)₂, S(O)R³, S(O)₂R³, eine geradkettige oder verzweigte Alkenylgruppe mit 2 bis 20 C-Atomen und einer oder mehreren Doppelbindungen oder eine geradkettige oder verzweigte Alkinylgruppe mit 2 bis 20 C-Atomen und mindestens einer Dreifachbindung und gegebenenfalls einer oder mehrerer Doppelbindungen, wobei R³, Hal, Alkyl, Aryl und Cycloalkyl eine zuvor genannte Bedeutung haben. Vorzugsweise ist R⁴ H, Hal oder Alkyl, ganz besonders bevorzugt H. Alkyl bei der Definition von R⁴ ist vorzugsweise eine geradkettige oder verzweigte Alkylgruppe mit 1 bis 8 C-Atomen, die gegebenenfalls auch teilfluoriert sein kann.

Liegt der Fokus der Anwendung im Einsatz als fluoreszierender Emitter, so werden für X und Y jeweils unabhängig voneinander bevorzugt eine Gruppe von unsubstituierten oder einfach oder mehrfach durch R² substituierten Aryl- und/oder Heteroarylgruppen mit 5 bis 24 Ringatomen, wobei die Aryl- und/oder Heteroarylgruppen dieser Gruppe jeweils unabhängig voneinander einfach oder mehrfach durch eine Einfachbindung oder ein O-Atom verknüpft sind, ausgewählt, wobei R² Alkyl bedeutet und Alkyl vorzugsweise eine geradkettige oder verzweigte Alkylgruppe mit 1 bis 4 C-Atomen bedeutet, insbesondere Biphenyl, Terphenyl oder die Gruppen der Formel

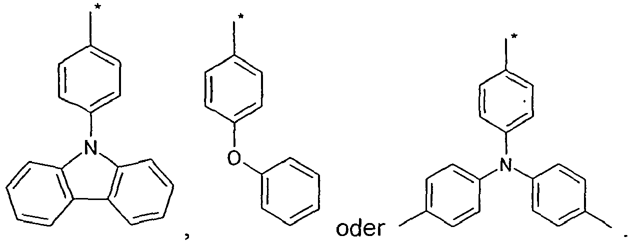

Bevorzugt ist der Substituent R¹ eine geradkettige oder verzweigte Alkylgruppe mit 1 bis 4 C-Atomen.

Bevorzugt ist der Substituent R eine geradkettige oder verzweigte Alkylgruppe mit 1 bis 4 C-Atomen.

Bevorzugt sind X und Y jeweils unabhängig voneinander eine unsubstituierte oder einfach oder mehrfach durch R² substituierte Aryl- oder Heteroarylgruppe mit 5 bis 18 Ringatomen.

Liegt der Fokus der Anwendung im Einsatz als Farbstoff oder zum Schutz von Haut und Haar vor Photoalterung, insbesondere in der weiteren Verwendung als Bestandteil von kosmetischen, pharmazeutischen, dermatologischen Zubereitungen oder Haushaltsprodukten, so werden für X und Y jeweils unabhängig voneinander bevorzugt unsubstituierte oder einfach oder mehrfach durch R² substituierte Aryl- oder Heteroarylgruppen, aus der Gruppe Phenyl, Naphthyl, Anthracenyl, Indolyl, 9-Carbazol-4-yl, Azulenyl, Fluorenyl, Thienyl, Chinolinyl, Dibenzopyrrolyl, ausgewählt.

Es werden besonders bevorzugt unsubstituierte oder einfach oder mehrfach durch R² substituierte Aryl- oder Heteroarylgruppen aus der Gruppe Phenyl, Naphthyl, Azulenyl, Indolyl oder Thienyl zum Einsatz als Farbstoff oder zum Schutz von Haut und Haar vor Photoalterung eingesetzt.

Verbindungen der Formel I als Farbstoffe sind besonders bevorzugt, die an ihrem langwelligen Absorptionsmaximum Licht einer Wellenlänge von größer 410 nm, besonders bevorzugt von größer 450nm und ganz besonders bevorzugt von größer 490 nm absorbieren.

Bei den Verbindungen der Formel I ist es besonders bevorzugt, wenn X und Y gleich sind.

Bevorzugte Einzelverbindungen der Formel I sowie deren Doppelbindungsisomere und Photoisomere oder ganz besonders bevorzugte Einzelverbindungen der Formel I sind
(4Z,6Z)-2-Ethoxy-4,6-bis[(4-methoxyphenyl)methylen]-2-methyl-1,3-dioxan-5-on,
(4Z,6Z)-4,6-Bis[(3,4-dimethoxyphenyl)methylen]-2-ethoxy-2-methyl-1,3-dioxan-5-on,
(4Z,6Z)-4,6-Bis[(2,4-dimethoxyphenyl)methylen]-2-ethoxy-2-methyl-1,3-dioxan-5-on,
(4Z,6Z)-2-Ethoxy-2-methyl-4,6-bis[(2,4,5-trimethoxyphenyl)methylen]-1,3-dioxan-5-on,
(4Z,6Z)-2-Ethoxy-2-methyl-4,6-bis[(3,4,5-trimethoxyphenyl)methylen]-1,3-dioxan-5-on,
(4Z,6Z)-2-Ethoxy-2-methyl-4,6-bis[(4-octoxyphenyl)methylen]-1,3-dioxan-5-on,
(4Z,6Z)-2-Ethoxy-2-methyl-4,6-bis[(4-phenoxyphenyl)methylen]-1,3-dioxan-5-on,
(4Z,6Z)-4,6-Bis[[4-(dibutylamino)phenyl]methylen]-2-ethoxy-2-methyl-1,3-dioxan-5-on,
2-Ethoxy-4,6-bis-[1-(4-fluoro-phenyl)-meth-(Z)-yliden]-2-methyl-[1,3]dioxan-5-on,2-Ethoxy-4,6-bis-[1-(4-trifluoromethyl-phenyl)-meth-(Z)-yliden]-2-methyl-[1,3] dioxan-5-on,
2-Ethoxy-2-methyl-4,6-bis-[1-(2,4,6-trimethoxy-phenyl)-meth-(Z)-yliden]-[1,3]dioxan-5-on,
2-Ethoxy-2-methyl-4,6-bis-[1-(2,3,4-trimethoxy-phenyl)-meth-(Z)-yliden]-[1,3]dioxan-5-on,
4,6-Bis-[1-biphenyl-4-yl-meth-(Z)-yliden]-2-ethoxy-2-methyl-[1,3]dioxan-5-on,
2-Ethoxy-2-methyl-4,6-bis-[1-naphthalin-2-yl-meth-(Z)-yliden]-[1,3]dioxan-5-on,
2-Ethoxy-2-methyl-4,6-bis-[1-(1-methyl-1H-indol-3-yl)-meth-(Z)-yliden]-[1,3]dioxan-5-on,
2-Ethoxy-4,6-bis-[1-(9-ethyl-9H-carbazol-3-yl)-meth-(Z)-yliden]-2-methyl-[1,3]dioxan-5-on,
4,6-Bis-[1-(4-dimethylamino-2-methoxy-phenyl)-meth-(Z)-yliden]-2-ethoxy-2-methyl-[1,3]dioxan-5-on,
4,6-Bis-[1-(4-dimethylamino-naphthalin-1-yl)-meth-(Z)-yliden]-2-ethoxy-2-methyl-[1,3]dioxan-5-on,
2-Ethoxy-4,6-bis-[1-(4-bromphenyl)-meth-(Z)-yliden]-2-methyl-[1,3]dioxan-5-on,
2-Ethoxy-2-methyl-4,6-bis-[1-thiophen-2-yl-meth-(Z)-yliden]-[1,3]dioxan-5-on,
4-[1-(4-Dibutylamino-phenyl)-meth-(Z)-ylliden]-2-ethoxy-6-[1-(4-methoxy-phenyl)-meth-(Z)-yliden]-2-methyl-[1,3]dioxan-5-on,
2-Ethoxy-4,6-bis-[1-(5-isopropyl-3,8-dimethyl-azulen-1-yl)-meth-(Z)-yliden]-2-methyl-[1,3]dioxan-5-on,
4-,6-Bis-[1-(4-Carbazol-9-yl-phenyl)-meth-(Z)-yliden]-2-ethoxy-2-methyl-[1,3]dioxan-5-on,
2-Ethoxy-2-methyl-4,6-bis-[1-[1,1';3',1"]terphenyl-2'-yl-meth-(Z)-yliden]-[1,3]dioxan-5-on,
4,6-Bis-[1-[3-(di-p-tolyl-amino)-phenyl]-meth-(Z)-yliden]-2-methoxy-2-methyl-[1,3]dioxan-5-on.

Die Verbindungen der Formel I, wie zuvor beschrieben oder als bevorzugt beschrieben, weisen sehr gute Löslichkeiten und Dispergierbarkeiten auf, insbesondere in lipophileren, nicht-wässrigen Lösungsmitteln und Lösungsmittelgemischen. Die Farbstoffe der Formel I sind daher lipophil. Die Farben zeichnen sich durch eine hohe Lichtechtheit, Wärme- und pH-Stabilität aus, sowie durch hohe Farbintensitäten und intensive Fluoreszenzeigenschaften. Die Verbindungen der Formel I sind selbst ebenfalls lichtecht und thermostabil. Ein weiterer Vorteil der Verbindungen der Formel I ist ihre hohe Substantivität zu Oberflächen, insbesondere zu keratinhaltigen Oberflächen wie Haut, Haar oder Nägel. Beispiele für weitere färbbare Oberflächen bzw. Substrate umfassen Papier, Baumwolle, Wolle, Kunststoffe beispielsweise auf Basis von Polyethylen, Polypropylen, Polyurethan, Polyamid, Cellulose oder Glas, wobei der Farbstoff entweder bei der Substratherstellung zugesetzt oder das Substrat nachträglich gefärbt werden kann.

Ein weiterer Gegenstand der Erfindung ist daher die Verwendung der Verbindungen der Formel I nach Anspruch 8 als Farbstoff.

Die Lipophilie der Verbindungen der Formel I kann variiert werden, indem weitere Substituenten R² eingeführt werden, die hydrophil sind, beispielsweise COOH-Gruppen, SO₃H-Gruppen oder deren korrespondierenden salzbildenden Gruppen, beispielsweise -COOKt, -SO₃Kt, wobei das Kation Kt vorzugsweise ein Ammoniumion oder ein Alkalimetall- oder Erdalkalimetallkation wie Na⁺, K⁺, Mg²⁺ oder Ca²⁺ ist.

Die Farbstoffe eignen sich insbesondere zur Färbung von Haut, Haaren oder zur Färbung von kosmetischen, pharmazeutischen oder dermatologischen Zubereitungen oder von Haushaltsprodukten.

Eine weitere bevorzugte Verwendung der Verbindungen der Formel I ist der Schutz von Haut und Haar vor Photoalterung durch sichtbares Licht. Die wissenschaftliche Erkenntnis hierzu ist beispielsweise in Zastrow et al, Skin Pharmacol. Physiol 2009, 22, 31-44 beschrieben. Aus diesem Grund ist es besonders bevorzugt, in Zubereitungen mit bekannten UVB- und UVA-Filtern zu kombinieren, um ein Breitbandschutzsystem zu generieren, daß im Idealfall den gesamten UV- und Vis-Bereich abdecken kann.

Gegenstand der Erfindung ist auch ein Verfahren nach Anspruch 9 zur Herstellung der Verbindungen der Formel I nach Anspruch 8, 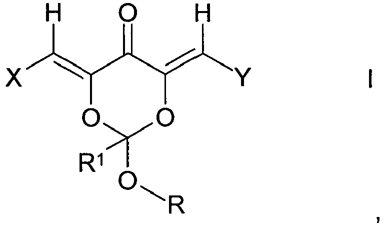 wobei
R eine geradkettige oder verzweigte Alkylgruppe mit 1 bis 20 C-Atomen bedeutet,
R¹ eine geradkettige oder verzweigte Alkylgruppe mit 1 bis 20 C-Atomen bedeutet,
X und Y jeweils unabhängig voneinander eine unsubstituierte oder einfach- oder mehrfach durch R² substituierte Heteroarylgruppe mit 5 bis 24 Ringatomen bedeuten, wie in Anspruch 8 beschrieben oder
eine Gruppe von unsubstituierten oder einfach oder mehrfach durch R² substituierten Aryl- und/oder Heteroarylgruppen mit 5 bis 24 Ringatomen bedeuten, wie in Anspruch 8 beschrieben,
R² jeweils unabhängig voneinander bei jedem Auftreten D, Hal, Alkyl, OH, O-Alkyl, O-Aryl, S-Alkyl, NH₂, NHAlkyl, N(Alkyl)₂, N(Aryl)₂, Cycloalkyl, O-Cycloalkyl, S-Cycloalkyl, NH-Cycloalkyl, N(Cycloalkyl)₂, CN, NO₂, Si(Alkyl)₃, B(OR³)₂, C(O)R³, P(O)(R³)₂, S(O)R³, S(O)₂R³, eine geradkettige oder verzweigte Alkenylgruppe mit 2 bis 20 C-Atomen und einer oder mehreren Doppelbindungen oder eine geradkettige oder verzweigte Alkinylgruppe mit 2 bis 20 C-Atomen und mindestens einer Dreifachbindung und gegebenenfalls einer oder mehrerer Doppelbindungen bedeutet,
R³ jeweils unabhängig voneinander H, D, OH, Alkyl, Aryl, Cycloalkyl, OAlkyl, OAryl oder O-Cycloalkyl bedeutet,
Alkyl eine geradkettige oder verzweigte Alkylgruppe mit 1 bis 20 C-Atomen bedeutet, die teilweise oder vollständig durch Halogen substituiert sein kann,
Cycloalkyl eine cyclische gesättigte oder teilweise ungesättigte Cycloalkylgruppe mit 3 bis 7 C-Atomen bedeutet,
Aryl eine Arylgruppe mit 6 bis 10 C-Atomen bedeutet, die einfach oder mehrfach durch Alkyl, OAlkyl, N(Alkyl)₂ oder Hal substiituiert sein kann,
Hal F, Cl, Br oder I bedeutet,
dadurch gekennzeichnet, dass eine Verbindung der Formel II 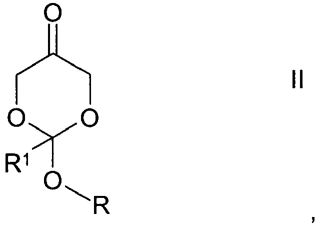
wobei R und R¹ eine zuvor genannte oder bevorzugt genannte Bedeutung haben, mit einer Verbindung der Formel IIIa und/oder IIIb 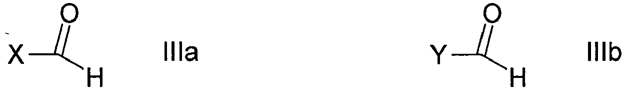
umgesetzt wird, wobei X und Y eine zuvor genannte oder bevorzugt genannte Bedeutung haben.

Die genannte Umsetzung der Verbindungen der Formel II mit mindestens einer Verbindung der Formel IIIa oder IIIb wird in der Regel nach Bedingungen der Michael-Addition durchgeführt, die dem Fachmann auf dem Gebiet der Synthesechemie bekannt ist. Die Umsetzung erfordert in der Regel die Anwesenheit einer starken Base, beispielsweise Alkalimetallhydroxide wie Natriumhydroxid oder Kaliumhydroxid oder starke organische Basen wie Lithiumdiisopropylamid. Bevorzugt werden Alkalimetallhydroxide verwendet. Der mindestens eine Aldehyd der Formel IIIa oder IIIb wird in der Regel im Überschuss, jedoch mindestens mit einem Äquivalenten in Bezug auf die Verbindung der Formel II eingesetzt. Will man unsymmetrische Verbindungen der Formel I herstellen, so wird eine Michung aus 2 Aldehyden der Formel IIIa und/oder IIIb zugesetzt. Unterscheiden sich die Reaktionskinetiken beider Aldehyde stark, können die entsprechenden Aldehyde der Formeln IIIa und/oder IIIb entsprechend ihrere Kinetik einzeln zudosiert werden.

Eine Auftrennung der eventuell entstehenden Mischungen an Verbindungen der Formel I zu isolierten Verbindungen der Formel I ist mit den herkömmlichen Methoden möglich.

Das oben genannte Verfahren wird vorzugsweise bei Temperaturen zwischen 0°C und 150°C, besonders bevorzugt bei Siedetemperatur des verwendeten Lösungsmittels durchgeführt. Geeignete Lösungsmittel für die genannte Umsetzung sind Alkohole, wie beispielsweise Methanol, Ethanol, Butanol und andere organische Lösungsmittel wie Dioxan, tert-Butylmethylether, Dichlormethan, Chloroform und Toluol. Bevorzugt findet die Umsetzung in Ethanol statt.

Die Verbindungen der Formel II, wie zuvor beschrieben, sind käuflich zu erhalten oder können auf Basis der Veröffentlichung in JACS, 1997, 119, 2795-2803 hergestellt werden. Dabei wird in der Regel Dihydroxyaceton mit Camphersulfonsäure als Katalysator und einem entsprechenden Acetat, beispielsweise der Formel CH₃-C(OR)₃, wobei R eine der zuvor genannten Bedeutungen hat, beispielsweise Trimethoxyorthoacetat, umgesetzt. Im Falle der Umsetzung mit Trimethoxyorthoacetat gemäß dem nachfolgenden Reaktionsschema entsteht 2-Methyl-2-methoxy-1,3-dioxan-5-on.

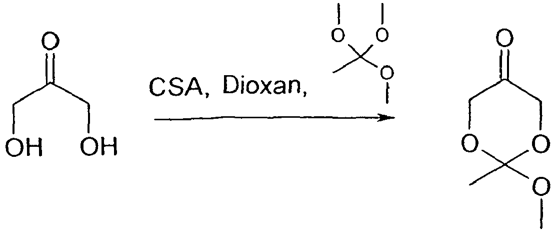

Durch die Verwendung anderer Orthoester können auch andere Verbindungen der Formel II synthetisiert werden. Der beispielsweise kommerziell erhältliche Orthovaleriansäure-trimethylester kann mit Dihydroxyaceton umgesetzt werden und man erhält eine Verbindung der Formel II, wobei R¹ = C₄H₉ und R = Methyl bedeutet. Nicht kommerziell erhältliche Orthoester können aus ihren enstprechenden Amiden oder Cyaniden durch Umwandlung in die entsprechenden Imidoester und anschließender Alkoholyse in die Orthoester überführt werden.

Die Aldehyde der Formel IIIa oder IIIb sind in der Regel kommerziell erhältlich oder können nach bekannten Methoden hergestellt werden, beispielsweise durch Reaktion einer entsprechenden Grignard-Verbindung mit Ethylformiat (HCO₂Et).

Die Aufreinigung der Verbindungen der Formel I, hergestellt nach dem zuvor geschilderten Verfahren kann nach den unterschiedlichsten Aufreinigungsmethoden erfolgen, die dem Fachmann hinlänglich bekannt sind, beispielsweise durch Chromatographie oder Umkristallisation.

Die Umwandlung in Salze der Verbindungen der Formel I gelingt beispielsweise durch Zusatz eines Alkali- oder Erdalkalimetall-hydroxids, - carbonats oder -bicarbonats in einem polaren Lösungsmittel, beispielsweise in Ethanol, Methanol oder Isopropanol, sofern die Verbindungen der Formel I Substituenten R² tragen, die in ein Salz umgewandelt werden können, beispielsweise COOH- oder SO₃H-Gruppen. Die oben beschriebenen erfindungsgemäßen Verbindungen der Formel I, die insbesondere Substituenten R² ausgewählt aus der Gruppe Hal oder B(OR₃)₂ tragen, können beispielsweise als Comonomere zur Erzeugung entsprechender konjugierter, teilkonjugierter oder nicht-konjugierter Polymere, Oligomere oder auch als Kern von Dendrimeren Verwendung finden. Die Polymerisation erfolgt dabei bevorzugt über die Halogenfunktionalität. Für diese weitere Umwandlung bevorzugte Subsituenten R² sind Cl, Br, I, B(OH)₂ oder entsprechende Borsäureester B(OAlkyl)₂, wobei Alkyl vorzugsweise eine geradkettige oder verzweigte Alkylgruppe mit 1 bis 4 C-Atomen bedeutet, ganz besonders bevorzugt B(OMethyl)₂.

Weiterer Gegenstand der Erfindung sind somit konjugierte, teilkonjugierte und nicht-konjugierte Polymere, Oligomere oder Dendrimere enthaltend eine oder mehrere Verbindungen gemäß Formel I nach Anspruch 8, wobei die Verknüpfungsstelle zwischen der mindestens einen Verbindung der Formel I und dem Polymer, Oligomer oder Dendrimer an der Position liegt, an der sich vor der Umsetzung der mindestens eine Rest R² der Verbindung der Formel I befunden hat.

Diese Polymere können weitere Wiederholeinheiten enthalten. Diese weiteren Wiederholeinheiten sind vorzugsweise ausgewählt aus der Gruppe, bestehend aus Fluorenen (z. B. gemäß EP 842208 oder WO 00/22026), Spirobifluorenen (z. B. gemäß EP 707020, EP 894107 oder EP 04028865.6), Triarylaminen, Para-phenylenen (z. B. gemäß WO 92/18552), Carbazolen (z. B. gemäß WO 04/070772 und WO 04/113468), Thiophenen (z. B. gemäß EP 1028136), Dihydrophenanthrenen (z. B. gemäß WO 05/014689), Indenofluorenen (z. B. gemäß WO 04/041901 und WO 04/113412), aromatischen Ketonen (z. B. gemäß WO 05/040302), Phenanthrenen (z. B. gemäß WO 05/104264) und/oder Metallkomplexen, insbesondere ortho-metallierten Iridiumkomplexen. Dabei sei ausdrücklich darauf hingewiesen, dass die Polymere auch mehrere verschiedene Wiederholeinheiten aufweisen können, welche aus einer oder mehreren der oben genannten Gruppen ausgewählt sind.

Ein weiterer Gegenstand der vorliegenden Erfindung ist ist eine Zubereitung, enthaltend zumindest eine Verbindung der Formel I nach Anspruch 8.

Im Sinne der vorliegenden Erfindung wird neben dem Begriff Zubereitung gleichbedeutend auch der Begriff Mittel oder Formulierung verwendet.

Die Zubereitung kann die genannten notwendigen oder optionalen Bestandteile umfassen oder enthalten, daraus im wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

Die Zubereitung kann dabei zusätzlich zu der mindestens einen Verbindung der Formel I nach Anspruch 8 einen für kosmetische, pharmazeutische, dermatologische Zubereitungen oder Haushaltsprodukte geeigneten Träger enthalten. Geeignete Trägermaterialien werden nachfolgend beschrieben.

Gegenstand der Beschreibung ist auch ein Verfahren zur Herstellung einer solchen Zubereitung, dadurch gekennzeichnet, dass die zumindest eine Verbindung der Formel I mit zumindest einem für kosmetische, pharmazeutische, dermatologische Zubereitungen oder Haushaltsprodukte geeigneten Träger und gegebenenfalls Hilfsstoffen und/oder Füllstoffen gemischt, insbesondere dispergiert und/oder emulgiert und/oder gelöst wird.

Geeignete Hilfsstoffe oder Füllstoffe werden nachfolgend beschrieben.

Die Verbindungen der Formel I sind Farbstoffe, die sich zur Färbung der Haut oder der Haare eignen und können daher auch Bestandteil von Färbemitteln sein.

Die Verbindungen der Formel I, wie zuvor beschrieben oder als bevorzugt beschrieben, können in einer bevorzugten Anwendung in Mitteln zum Färben von keratinhaltigen Fasern, insbesondere zum Färben menschlicher Haare, eingesetzt werden, die beispielsweise ausgewählt werden aus einem Farbfestiger, einer Farbfönlotion, einem Farbfönschaum, einer Farbspülung, einem Farbgel oder einer Farbcreme. Sie können jedoch auch in Mitteln zur permanenten Haarfärbung, beispielsweise in Mehrkomponentensystemen enthalten sein.

Unter keratinhaltigen Fasern sind vorzugsweise menschliche Haare, Wolle, Pelze oder Federn zu verstehen. Die erfindungsgemäßen Verbindungen eignen sich prinzipiell aber auch zum Färben anderer Naturfasern, wie beispielsweise Baumwolle, Jute, Sisal, Leinen oder Seide, oder zum Färben modifizierter Naturfasern, wie beispielsweise Regeneratcellulose, Nitro-, Alkyl- oder Hydroxyalkyl- oder Acetylcellulose. Besonders bevorzugt ist die keratinhaltige Faser menschliches Haar.

Die entsprechenden Mittel zum Färben der keratinhaltigen Fasern, wie zuvor beschrieben, enthalten die Verbindung(en) der Formel I vorzugsweise in Mengen oberhalb von 0,01 Gew.-% und unterhalb von 10 Gew.-%, jeweils bezogen auf das gesamte Mittel. Bevorzugte Mittel zum Färben keratinhaltiger Fasern sind dadurch gekennzeichnet, dass sie die Verbindung(en) der Formel I in Mengen von 0,05 bis 5 Gew.-%, vorzugsweise von 0,1 bis 2,5 Gew.-%, besonders bevorzugt von 0,25 bis 1,5 Gew.-% und insbesondere von 0,4 bis 1 Gew.-%, jeweils bezogen auf das gesamte Mittel enthalten.

Die entsprechenden Mittel enthaltend mindestens eine Verbindung der Formel I dienen der Farbveränderung keratinhaltiger Fasern, wie zuvor beschrieben, insbesondere menschlicher Haare. Die Farbveränderung kann allein aufgrund der Verbindung(en) der Formel I erfolgen, die Mittel können aber auch zusätzlich weitere farbverändernde Substanzen enthalten, beispielsweise weitere direktziehende Farbstoffe und/oder Oxidationsfärbemittel.

Das Mittel zum Färben der keratinhaltigen Fasern enthaltend mindestens eine Verbindung der Formel I, wie zuvor beschrieben, kann als Einkomponentenmittel, als Zweikomponentenmittel oder als Dreikomponentenmittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind. Das einzusetzende Mittel wird bei solchen Systemen vom Verbraucher direkt vor der Anwendung durch Vermischen der Komponenten hergestellt.

Ein weiterer Gegenstand der Beschreibung ist ein Verfahren zum Färben von keratinhaltigen Fasern, worin ein Mittel zum Färben von keratinhaltigen Fasern enthaltend mindestens eine Verbindung der Formel I, wie zuvor beschrieben oder als bevorzugt beschrieben, mindestens einmal täglich oder mindestens zwei Mal oder mehrere Male hintereinander auf die keratinhaltige Faser aufgebracht, einige Zeit, üblicherweise ca. 20 bis 45 Minuten, auf der Faser belassen, und anschließend wieder ausgespült oder mit einem Shampoo ausgewaschen wird.

Es ist jedoch auch möglich, eine Vorbehandlung der keratinhaltigen Fasern durchzuführen und dann das Mittel enthaltend die mindestens eine Verbindung der Formel I aufzutragen.

Des Weiteren können, um z.B. weitere Farbanpassungen vornehmen zu können, die Mittel enthaltend die mindestens eine Verbindung der Formel I weitere Oxidationsfarbstoffkomponenten enthalten.

Kupplerkomponenten erlauben in der Regel mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Diese Gruppen stehen durch ein Doppelbindungssystem in Konjugation. Wenn die zyklische Verbindung ein Sechsring ist, so befinden sich die besagten Gruppen bevorzugt in ortho-Position oder meta-Position zueinander.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem MolVerhältnis von 1:0,5 bis 1:3, insbesondere 1:1 bis 1:2, stehen können.

Geeignete Oxidationsfarbstoffkomponenten vom Entwicklertyp sind p-Phenylendiamin und dessen Derivate. Geeignete p-Phenylendiamine werden ausgewählt aus einer oder mehrerer Verbindungen der Gruppe, die gebildet wird aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)-amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Weitere geeignete p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie den physiologisch verträglichen Salzen dieser Verbindungen.

Als weitere geeignete Entwicklerkomponenten können Verbindungen eingesetzt werden, die mindestens zwei aromatische Kerne enthalten, die mit Amino- und/oder Hydroxylgruppen substituiert sind. Weitere geeignete Entwicklerkomponenten werden insbesondere ausgewählt aus mindestens einer Verbindung der Gruppe, gebildet aus N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)ethylendiamin, N,N'-Bis-(4'-aminophenyl)tetra-methylendiamin, N,N'-Bis-(2-hydroxyethyl)-N,N'-bis-(4'-aminophenyl)-tetramethylendiamin, N,N'-Bis-(4-(methylamino)phenyl)tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Weitere geeignete zweikernige Entwicklerkomponenten werden ausgewählt aus N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es möglich sein, als Entwicklerkomponente ein p-Aminophenolderivat oder eines seiner physiologisch verträglichen Salze einzusetzen. Bevorzugte p-Aminophenole sind p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze. Besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol. Weiterhin kann die Entwicklerkomponente ausgewählt sein aus heterocyclischen Entwicklerkomponenten, wie beispielsweise aus Pyrimidin-Derivaten, Pyrazol-Derivaten, Pyrazolopyrimidin-Derivaten bzw. ihren physiologisch verträglichen Salzen. Bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Weitere geeignete Pyrazol-Derivate sind die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1 -t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-l-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze, insbesondere jedoch 4,5-Diamino-1-(2-hydro-xyethyl)pyrazol. Als Pyrazolopyrimidine sind insbesondere Pyrazolo[1,5-a]pyrimidine geeignet, wobei bevorzugte Pyrazolo[1,5-a]pyrimidine ausgewählt sind aus Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethylpyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)-(2-hydroxyethyl)amino]ethanol, 2-[(7-Aminopyrazolo[1,5-a]pyrimidin-3-yl)-(2-hydroxyethyl)amino]ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihre physiologisch verträglichen Salze und ihre tautomeren Formen.

Weitere geeignete Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,M-Bis-(2-hydroxy-ethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Weitere geeignete Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)pyrazol sowie deren physiologisch verträglichen Salze.

Die Entwicklerkomponenten werden bevorzugt in einer Menge von 0,0001 bis 10 Gew.-%, vorzugsweise 0,001 bis 5 Gew.-%, jeweils bezogen auf das gesamte Färbemittel, verwendet.

Geeignete Oxidationsfarbstoffkomponenten vom Kupplertyp werden bevorzugt ausgewählt aus m-Aminophenol und/oder dessen Derivaten, m-Diaminobenzol und/oder dessen Derivaten, o-Diaminobenzol und/oder dessen Derivaten, o-Aminophenol und/oder dessen Derivaten, Naphthalinderivaten mit mindestens einer Hydroxygruppe, Dibeziehungsweise Trihydroxybenzol und/oder deren Derivaten, Pyridinderivaten, Pyrimidinderivaten, Monohydroxyindol-Derivaten und/oder Monoaminoindol-Derivaten, Monohydroxyindolin-Derivaten und/oder Monoaminoindolin-Derivaten, Pyrazolonderivaten, wie beispielsweise 1-Phenyl-3-methylpyrazol-5-on, Morpholinderivaten, wie beispielsweise 6-Hydroxybenzomorpholin oder 6-Aminobenzomorpholin, Chinoxalinderivaten, wie beispielsweise 6-Methyl-1,2,3,4-tetrahydrochinoxalin, und/oder Gemischen aus zwei oder mehreren Verbindungen aus einer oder mehreren dieser Klassen.

Weitere verwendbare Kupplerkomponenten, wie m-Aminophenole bzw. deren Derivate, werden bevorzugt ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamitio)benzol, 3-Ethylamino-4-methylphenol, 2,4-Dichlor-3-aminophenol und deren physiologisch verträglichen Salzen.

Weitere verwendbare Kupplerkomponenten, wie z.B. 3-Diaminobenzole bzw. deren Derivate, werden bevorzugt ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen.

Weitere verwendbare Kupplerkomponenten, wie z.B. o-Diaminobenzole bzw. deren Derivate, werden bevorzugt ausgewählt aus mindestens einer Verbindung aus der Gruppe, die gebildet wird aus 3,4-Diaminobenzoesäure und 2,3-Diamino-1-methylbenzol und deren physiologisch verträglichen Salzen.

Weitere verwendbare Kupplerkomponenten, wie z.B. Di- beziehungsweise Trihydroxybenzole und deren Derivate, werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Weitere verwendbare Kupplerkomponenten, wie z.B. Pyridinderivate, werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimetho-xypyridin, 3,4-Diaminopyridin, 2-(2-Methoxyethyl)amino-3-amino-6-methoxypyridin, 2-(4'-Methoxyphenyl)amino-3-aminopyridin und deren physiologisch verträglichen Salzen.

Als Kupplerkomponente geeignete Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Als Kupplerkomponente geeignete Indolderivate werden ausgewählt aus 4-Hydroxyindol, 6-Hydroxyindol und 7-Hydroxyindol und deren physiologisch verträglichen Salzen.

Als Kupplerkomponente geeignete Indolinderivate werden bevorzugt ausgewählt aus 4-Hydroxyindolin, 6-Hydroxyindolin und 7-Hydroxyindolin und deren physiologisch verträglichen Salzen.

Als Kupplerkomponente geeignete Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen.

Geeignete Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Amino-phenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethylamino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydro-xyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Besonders bevorzugt sind dabei Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,0001 bis 10 Gew.-%, vorzugsweise 0,001 bis 5 Gew.-%, jeweils bezogen auf das gesamte Mittel, verwendet.

Weiterhin können die erfindungsgemäßen Mittel mindestens einen weiteren direktziehenden Farbstoff enthalten. Dabei handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Zubereitung, eingesetzt. Die Gesamtmenge an direktziehenden Farbstoffen beträgt vorzugsweise höchstens 20 Gew.-%.

Direktziehende Farbstoffe können in anionische, kationische und nichtionische direktziehende Farbstoffe unterteilt werden.

Bevorzugte anionische direktziehende Farbstoffe sind die unter den internationalen Bezeichnungen (INCI) bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehende Farbstoffe sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Als nichtionische direktziehende Farbstoffe eignen sich insbesondere nichtionische Nitro- und Chinonfarbstoffe und neutrale Azofarbstoffe. Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Eine weitere Möglichkeit zur Farbveränderung bietet die Verwendung von Färbemitteln, welche so genannte Oxofarbstoffvorprodukte enthalten. Eine erste Klasse der Oxofarbstoffvorprodukte sind Verbindungen mit mindestens einer reaktiven Carbonylgruppe. Diese erste Klasse wird als Komponente (Oxo1) bezeichnet. Eine zweite Klasse der Oxofarbstoffvorprodukte bilden CH-acide Verbindungen und Verbindungen mit primärer oder sekundärer Aminogruppe oder Hydroxygruppe, die wiederum ausgewählt werden aus Verbindungen der Gruppe, die gebildet wird aus primären oder sekundären aromatischen Aminen, stickstoffhaltigen heterozyklischen Verbindungen sowie aromatischen Hydroxyverbindungen. Diese zweite Klasse wird als Komponente (Oxo2) bezeichnet. Die vorgenannten Komponenten (Oxo1) und (Oxo2) sind im Allgemeinen selbst keine Farbstoffe, und eignen sich daher jede für sich genommen allein nicht zur Färbung keratinhaltiger Fasern. In Kombination bilden sie in einem nichtoxidativen Prozess, der so genannten Oxofärbung, Farbstoffe aus. Die resultierenden Färbungen besitzen teilweise Farbechtheiten auf der keratinhaltigen Faser, die mit denen der Oxidationsfärbung vergleichbar sind.

Als Oxofarbstoffvorprodukte kommt bevorzugt eine Kombination aus - mindestens einer Verbindung, die mindestens eine reaktive Carbonylgruppe enthält (Komponente (Oxo1)) mit mindestens einer Verbindung (Komponente Oxo2) - Verbindungen, ausgewählt aus (Oxo2a) CH-aciden Verbindungen und/oder aus (Oxo2b) Verbindungen mit primärer oder sekundärer Aminogruppe oder Hydroxygruppe, ausgewählt aus mindestens einer Verbindung der Gruppe, die gebildet wird aus primären oder sekundären aromatischen Aminen, stickstoffhaltigen heterozyklischen Verbindungen und aromatischen Hydroxyverbindungen zum Einsatz.

Reaktive Carbonylverbindungen als Komponente (Oxo1) besitzen im Sinne der Erfindung mindestens eine Carbonylgruppe als reaktive Gruppe, welche mit der Komponente (Oxo2) unter Ausbildung einer kovalenten Bindung reagiert. Bevorzugte reaktive Carbonylverbindungen sind ausgewählt aus Verbindungen die mindestens eine Formylgruppe und/oder mindestens eine Ketogruppe, insbesondere mindestens eine Formylgruppe, tragen. Ferner sind erfindungsgemäß auch solche Verbindungen als Komponente (Oxo1) verwendbar, in denen die reaktive Carbonylgruppe derart derivatisiert bzw. maskiert ist, dass die Reaktivität des Kohlenstoffatoms der derivatisierten Carbonylgruppe gegenüber der Komponente (Oxo2) stets vorhanden ist. Diese Derivate sind bevorzugt Additionsverbindungen
a) von Aminen und deren Derivate unter Bildung von Iminen oder Oximen als Additionsverbindung
b) von Alkoholen unter Bildung von Acetalen oder Ketalen als Additionsverbindung
c) von Wasser unter Bildung von Hydraten als Additionsverbindung (Komponente (Oxo1) leitet sich in diesem Fall c) von einem Aldehyd ab)
an das Kohlenstoffatom der Carbonylgruppe der reaktiven Carbonylverbindung.

Ganz besonders bevorzugt werden als reaktive Carbonylkomponente im Rahmen der Oxofärbung Benzaldehyd und/oder Zimtaldehyd und/oder Naphthaldehyd und/oder mindestens ein Derivat dieser vorgenannten Aldehyde, die insbesondere einen oder mehrere Hydroxy-, Alkoxy- oder Aminosubstituenten tragen, verwendet.

Als CH-acide Verbindungen werden im Allgemeinen solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-Wasserstoff-Bindung bewirkt wird. Prinzipiell sind der Auswahl der CH-aciden Verbindungen keine Grenzen gesetzt, solange nach der Kondensation mit den reaktiven Carbonylverbindungen der Komponente (Oxo1) eine für das menschliche Auge sichtbar farbige Verbindung erhalten wird. Es handelt sich erfindungsgemäß bevorzugt um solche CH-aciden Verbindungen, welche einen aromatischen und/oder einen heterozyklischen Rest enthalten. Der heterozyklische Rest kann wiederum alphatisch oder aromatisch sein. Besonders bevorzugt werden die CH-aciden Verbindungen ausgewählt aus heterozyklischen Verbindungen, insbesondere kationischen, heterozyklischen Verbindungen.

Die CH-aciden Verbindungen der Oxofarbstoffvorprodukte der Komponente (Oxo2a) werden ganz besonders bevorzugt ausgewählt aus mindestens einer Verbindung der Gruppe, bestehend aus 2-(2-Furoyl)-acetonitril, 2-(5-Brom-2-furoyl)-acetonitril, 2-(5-Methyl-2-trifluormethyl-3-furoyl)-acetonitril, 3-(2,5-Dimethyl-3-furyl)-3-oxopropanitril, 2-(2-Thenoyl)-acetonitril, 2-(3-Thenoyl)-acetonitril, 2-(5-Fluor-2-thenoyl)-acetonitril, 2-(5-Chlor-2-thenoyl)-acetonitril, 2-(5-Bro-2-thenoyl)-acetonitril, 2-(2,5-Dimethylpyrrol-3-oyl)-acetonitril, 1 H-Benzimidazol-2-ylacetonitril, 1 H-Benzothiazol-1-ylacetonitril, 2-(Pyrid-2-yl)acetonitril, 2,6-Bis(cyanmethyl)-pyridin, 2-(Indol-3-oyl)-acetonitril, 8-Canacetyl-7-methoxy-4-methylcumarin, 2-(Chinoxalin-2-yl)-acetonitril, 1,2,3,3-Tetramethyl-3H-indoliumiodid, 1,2,3,3-Tetramethyl-3H-indoliummethansulfonat, 2,3-Dimethyl-benzothiazoliumiodid, 1,2-Dihydro1,3-diethyl-4,6-dimethyl-2-oxo-pyrimidinium-hydrogensulfat, 1,2-Dihydro-1,3,4,6-tetramethyl-2-thioxo-pyrimidinium-chlorid, 1,2-Dihydro-1,3-diethyl-4-methyl-2-thioxo-pyrimidinium-hydrogensulfat, 1,2-Dihydro-1,3-dipropyl-4-methyl-2-thioxo-pyrimidinium-chlorid und 1,2-Dihydro-1,3-dipropyl-4-methyl-2-thioxo-pyrimidinium-hydrogensulfat.

Des weiteren kann als Komponente (Oxo2b) mindestens ein Oxidationsfarbstoffvorprodukt mit mindestens einer primären oder sekundären Aminogruppe und/oder mindestens einer Hydroxygruppe verwendet werden. Bevorzugt geeignete Vertreter finden sich unter der Ausführung der Oxidationsfarbstoffvorprodukte. Es ist jedoch erfindungsgemäß bevorzugt, wenn die Verbindungen der Komponente (Oxo2) nur unter CH-aciden Verbindungen ausgewählt werden.

Die voran stehend genannten Verbindungen der Komponente (Oxo1) sowie der Komponente (Oxo2) werden, wenn sie zum Einsatz kommen, jeweils vorzugsweise in einer Menge von 0,03 bis 65 mmol, insbesondere von 1 bis 40 mmol, bezogen auf 100 g des gesamten Mittels, verwendet.

Die Mittel zum Färben von Haaren enthaltend mindestens eine Verbindung der Formel I, wie zuvor beschrieben, enthalten mit besonderem Vorzug zusätzlich Wasserstoffperoxid. Derartige Mittel zum Färben und gegebenenfalis gleichzeitigem Aufhellen keratinhaltige Fasern sind besonders bevorzugt, die 0,5 bis 15 Gew.-%, vorzugsweise 1 bis 12,5 Gew.-%, besonders bevorzugt 2,5 bis 10 Gew.-% und insbesondere 3 bis 6 Gew.-% Wasserstoffperoxid (berechnet als 100%iges H₂O₂) enthalten.

Das Wasserstoffperoxid kann auch in Form dessen Anlagerungsverbindungen an feste Träger eingesetzt werden, bevorzugt wird Wasserstoffperoxid selbst verwendet. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon nH₂O₂ (n ist eine positive ganze Zahl größer 0), Harnstoffperoxid und Melaminperoxid, eingesetzt.

Ganz besonders bevorzugt sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; vorzugsweise werden 6- bis 12-prozentige Lösungen in Wasser verwendet.

Für eine Farbveränderung mittels Aufhellung bzw. Bleichung des Substrats, beispielsweise der Haare, wird bevorzugt in kosmetischen Mitteln neben den Oxidationsmitteln zusätzlich mindestens ein Bleichverstärker eingesetzt.

Bleichverstärker werden bevorzugt zur Steigerung der Bleichwirkung des Oxidationsmittels, insbesondere des Wasserstoffperoxids, eingesetzt. Geeignete Bleichverstärker sind
(BV-i) Verbindungen, die unter Perhydrolysebedingungen aliphatische Peroxocarbonsäuren und/oder gegebenenfalls substituierte Perbenzoesäure ergeben,
und/oder
(BV-ii) Carbonatsalze und/oder Hydrogencarbonatsalze,
und/oder
(BV-iii) organische Carbonate,
und/oder
(BV-iv) Carbonsäuren,
und/oder
(BV-v) Peroxoverbindungen.

Bleichverstärker sind bevorzugt Peroxoverbindungen, insbesondere anorganische Peroxoverbindungen. Unter die bleichverstärkenden Peroxoverbindungen fallen keine Anlagerungsprodukte von Wasserstoffperoxid an andere Komponenten und auch nicht Wasserstoffperoxid selbst. Die Auswahl der Peroxoverbindungen unterliegt darüber hinaus keinen Beschränkungen. Bevorzugte Peroxoverbindungen sind Peroxidisulfatsalze, Persulfatsalze, Peroxidiphosphatsalze (insbesondere Ammoniumperoxidisulfat, Kaliumperoxidisulfat, Natriumperoxidisulfat, Ammoniumpersulfat, Kaliumpersulfat, Natriumpersulfat, Kaliumperoxidiphosphat) und Peroxide (wie Bariumperoxid und Magnesiumperoxid). Unter diesen Peroxoverbindungen, die auch in Kombination eingesetzt werden können, sind erfindungsgemäß die Peroxidisulfate, insbesondere Ammoniumperoxidisulfat, bevorzugt. Hier sind Mittel zum Färben und gegebenenfalls gleichzeitigem Aufhellen keratinischer Fasern bevorzugt, die zusätzlich 0,01 bis 2 Gew.-% mindestens einer festen Peroxoverbindung, die ausgewählt ist aus Ammonium-, Alkalimetall- und Erdalkalimetallpersulfaten, - peroxomonosulfaten und -peroxidisulfaten enthalten, wobei bevorzugte Mittel Peroxidisulfate enthalten, die vorzugsweise ausgewählt sind aus Natriumperoxidisulfat und/oder Kaliumperoxidisulfat und/oder Ammoniumperoxidisulfat und wobei bevorzugte Mittel mindestens zwei verschiedene Peroxidisulfate enthalten.

Weiterhin besonders bevorzugt sind Persulfate, insbesondere das als Carosche Salz bezeichnete Gemisch aus Kaliumperoxosulfat, Kaliumhydrogensulfat und Kaliumsulfat.

Die Bleichverstärker sind in den erfindungsgemäßen, kosmetischen Mitteln bevorzugt in Mengen von 5 bis 30 Gew.-%, insbesondere in Mengen von 8 bis 20 Gew.-%, jeweils bezogen auf das Gewicht des anwendungsbereiten Mittels, enthalten.

Weiterhin hat es sich als vorteilhaft erwiesen, wenn die Färbe- und/oder Aufhellmittel nichtionogene grenzflächenaktive Stoffe enthalten.

Dabei sind solche grenzflächenaktive Stoffe, die einen HLB-Wert von 5,0 und größer aufweisen, bevorzugt. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen.

Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, dass die gewählten Substanzen bevorzugt frei von Lösungsmitteln, Stellmitteln und anderen Begleitstoffen sein sollen.

Als weiteren Bestandteil können die erfindungsgemäßen Zusammensetzungen als Haarfärbemittel mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Ferner können die erfindungsgemäßen Färbe- und/oder Aufhellmittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/lsobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z.B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Maleinsäure und Milchesäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazoliniummethosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genusssäuren und Basen,
- Wirkstoffe wie Panthenol, Pantothensäure, Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennnessel, Hamamelis, Hopfen, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,
- Cholesterin,
- Konsistenzgeber wie Zuckerester, Polyolester oder Polyalkylether,
- Fette und Wachse wie Walrat, Bienenwachs, Montanwachs und Paraffine, Fettalkohole und Fettsäureester,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien,
enthalten.

Die zuvor genannten Wirk-, Hilfs- und Zusatzstoffe können auch in den erfindungsgemäßen Zubereitungen enthalten sein, enthaltend mindestens eine Verbindung der Formel I und einen für kosmetische, pharmazeutische, dermatologische Zubereitungen oder Haushaltsprodukte geeigneten Träger, die beispielsweise zum Färben der Haut verwendet werden oder wobei die Zubereitung als solche gefärbt werden soll. Beschränkungen hinsichtlich der Inhaltsstoffe solcher Zubereitungen gibt es nicht.

In bevorzugten Ausführungsformen wird die mindestens eine Verbindung nach Formel I mit den definierten oder als bevorzugt angegebenen Substituenten oder bevorzugte Einzelverbindungen in den erfindungsgemäßen Zubereitungen für die Färbung der Haut oder anderen Substraten sowie zur Färbung von Zubereitungen per se typischerweise in Mengen von 0,05 bis 10 Gew.-%, bevorzugt in Mengen von 0,1 Gew.-% bis 5 Gew.-% und besonders bevorzugt in Mengen von 0,5 bis 2 Gew.-%, eingesetzt. Dabei bereitet es dem Fachmann keinerlei Schwierigkeiten die Mengen abhängig von der beabsichtigten Wirkung der Zubereitung entsprechend auszuwählen.

Die erfindungsgemäßen Verbindungen der Formel I können zudem zum Färben von Haushaltsprodukten, insbesondere transparent verpackten Haushaltsprodukten eingesetzt werden. Zu Haushaltsprodukten zählen beispielsweise Spülmittel, Reinigungs- und Waschmittel sowie Lufterfrischer für Räume, Autos und Toiletten.

In den beschriebenen kosmetischen, dermatologischen, pharmazeutischen Zubereitungen oder Haushaltsprodukten, die erfindungsgemäß mindestens eine Verbindung nach Formel I nach Anspruch 8 enthalten, können weiterhin auch Farbpigmente enthalten sein, wobei der Schichtaufbau der Pigmente nicht limitiert ist.

Vorzugsweise sollte das Farbpigment bei Einssatz von 0,5 bis 5 Gew.-% hautfarben oder bräunlich sein. Die Auswahl eines entsprechenden Pigments ist für den Fachmann geläufig.

Die Zubereitungen können neben den Verbindungen nach Formel I nach Anspruch 8 sowie den gegebenenfalls anderen Inhaltsstoffen weitere organische UV-Filter, sogenannte hydrophile oder lipophile Sonnenschutzfilter, die im UVA-Bereich und/oder UVB-Bereich und/oder IR und/oder VIS-Bereich (Absorber) wirksam sind, enthalten. Diese Substanzen können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische wie auch anorganische UV Filter sind in den Patentanmeldungen EP-A 0 487 404 sowie WO2009/077356 angegeben. Im Folgenden werden die genannten UV-Filter meist nach der INCI-Nomenklatur benannt.

Insbesondere für eine Kombination geeignet sind: para-Aminobenzoesäure und deren Derivate: PABA, Ethyl PABA, Ethyl dihydroxypropyl PABA, Ethylhexyl dimethyl PABA, z. B. vetrieben unter dem Namen "Escaloi 507" von der Fa. ISP, Glyceryl PABA, PEG-25 PABA, z. B. vetrieben unter dem Namen "Uvinul P25" von der Fa. BASF.

Salicylate: Homosalate vetrieben unter dem Namen "Eusolex HMS" von der Fa. Merck; Ethylhexyl salicylate, z. B. vetrieben unter dem Namen "Neo Heliopan OS" von der Fa. Symrise, Dipropylene glycol salicylate, z. B. vetrieben unter dem Namen "Dipsal" von der Fa. Scher, TEA salicylate, z. B. vetrieben unter dem Namen "Neo Heliopan TS" von der Fa. Symrise.

β,β-Diphenylacrylate Derivate: Octocrylene, z. B. vetrieben unter dem Namen "Eusolex® OCR" von der Firma Merck, "Uvinul N539" von der Fa. BASF, Etocrylene, z. B. vetrieben unter dem Namen "Uvinul N35" von der BASF.

Benzophenone Derivate: Benzophenone-1, z. B. vetrieben unter dem Namen "Uvinul 400"; Benzophenone-2, z. B. vetrieben unter dem Namen "Uvinul D50" ; Benzophenone-3 oder Oxybenzone, z. B. vetrieben unter dem Namen "Uvinul M40";Benzophenone-4, z. B. vetrieben unter dem Namen "Uvinul MS40" ; Benzophenone-9, z. B. vetrieben unter dem Namen "Uvinul DS-49" von der Fa. BASF, Benzophenone-5, Benzophenone-6, z. B. vetrieben unter dem Namen "Helisorb 11" von der Fa. Norquay, Benzophenone-8, z. B. vetrieben unter dem Namen "Spectra-Sorb UV-24" von der Fa. American Cyanamid, Benzophenone-12 n-hexyl 2-(4-diethylamino-2-hydroxybenzoyl) benzoate oder 2-Hydroxy-4-methoxybenzophenon, vertrieben von der Fa. Merck, Darmstadt unter dem Namen Eusolex® 4360.

Benzylidencampher Derivate: 3-Benzylidenecamphor, z. B. vetrieben unter dem Namen "Mexoryl SD" von der Fa. Chimex, 4-Methylbenzylidenecamphor, z. B. vetrieben unter dem Namen "Eusolex 6300" von der Fa. Merck, Benzylidenecamphorsulfonsäure, z. B. vetrieben unter dem Namen "Mexoryl SL" von der Fa. Chimex, Camphor benzalkonium methosulfate, z. B. vetrieben unter dem Namen "Mexoryl SO" von der Fa. Chimex, Terephthalylidenedicamphorsulfonsäure, z. B. vetrieben unter dem Namen "Mexoryl SX" von der Fa Chimex, Polyacrylamidomethylbenzylidenecamphor vetrieben unter dem Namen "Mexoryl SW" von der Fa. Chimex.

Phenylbenzimidazol Derivate: Phenylbenzimidazolesutfonsäure, z. B. vetrieben unter dem Namen "Eusolex 232" von der Fa. Merck, Dinatrium phenyl dibenzimidazole tetrasulfonat, z. B. vetrieben unter dem Namen "Neo Heliopan AP" von der Fa. Symrise.

Phenylbenzotriazol Derivate: Drometrizole trisiloxane, z. B. vetrieben unter dem Namen "Silatrizole" von der Fa. Rhodia Chimie, Methylenebis(benzotriazolyl)tetramethylbutylphenol in fester Form, z. B. vetrieben unter dem Namen "MIXXIM BB/100" von der Fa. Fairmount Chemical, oder in mikronisierter Form als wässrige Dispersion, z. B. vetrieben unter dem Namen "Tinosorb M" von der Fa. Ciba Specialty Chemicals.

Triazin Derivate: Ethylhexyltriazone, z. B. vetrieben unter dem Namen "Uvinul T150" von der Fa. BASF, Diethylhexylbutamidotriazone, z. B. vetrieben unter dem Namen "Uvasorb HEB" von der Fa. Sigma 3V, 2,4,6-tris(diisobutyl 4'-aminobenzalmalonate)-s-triazine oder 2,4,6-Tris-(biphenyl)-1,3,5-triazine.

Anthranilin Derivate: Menthyl anthranilate, z. B. vetrieben unter dem Namen "Neo Heliopan MA" von der Fa. Symrise.

Imidazol Derivate: Ethylhexyldimethoxybenzylidenedioxoimidazoline propionat.

Benzalmalonat Derivate: Polyorganosiloxane enthaltend funktionelle benzalmalonate Gruppen, wie z.B. Polysilicone-15, z. B. vetrieben unter dem Namen "Parsol SLX" von der Hoffmann LaRoche.

4,4-Diarylbutadien Derivate: 1,1-Dicarboxy(2,2'-dimethylpropyl)-4,4-diphenylbutadiene.

Benzoxazole Derivate: 2,4-bis[5-(1-dimethylpropyl)benzoxazol-2-yl(4-phenyl)imino]-6-(2-ethylhexyl)imino-1,3,5-triazine, z. B. vetrieben unter dem Namen Uvasorb K2A von der Fa. Sigma 3V und Mischungen dieses enthaltend.

Piperazinderivate wie beispielsweise die Verbindung

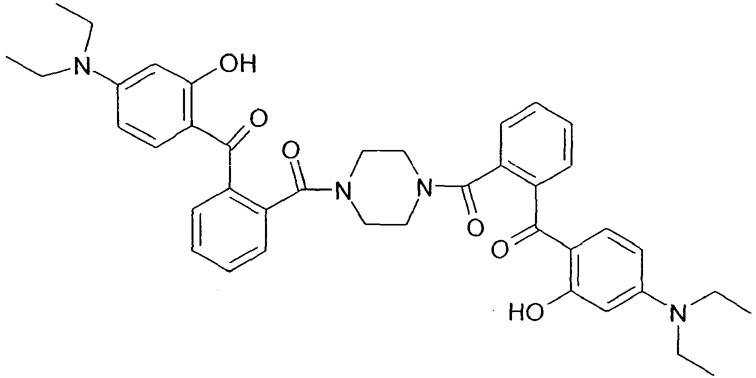

Die in der Liste aufgeführten Verbindungen sind nur als Beispiele aufzufassen. Selbstverständlich können auch andere UV-Filter verwendet werden.

Geeignete organischen UV-schützende Substanzen sind bevorzugt aus der folgenden Liste auszuwählen: Ethylhexyl salicylate, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine und Mischungen davon.

Diese organischen UV-Filter werden in der Regel in einer Menge von 0,01 Gewichtsprozent bis 20 Gewichtsprozent, vorzugsweise 1 Gew.-% - 10 Gew.-%, in Formulierungen eingearbeitet.

Die Zubereitungen können neben den Verbindungen der Formel I sowie den gegebenenfalls anderen organischen UV-Filtern, wie zuvor beschrieben, weitere anorganische UV-Filter, sogenannte partikuläre UV-Filter enthalten.

Diese Kombinationen mit partikulären UV-Filtern sind sowohl als Pulver als auch als Dispersion oder Paste der folgenden Typen möglich.

Hierbei sind sowohl solche aus der Gruppe der Titandioxide wie z.B. gecoatetes Titandioxid (z.B. Eusolex^{®} T-2000, Eusolex^{®}T-AQUA, Eusolex^{®}T-AVO, Eusolex^{®}T-OLEO), Zinkoxide (z.B. Sachtotec^{®}), Eisenoxide oder auch Ceroxide und/oder Zirkonoxide bevorzugt.

Ferner sind auch Kombinationen mit pigmentärem Titandixoxid oder Zinkoxid möglich, wobei die Partikelgröße dieser Pigmente größer oder gleich 200 nm sind, beispielsweise Hombitan® FG oder Hombitan® FF-Pharma.

Weiter kann es bevorzugt sein, wenn die Zubereitungen anorganische UV-Filter enthalten, die mit üblichen Methoden, wie beispielsweise in Cosmetics & Toiletries, February 1990, Vol. 105, pp. 53-64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Bevorzugt einzusetzende partikuläre UV-Filter sind dabei:
- unbehandelte Titandioxide wie z.B. die Produkte Microtitanium Dioxide MT 500 B der Fa. Tayca; Titandioxd P25 der Fa. Degussa,
- Nachbehandelte mikronisierte Titandioxide mit Aluminiumoxid und Siliciumdioxid Nachbahandlung wie z.B. das Produkt "Microtitanium Dioxide MT 100 SA der Tayca; oder das Produkt "Tioveil Fin" der Fa. Uniqema,
- Nachbehandelte mikronisierte Titandioxide mit Aluminiumoxid und/oder Aluminiumstearate/laurate Nachbehandlung wie z.B. Microtitanium Dioxide MT 100 T der Fa. Tayca, Eusolex T-2000 der Firma Merck,
- Nachbehandelte mikronisierte Titandioxide mit Eisenoxid und/oder Eisenstearate Nachbehandlung wie z.B. das Produkt "Microtitanium Dioxide MT 100 F" der Fa. Tayca,
- Nachbehandelte mikronisierte Titandioxide mit Siliciumdioxide, Aluminiumoxid und Silicon Nachbehandlung wie z.B. das Produkt "Microtitanium Dioxide MT 100 SAS",der Fa. Tayca,
- Nachbehandelte mikronisierte Titandioxide mit Natrumhexametaphosphate, wie z.B. das Produkt "Microtitanium Dioxide MT 150 W" der Fa. Tayca.

Die zur Kombination einzusetzenden behandelten mikronisierten Titandioxide können auch nachbehandelt sein mit:
- Octyltrimethoxysilane; wie z.B. das Produkt Tego Sun T 805 der Fa. Degussa,
- Siliciumdioxid; wie z.B. das Produkt Parsol T-X der Fa. DSM,
- Aluminiumoxid und Stearinsäure; wie z.B. das Produkt UV-Titan M160 der Fa. Sachtleben,
- Aluminium und Glycerin; wie z.B. das Produkt UV-Titan der Fa. Sachtleben,
- Aluminium und Silikonölen, wie z.B. das Produkt UV-Titan M262 der Fa. Sachtleben,
- Natriumhexamethaphosphat und Polyvinylpyrrolidon,
- Polydimethylsiloxane, wie z.B. das Produkt 70250 Cardre UF TiO2SI3" der Fa. Cardre,
- Polydimethylhydrogensiloxane, wie z.B. das Produkt Microtitanium Dioxide USP Grade Hydrophobic" der Fa. Color Techniques.

Ferner kann auch die Kombination mit folgenden Produkten vorteilhaft sein:
- Unbehandelte Zinkoxide wie z. B. das Produkt Z-Cote der Fa. BASF (Sunsmart), Nanox der Fa. Elementis
- Nachbehandelte Zinkoxide wie z.B die folgenden Produkte:
   ∘ "Zinc Oxide CS-5" der Fa. Toshibi (ZnO nachbehandelt mit polymethylhydrogenosiloxane)
   ∘ Nanogard Zinc Oxide FN der Fa. Nanophase Technologies
   ∘ "SPD-Z1" der Fa Shin-Etsu (ZnO nachbehandelt mit einem Silikongepfropften Acrylpolymer, dispergiert in Cyclodimethylsiloxane
   ∘ "Escalol Z100" der Fa ISP (Aluminiumoxid nachbehandeltes ZnO dispergiert in einer ethylhexyl methoxycinnamate/PVPhexadecene/methicone copolymer Mischung)
   ∘ "Fuji ZNO-SMS-10" der Fa. Fuji Pigment (ZnO nachbehandelt mit Siliciumdioxid und Polymethylsilesquioxan);
   ∘ Unbehandeltes Ceroxide Mikropigment z.B. mit der Bezeichnung "Colloidal Cerium Oxide" der Fa Rhone Poulenc
   ∘ Unbehandelte und/oder nachbehandelte Eisenoxide mit der Bezeichnung Nanogar der Fa. Arnaud.

Beispielhaft können auch Mischungen verschiedener Metalloxide , wie z.B. Titandioxid und Ceroxid mit und ohne Nachbenhandlung eingesetzt werden, wie z.B. das Produkt Sunveil A der Fa. Ikeda. Außerdem können auch Mischungen von Aluminiumoxid, Siliciumdioxid und Silikonnachbehandelten Titandioxid. Zinkoxid-Mischungen wie z.B. das Produkt UV-Titan M261 der Fa. Sachtleben in Kombination mit dem erfindungsgemäßen UV-Schutzmittel verwendet werden.

Diese anorganischen UV-Filter werden in der Regel in einer Menge von 0,1 Gewichtsprozent bis 25 Gewichtsprozent, vorzugsweise 2 Gew.-% - 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen. Daher kann es bevorzugt sein, wenn ein oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Bevorzugte Zubereitungen können auch mindestens einen weiteren kosmetischen Wirkstoff enthalten, beispielsweise ausgewählt aus Antioxidantien, Anti-aging-Wirkstoffen, Anti-Cellulite Wirkstoffen, Selbstbräunungssubstanzen, hautaufhellenden Wirkstoffen oder Vitaminen.

Ferner sind erfindungsgemäße Farbstoffe mit allen Wirkstoffen und Hilfstoffen kombinierbar, wie sie in WO2009/098139 systematisch gelistet sind. Insbesondere gehören diese Stoffe zu den darin aufgeführten Verwendungskategorien "Moisturizers and Humectants", "Desquamating agents", "Agents for improving the barrier function", "Depigmenting Agents", "Antioxidants", "Dermo-relaxing or dermo-decontracting agents", "Antiglycation agents", "Agents for stimulating the synthesis of dermal and/or epidermal macromolecules and/or for preventing their degradation", "Agents for stimulating fibroblast or keratinocyte proliferation and/or keratinocyte differentiation", "Agents for promoting the maturation of the horny envelope", "NO-synthase inhibitors", "Peripheral benzodiazepine receptor (PBR) antagonists", "Agents for increasing the activity of the sebaceous glands", "Agents for stimulating the energy metabolism of cells", "Tensioning agents", "Fat-restructuring agents", "Sliming agents", "Agents for promoting the cutaneous microcirculation", "Calmatives or anti-irritants", "Sebo-regulating or anti-seborrhoic agents", "Astringents", "Cicatrizing agents", "Anti-inflammatory agents", "Antiacne agents".

Die schützende Wirkung von Zubereitungen gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet geeignet schnell oder zeitverzögert wirkende Antioxidantien auszuwählen.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Geeignete Antioxidantien sind auch Verbindungen der Formeln A oder B 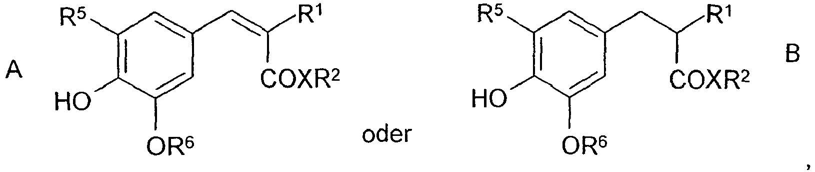 worin
R¹ aus der Gruppe -C(O)CH₃, -CO₂R³, -C(O)NH₂ und -C(O)N(R⁴)₂ ausgewählt werden kann,
X O oder NH,
R² lineares oder verzweigtes Alkyl mit 1 bis 30 C-Atomen,
R³ lineares oder verzweigtes Alkyl mit 1 bis 20 C-Atomen,
R⁴ jeweils ungabhängig voneinander H oder lineares oder verzweigtes Alkyl mit 1 bis 8 C-Atomen,
R⁵H oder lineares oder verzweigtes Alkyl mit 1 bis 8 C-Atomen oder lineares oder verzweigtes Alkoxy mit 1 bis 8 C-Atomen und
R⁶ lineares oder verzweigtes Alkyl mit 1 bis 8 C-Atomen bedeutet, vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex^{®} ST Liquid) und/oder 2-(4-Hydroxy-3,5-dimethoxybenzyl)-malonsäure-bis-(2-ethylhexyl)ester (z.B. RonaCare^{®} AP). Ferner ist die Kombination mit 2-(4-Hydroxy-3-methoxybenzyliden)-malonsäure-bis-isopropylester bzw. 2-(4-Hydroxy-3-methoxybenzyl)-malonsäure-bis-isopropylester (hydrogenated diisopropyl vanilidene malonate) bevorzugt. Analoges gilt für entsprechende Bis-ethylester.

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure, natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004). Derartige Antioxidantien werden mit den erfindungsgemäßen Verbindungen in solchen Zusammensetzungen üblicherweise in Gewichtsprozentverhältnissen im Bereich von 1000:1 bis 1:1000, bevorzugt in Gewichtsprozentverhältnissen von 100:1 bis 1:100 eingesetzt.

Unter den Phenolen, die erfindungsgemäß verwendet werden können, sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH-Gruppen in 3'4'-oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers und I.M.C.M. Rietjens (Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete anti-aging Wirkstoffe, insbesondere für hautpflegende Zubereitungen, sind vorzugsweise sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin- Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myo-inositol-phosphat (DIP), cyclisches 2,3-diphosphoglycerat (cDPG), 1,1-Diglycerin-Phosphat (DGP), ß-Mannosylglycerat (Firoin), ß-Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure) und deren Derivate zu nennen.

Additional können als anti-aging Wirkstoffe Produkte der Firma Merck wie z.B. 5,7-Dihydroxy-2-methyl-chromon, vermarktet unter dem Handelsnamen RonaCare®Luremine, Ronacare®Isoquercetin, Ronacare®Tilirosid oder Ronacare®Cyclopeptide 5 verwendet werden.

Ferner können die erfindungsgemäßen Zubereitungen mindestens einen Selbstbräuner als weiteren Inhaltsstoff enthalten.

Als vorteilhafte Selbstbräuner können unter anderem eingesetzt werden: 1,3-Dihydroxyaceton, Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon) oder 2-Hydroxy-1,4-naphtochinon (Lawson). Ganz besonders bevorzugt ist das 1,3-Dihydroxyaceton, Erythrulose oder deren Kombination.

Zubereitungen mit Selbstbräunereigenschaften, insbesondere solche, die Dihydroxyaceton enthalten, neigen bei der Anwendung auf der menschlichen Haut zu Fehlgerüchen, die vermutlich durch Abbauprodukte des Dihydroxyacetons selbst oder durch Produkte von Nebenreaktionen verursacht werden und die von den Anwendern teilweise als unangenehm empfunden werden. Es hat sich gezeigt, dass diese Fehlgerüche bei Verwendung von Formaldehydfängern und/oder Flavonoiden vermieden werden. Daher kann die erfindungsgemäße Zubereitung enthaltend mindestens einen Selbstbräuner vorzugsweise auch Formaldehydfänger sowie gegebenenfalls Flavonoide zur Verbesserung des Geruches enthalten.

Vorzugsweise wird der Formaldehydfänger ausgewählt aus der Gruppe Alkalimetall-, Erdalkalimetall- oder Ammoniumdisulfit. Besonders bevorzugt ist eine Zubereitung, die in Kombination DHA Plus, eine Mischung aus DHA, Natriumdisulfit und Magnesiumstearat, enthält.

DHA Plus ist eine Produktmischung, welche zur Maskierung, Eliminierung oder Neutralisierung des Formaldehyds Natriummetabisulfit, gleichbedeutend mit Na₂S₂O₅ oder INCI: sodium disulfite, enthält. Der Zusatz von Natriumdisulfit in fertigen Formulierungen führt zur signifikanten Reduktion oder Unterbindung des unangenehmen Geruchs. DHA Plus wird von der Firma Merck, Darmstadt vertrieben.

Das gegebenenfalls in der Zubereitung vorhandene Flavonoid wirkt dabei zusätzlich als Stabilisator für den Selbstbräuner bzw. die Selbstsbräunungssubstanzen und/oder reduziert oder vermeidet oder verbessert lagerabhängige Fehlgerüche, die auch durch enthaltene Zusatz- oder Hilfsstoffe entstehen können.

Vorzugsweise handelt es sich um ein Flavonoid, bei dem eine oder mehrere phenolische Hydroxygruppen durch Veretherung oder Veresterung blockiert sind. Beispielsweise haben sich Hydroxyethyl-substituierte Flavonoide, wie vorzugsweise Troxerutin, Troxequercetin, Troxeisoquercetin oder Troxeluteolin, und Flavonoid-sulfate oder Flavonoid-phosphate, wie vorzugsweise Rutinsulfate, dabei als besonderes gut geeignete Flavonoide erwiesen. Besonders bevorzugt im Sinne der erfindungsgemäßen Verwendung sind Rutinsulfat und Troxerutin. Ganz besonders bevorzugt ist die Verwendung von Troxerutin.

Die bevorzugten Flavonoide verfügen über einen nicht positiv geladenen Flavangrundkörper. Es wird vermutet, dass durch diese Flavonoide Metallionen wie z.B. Fe²⁺/Cu²⁺ komplexiert werden und so Autooxidationsvorgänge bei Duftstoffen oder Verbindungen, deren Abbau zu Fehlgerüchen führt, verhindert bzw. vermindert werden.

Besonders bevorzugt ist eine Zubereitung, die neben den Verbindungen der Formel I nach Anspruch 8 DHA Rapid und/oder Natriummetabisulfit enthalten. DHA Rapid ist eine Produktmischung enthaltend Dihydroxyaceton und Troxerutin, der Firma Merck, Darmstadt.

Entsprechende Vormischungen und Zubereitungen, die Formaldehydfänger sowie gegebenenfalls Flavonoide zur Verbesserung des Geruches auf der Haut enthalten, sind in der Deutschen Patentanmeldung mit dem Anmeldeaktenzeichen DE 10 2007 013 368.7 beschrieben, deren diesbezüglicher Inhalt ausdrücklich auch zum Offenbarungsgehalt der vorliegenden Anmeldung gehört.

Die Kombination der erfindungsgemäßen Verbindungen der Formel I mit Selbstbräunungsubstanzen ist besonders bevorzugt, um den durch den Selbstbräuner erzielbaren Farbeffekt zu verbessern, beispielsweise durch Erhöhung des Rotanteils im Farbbild zur Reduzierung des Gelbeindruckes. Daneben können die erfindungsgemäßen Verbindungen der Formel I die für Selbstbräuner bekannte Fehlgeruchproblematik reduzieren und Selbstbräuner stabilisieren.

Die Zubereitungen können auch ein oder mehrere weitere hautaufhellende Wirkstoffe oder synonym Depigmentierungswirkstoffe enthalten. Hautaufhellende Wirkstoffe können prinzipiell alle dem Fachmann bekannte Wirkstoffe sein. Beispiele von Verbindungen mit hautaufhellender Aktivität sind Hydrochinon, Kojisäure, Arbutin, Aloesin oder Rucinol. Durch solche Zubereitungen kann beispielsweise der Hautkontrast zwischen Hell- und Dunkelpartien reduziert werden. Die Haut erscheint somit homogener gefärbt.

Die Zubereitungen können auch anti-Aging-Wirkstoffe enthalten und dadurch den vorwiegend visuellen anti-Aging-Effekt (Schutz vor Photoalterung) durch die erfindungsgemäßen Verbindungen der Formel I unterstützen. Dieser visuelle Anti-aging-Effekt beruht auf einer erzielbaren homogenen Hautfärbung. Geeignete anti-aging Wirkstoffe sind beispielsweise die von der Firma Merck vertriebenen Produkte 5,7-Dihydroxy-2-methyl-chromon, vermarktet unter dem Handelsnamen RonaCare®Luremine oder die Produkte Ronacare®Isoquercetin, Ronacare®Tilirosid oder Ronacare®Cyclopeptide 5.

Die einzusetzenden Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂), insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den flavonoidhaltigen Vormischungen oder Zubereitungen üblicherweise bei kosmetischer Anwendung in Bereichen von 0,01 bis 5,0 Gew.-%, bezogen auf das Gesamtgewicht, zugesetzt. Ernährungsphysiologische Anwendungen orientieren sich am jeweiligen empfohlenen Vitaminbedarf.

Die beschriebenen Retinoide sind gleichzeitig auch wirksame Anti-Cellulite-Wirkstoffe. Ein ebenfalls bekannter Anti-Cellulite-Wirkstoff ist Koffein.

Die genannten Bestandteile der Zubereitung können in der üblichen Weise eingearbeitet werden, mit Hilfe von Techniken, die dem Fachmann wohl bekannt sind.

Geeignet sind Zubereitungen für eine äußerliche Anwendung, beispielsweise als Creme oder Milch (O/W, W/O, O/W/O, W/O/W), als Lotion oder Emulsion, in Form ölig-alkoholischer, ölig-wässriger oder wässrigalkoholischer Gele bzw. Lösungen auf die Haut aufgesprüht werden kann. Sie können als feste Stifte vorliegen oder als Aerosol konfektioniert sein. Für eine innerliche Anwendung sind Darreichungsformeln wie Kapseln, Dragees, Pulver, Tabletten-Lösungen oder Lösungen geeignet.

Als Anwendungsform der einzusetzenden Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays.

Bevorzugte Hilfsstoffe stammen aus der Gruppe der Konservierungsstoffe, Stabilisatoren, Lösungsvermittler, Färbemittel, Geruchsverbesserer.

Salben, Pasten, Cremes und Gele können die üblichen Trägerstoffe enthalten, die für die topische Verabreichung geeignet sind, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Ein bevorzugter Lösungsvermittler generell ist 2-Isopropyl-5-methylcyclohexancarbonyl-D-Alaninmethylester.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohoie, Polyoxyethyiensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Die erfindungsgemäße Mischung kann in bevorzugter Weise Hilfsstoffe enthalten, wie beispielsweise kosmetische Öle (z.B. Caprylic/Capric Triglycerides, C12-15 Alkyl Benzoate, Isopropylmyristat, Arylalkyl Benzoate wie z.B. Phenethylbenzoat (X-Tend 226) oder Ölkomponenten der Marke Cosmacol wie Dimyristyl Tartrate, Tri C14-C15 Alkyl Citrate, C12-C13 Alkyl Lactate, Tridecyl Salicylate, C12-C13 Alkyl Octanoate, C12-C13 Alkyl Malate, C12-C13 Alkyl Citrate, C12-C13 Alkyl Tartrate), oder polarprotische Hilfsstoffe (z.B. Propylenglycol, Glycerin, Isopropanol, Ethanol) oder sog. Lösungsvermittler (z.B. Butylphthalimide, Isopropylphthalimide, Dimethylisosorbide).

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der einzusetzenden Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

In einer bevorzugten Ausführungsform enthalten die einzusetzenden Zubereitungen hydrophile Tenside. Die hydrophilen Tenside werden bevorzugt gewählt aus der Gruppe der Alkylglucoside, der Acyllactylate, der Betaine sowie der Cocoamphoacetate.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren^{®} 1200 (Henkel KGaA), Oramix^{®} NS 10 (Seppic).

Die kosmetischen und dermatologischen Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-A-43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-Emulgatoren in den bevorzugten O/W-Emulsionen zu verwenden.

Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen.

Es ist ferner von Vorteil, die Fettsäureethoxylate ausfolgender Gruppe zu wählen:
Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)-glyceryllaurat, Polyethylenglycol(22)glyceryllaurat, Polyethylenglycol-(23)glyceryllaurat, Polyethylenglycol(6)glycerylcaprat/cprinat, Polyethylenglycol(20)glyceryloleat, Polyethylenglycol(20)glycerylisostearat, Polyethylenglycol(18)glyceryloleat(cocoat) zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:
Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome,
Diglycerinester gesättigter und/oder ungesättigter, verzweigter und/oder unverzweigter Alkancarbonsäuren einer Kettenlänge von 8 bis 24,
insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die öligalkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane , bevorzugt Alkane.

Die Verbindungen gemäß Formel I, wie zuvor beschrieben sind fluoreszierende Emitter und können daher ebenfalls in einer elektronischen Vorrichtung eingesetzt werden.

Ein weiterer Gegenstand der Erfindung ist daher eine elektronische Vorrichtung enthaltend mindestens eine Verbindung der Formel I, wie in Anspruch 5 beschrieben.

Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser), "organic plasmon emitting devices" (D. M. Koller et al., Nature Photonics 2008, 1-4) und Elektrophotographie-Vorrichtungen, bevorzugt organischen Elektrolumineszenzvorrichtungen (OLEDs) bzw. organischen lichtemittierenden elektrochemischen Zellen (OLECs).

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Ein möglicher Schichtaufbau ist bspw. der folgende: Kathode/ EML/Zwischeschicht/Pufferschicht/Anode, wobei EML die emittierende Schicht repräsentiert. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B.

WO 2005/011013). Weiterhin kann auf eine oder beide der Elektroden eine optische Auskopplungsschicht aufgebracht sein.

Weitere organisch funktionelle Materialien, die mit den Verbindungen der Formel I für diese spezielle Anwendung kombiniert werden können, sind beispielsweise Host Materialien, Matrix Materialien, Elektronentransportmaterialien (ETM), Elektroneninjektionsmateriaien (EIM), Lochtransportmaterialien (HTM), Lochinjektionsmaterialien (HIM), Elektronenblockiermaterialien (EBM), Lochblockiermaterialien (HBM), Exzitonenblockiermaterialien (ExBM) und/oder Emitter.

Ein Gegenstand der Erfindung ist daher weiterhin eine Formulierung oder auch Zusammensetzung enthaltend eine oder mehrere Verbindungen der Formel I nach Anspruch 8 und mindestens ein weiteres organisch funktionelles Material ausgewählt aus der Gruppe der Host Materialien,

Matrix Materialien, Elektronentransportmaterialien, Elektroneninjektionsmaterialien, Lochtransportmaterialien, Lochinjektionsmaterialien, Elektronenblockiermaterielien, Lochblockiermaterialien, Exzitonenblockiermaterialien und/oder Emitter.

In einer bevorzugten Ausführungsform der Erfindung bei Verwendung der Verbindungen der Formel I in einer elektronischen Vorrichtung, wird die mindestens eine Verbindung gemäß Formel I in der emittierenden Schicht eingesetzt, bevorzugt in Mischung mit mindestens einer weiteren Verbindung eingesetzt. Es ist bevorzugt, wenn die Verbindung gemäß Formel I in der Mischung die emittierende Verbindung (der Dotand) ist. Bevorzugte Hostmaterialien sind organische Verbindungen, deren Emission kurzwelliger ist als die der Verbindung gemäß Formel I oder die überhaupt nicht emittieren.

Ein weiterer Gegenstand der Erfindung ist daher eine organische Elektrolumineszenzvorrichtung nach Anspruch 7, dadurch gekennzeichnet, dass die mindestens eine Verbindung der Formel I, wie in Anspruch 7 beschrieben, als fluoreszierender Emitter eingesetzt wird.

Der Anteil der Verbindung gemäß Formel I in der Mischung der emittierenden Schicht beträgt zwischen 0.1 und 99.0 Gew.%, bevorzugt zwischen 0.5 und 50.0 Gew.%, besonders bevorzugt zwischen 1.0 und 20.0 Gew.%, insbesondere zwischen 1.0 und 10.0 Gew.%. Entsprechend beträgt der Anteil des Hostmaterials in der Schicht zwischen 1.0 und 99.9 Gew.%, bevorzugt zwischen 50.0 und 99.5 Gew.%, besonders bevorzugt zwischen 80.0 und 99.0 Gew.%, insbesondere zwischen 90.0 und 99.0 Gew.%.

Als Hostmaterialien kommen verschiedene Stoffklassen in Frage. Bevorzugte Hostmaterialien sind ausgewählt aus den Klassen der Oligoarylene (z. B. 2,2',7,7'-tetraphenyl-spirobifluoren gemäß EP 676461 oder Dinaphthylanthracen), insbesondere der Oligo-arylene enthaltend kondensierte aromatische Gruppen, der Oligo-arylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911), der elektronenleitenden Verbindungen, insbesondere Ketone, Phosphinoxide, Sulfoxide, etc. (z. B. gemäß WO 05/084081 oder WO 05/084082), der Atropisomere (z. B. gemäß der nicht offen gelegten Anmeldung EP 04026402.0) oder der Boronsäurederivate (z. B. gemäß der nicht offen gelegten Anmeldung EP 05009643.7). Besonders bevorzugte Hostmaterialien sind ausgewählt aus den Klassen der Oligoarylene, enthaltend Naphthalin, Anthracen und/oder Pyren oder Atropisomeren dieser Verbindungen, der Oligo-arylenvinylene, der Ketone, der Phosphinoxide und der Sulfoxide. Ganz besonders bevorzugte Hostmaterialien sind ausgewählt aus den Klassen der Oligoarylene, enthaltend Anthracen und/oder Pyren oder Atropisomeren dieser Verbindungen, der Phosphinoxide und der Sulfoxide.

Besonders geeignete Matrixmaterialien, welche in Kombination mit den Verbindungen gemäß Formel I eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z.B. gemäß der nicht offen gelegten Anmeldung DE 102008056688.8, Diazaphosphol-Derivate, z. B. gemäß der nicht offen gelegten Anmeldung DE 102009022858.6, oder Indenocarbazolderivate, z. B. gemäß den nicht offen gelegten Anmeldungen DE 102009023155.2 und DE 102009031021.5.

Als phosphoreszierende Verbindungen (Triplettemitter) eignen sich insbesondere Verbindungen, die bei geeigneter Anregung Licht bzw. Strahlung, bspw. im sichtbaren Bereich und/oder ultravioletten Bereich und/oder im infraroten Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Phosphoreszierende Metallkomplexe enthalten bevorzugt Ir, Ru, Pd, Pt, Os oder Re. Bevorzugte Liganden für phosphoreszierende Metallkomplexe sind 2-Phenylpyridin-Derivate, 7,8-Benzochinolin-Derivate, 2-(2-Thienyl)-pyridin-Derivate, 2-(1-Naphthyl)pyridin-Derivate oder 2-Phenylchinolin-Derivate. Alle diese Verbindungen können substituiert sein, z.B. für blau mit Fluor-, Cyano- und/oder Trifluormethylsubstituenten. Auxiliäre Liganden sind bevorzugt Acetylacetonat oder Picolinsäure.

Insbesondere sind Komplexe von Pt oder Pd mit tetradentaten Liganden, (US 2007/0087219), Pt-Porphyrinkomplexe mit vergrößertem Ringsystem (US 2009/0061681 A1) und Ir-Komplexe geeignet, z.B. 2,3,7,8,12,13,17, 18-Octaethyl-21H, 23H-porphyrin-Pt(II), Tetraphenyl-Pt(II)-tetrabenzoporphyrin (US 2009/0061681), Cis-Bis(2-phenylpyridinato-N,C²')Pt(II), Cis-Bis(2-(2'-thienyl)pyridinato-N,C³')Pt(II), Cis-Bis-(2-(2'-thienyl)chinolinato-N,C⁵')Pt(II), (2-(4,6-Difluorophenyl)pyridinato-N,C²')Pt(II)(acetylacetonat), oder Tris(2-phenylpyridinato-N,C²')Ir(III) (= Ir(ppy)₃, grün), Bis(2-phenylpyridinato-N,C²)Ir(III)(acetylacetonat) (= Ir(ppy)₂acetylacetonat, grün, US 2001/0053462 A1, Baldo, Thompson et.al. Nature 403, (2000), 750-753), Bis(1-phenylisochinolinato-N,C²')(2-phenylpyridinato-N,C²')Iridium(III), Bis(2-phenylpyridinato-N,C²')(1-phenylisochinolinato-N,C²')Iridium(III), Bis(2-(2'-benzothienyl)pyridinato-N,C³')Iridium(III)(acetylacetonat), Bis(2-(4',6'-difluorophenyl)pyridinato-N,C²')Iridium(III)(piccolinat) (Flrpic, blau), Bis(2-(4',6'-difluorophenyl)pyridinato-N,C²')Ir(III)(tetrakis(1-pyrazoly)borat), Tris(2-(biphenyl-3-yl)-4-tertbutylpyridin)iridium(III), (ppz)₂Ir(5phdpym) (US 2009/0061681 A1), (45ooppz)₂Ir(5phdpym) (US 2009/0061681 A1), Derivate von 2-Phenylpyridin-Ir-Komplexen, wie z.B. PQIr (= Iridium(III)bis(2-phenyl-quinolyl-N,C²')acetylacetonat), Tris(2-phenylisochinolinato-N,C)Ir(III) (rot), Bis(2-(2'-benzo[4,5-a]thienyl)-pyridinato-N,C³)Ir(acetylacetonat) ([Btₚ2lr(acac)], rot, Adachi et al. Appl. Phys. Lett. 78 (2001), 1622-1624).

Ebenfalls geeignet sind Kompexe von trivalenten Lanthaniden wie z.B: Tb³⁺ und Eu³⁺ (J.Kido et al. Appl.Phys.Lett. 65 (1994), 2124, Kido et al. Chem. Lett.657, 1990, US 2007-0252517 A1) oder phosphoreszente Komplexe von Pt(II), Ir(I), Rh(I) mit Maleonitrildithiolat (Johnson et al., JACS 105, 1983, 1795), Re(I)-Tricarbonyl-diimin-Komplexe (Wrighton, JACS 96, 1974, 998 u.a.), Os(II)-Komplexe mit Cyanoliganden und Bipyridyl- oder Phenanthrolin-Liganden (Ma et al., Synth. Metals 94, 1998, 245).

Weitere phosphoreszierende Emitter mit tridentaten Liganden werden beschrieben in US 6824895 und US 10/729238. Rot emittierende phosphoreszente Komplexe findet man in US 6835469 und US 6830828.

Die Verbindungen der Formel I, wie zuvor beschrieben, können in dieser Anwendung bevorzugt in Kombination mit einem oder mehreren weiteren fluoreszierenden Materialien (Singulettemitter) eingesetzt werden. Unter Fluoreszenz im Sinne dieser Erfindung wird die Lumineszenz aus einem angeregten Zustand mit niedriger Spinmultiplizität verstanden, also aus einem Spinzustand S = 1.

Als fluoreszierende Verbindungen (Singulettemitter) eignen sich insbesondere Verbindungen, die bei geeigneter Anregung Licht bzw. Strahlung, bspw. im sichtbaren Bereich und/oder ultravioletten Bereich und/oder im infraroten Bereich emittieren.

Bevorzugte Dotanden (Emitter) sind ausgewählt aus der Klasse der Monostyrylamine, der Distyrylamine, der Tristyrylamine, der Tetrastyrylamine, der Styrylphosphine, der Styrylether und der Arylamine.

Unter einem Monostyrylamin wird eine Verbindung verstanden, die eine substituierte oder unsubstituierte Styrylgruppe und mindestens ein, bevorzugt aromatisches, Amin enthält. Unter einem Distyrylamin wird eine Verbindung verstanden, die zwei substituierte oder unsubstituierte Styrylgruppen und mindestens ein, bevorzugt aromatisches, Amin enthält. Unter einem Tristyrylamin wird eine Verbindung verstanden, die drei substituierte oder unsubstituierte Styrylgruppen und mindestens ein, bevorzugt aromatisches, Amin enthält. Unter einem Tetrastyrylamin wird eine Verbindung verstanden, die vier substituierte oder unsubstituierte Styrylgruppen und mindestens ein, bevorzugt aromatisches, Amin enthält.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Eine weitere Ausführungsform der vorliegenden Erfindung in der speziellen Anwendung in elektronischen Vorrichtungen, bezieht sich auf Formulierungen enthaltend eine oder mehrere der erfindungsgemäßen Verbindungen sowie ein oder mehrere Lösungsmittel. Die Formulierung eignet sich hervorragend zum Erzeugen von Schichten aus Lösung.

Geeignete und bevorzugte Lösungsmittel sind beispielsweise Toluol, Anisol, Xylole, Methylbenzoat, Dimethylanisole, Trimethylbenzole, Tetralin, Veratrole, Tetrahydrofuran, Chlorbenzol oder Dichlorbenzole sowie Gemische derselben.

Die erfindungsgemäße organische Elektrolumineszenzvorrichtung kann beispielsweise in Displays oder für Beleuchtungszwecke verwendet werden, aber auch für medizinische oder kosmetische Anwendungen. Die erfindungsgemäßen Verbindungen eignen sich zum Einsatz in lichtemittierenden Vorrichtungen. Somit sind diese Verbindungen sehr vielseitig einsetzbar. Einige der Hauptanwendungsgebiete sind dabei Display- oder Beleuchtungs-Technologien. Weiterhin ist es besonders vorteilhaft, die Verbindungen sowie Vorrichtungen enthaltend diese Verbindungen im Bereich der Phototherapie einzusetzen.

Phototherapie oder Lichttherapie findet in vielen medizinischen und/oder kosmetischen Bereichen Anwendung. Die erfindungsgemäßen Verbindungen sowie die Vorrichtungen enthaltend diese Verbindungen können daher zur Therapie und/oder Prophylaxe und/oder Diagnose von allen Erkrankungen und/oder in kosmetischen Anwendungen eingesetzt werden, für die der Fachmann die Anwendung von Phototherapie in Betracht zieht. Der Begriff Phototherapie beinhaltet dabei neben der Bestrahlung auch die photodynamische Therapie (PDT) sowie das Desinfizieren und Sterilisieren im Allgemeinen. Behandelt werden können mittels Phototherapie oder Lichttherapie nicht nur Menschen oder Tiere, sondern auch jegliche andere Art lebender oder unbelebter Materie. Hierzu gehören, beispielsweise Pilze, Bakterien, Mikroben, Viren, Eukaryonten, Prokaryonten, Nahrungsmittel, Getränke, Wasser und Trinkwasser.

Der Begriff Phototherapie beinhaltet auch jede Art der Kombination von Lichttherapie und anderen Therapiearten, wie bspw. die Behandlung mit Wirkstoffen. Viele Lichttherapien haben zum Ziel, äußere Partien eines Objektes zu bestrahlen oder zu behandeln, so wie die Haut von Menschen und Tieren, Wunden, Schleimhäute, Auge, Haare, Nägel, das Nagelbett, Zahnfleisch und die Zunge. Die erfindungsgemäße Behandlung oder Bestrahlung kann daneben auch innerhalb eines Objektes durchgeführt werden, um bspw. innere Organe (Herz, Lunge etc.) oder Blutgefäße oder die Brust zu behandeln.

Die erfindungsgemäßen therapeutischen und/oder kosmetischen Anwendungsgebiete sind bevorzugt ausgewählt aus der Gruppe der Hauterkrankungen und Haut-assoziierten Erkrankungen oder Veränderungen bzw. Umstände wie bspw. Psoriasis, Hautalterung, Hautfaltenbildung, Hautverjüngung, vergrößerte Hautporen, Cellulite, ölige/fettige Haut, Follikulitis, aktinische Keratose, precancerose aktinische Keratose, Haut Läsionen, sonnengeschädigte und sonnengestresste Haut, Krähenfüße, Haut Ulkus, Akne, Akne rosacea, Narben durch Akne, Akne Bakterien, Photomodulierung fettiger/öliger Talgdrüsen sowie deren umgebende Gewebe, Ikterus, Neugeborenenikterus, Vitiligo, Hautkrebs, Hauttumore, Crigler Naijar, Dermatitis, atopische Dermatitis, diabetische Hautgeschwüre sowie Desensibilisierung der Haut.

Besonders bevorzugt im Sinne der Erfindung sind die Behandlung und/oder Prophylaxe von Psoriasis, Akne, Cellulite, Hautfaltenbildung, Hautalterung, Ikterus und Vitiligo.

Weitere erfindungsgemäße Anwendungsgebiete für die Zusammensetzungen und/oder Vorrichtungen enthaltend die erfindungsgemäßen Zusammensetzungen sind ausgewählt aus der Gruppe der Entzündungserkrankungen, rheumatoide Arthritis, Schmerztherapie, Behandlung von Wunden, neurologische Erkrankungen und Umstände, Ödeme, Paget's Erkrankung, primäre und metastasierende Tumoren, Bindegewebserkrankungen bzw. -Veränderungen, Veränderungen des Kollagens, Fibroblasten und von Fibroblasten stammende Zellspiegel in Geweben von Säugetieren, Bestrahlung der Retina, neovasculare und hypertrophe Erkrankungen, allergische Reaktionen, Bestrahlung der Atemwege, Schwitzen, okulare neovaskulare Erkrankungen, virale Infektionen besonders Infektionen durch Herpes Simplex oder HPV (Humane Papillomviren) zur Behandlung von Warzen und Genitalwarzen. Besonders bevorzugt im Sinne der Erfindung sind die Behandlung und/oder Prophylaxe von rheumatoider Arthritis, viraler Infektionen, und Schmerzen.

Weitere erfindungsgemäße Anwendungsgebiete für die Verbindungen und/oder Vorrichtungen enthaltend die erfindungsgemäßen Verbindungen sind ausgewählt aus der Winterdepression, Schlafkrankheit, Bestrahlung zur Verbesserung der Stimmung, Linderung von Schmerzen besonders Muskelschmerzen durch bspw. Verspannungen oder Gelenkschmerzen, Beseitigung der Steifheit von Gelenken und das Aufhellen der Zähne (Bleaching).

Weitere erfindungsgemäße Anwendungsgebiete für die Verbindungen und/oder Vorrichtungen enthaltend die erfindungsgemäßen Verbindungen sind ausgewählt aus der Gruppe der Desinfektionen. Mit den erfindungsgemäßen Verbindungen und/oder mit den erfindungsgemäßen Vorrichtungen können jegliche Art von Objekten (unbelebte Materie) oder Subjekten (lebende Materie wie bspw. Mensch und Tier) zum Zweck der Desinfektion, Sterilisation oder Konservierung behandelt werden. Hierzu zählt, zum Beispiel, die Desinfektion von Wunden, die Reduktion von Bakterien, das Desinfizieren chirurgischer Instrumente oder anderer Gegenstände, das Desinfizieren oder Konservieren von Nahrungs- und Lebensmitteln, von Flüssigkeiten, insbesondere Wasser, Trinkwasser und andere Getränke, das Desinfizieren von Schleimhäuten und Zahnfleisch und Zähnen. Unter Desinfektion wird hierbei die Reduktion lebender mikrobiologischer Verursacher unerwünschter Effekte, wie Bakterien und Keime, verstanden.

Zu dem Zweck der oben genannten Phototherapie emittieren Vorrichtungen enthaltend die erfindungsgemäßen Verbindungen bevorzugt Licht der Wellenlänge zwischen 250 and 1250 nm, besonders bevorzugt zwischen 300 and 1000 nm und insbesondere bevorzugt zwischen 400 and 850 nm. In einer besonders bevorzugten Ausführungsform der vorliegenden Erfndung werden die erfindungsgemäßen Verbindungen in einer organischen lichtemittierenden Diode (OLED) oder einer organischen lichtemittierenden elektrochemischen Zelle (OLEC) zum Zwecke der Phototherapie eingesetzt. Sowohl die OLED als auch die OLEC können dabei einen planaren oder Fiber- bzw. Faser-artigen Aufbau mit beliebigem Querschnitt (z.B. rund, oval, polygonal, quadratisch) mit einem ein- oder mehrschichtigen Aufbau aufweisen. Diese OLECs und/oder OLEDs können in andere Vorrichtungen eingebaut werden, die weitere mechanische, adhäsive und/oder elektronische Bausteine (z.B. Batterie und/oder Steuerungseinheit zur Einstellung der Bestrahlungszeiten, -intensitäten und -wellenlängen) enthalten. Diese Vorrichtungen enthaltend die erfindungsgemäßen OLECs und/order OLEDs sind vorzugsweise ausgewählt aus der Gruppe enthaltend Pflaster, Pads, Tapes, Bandagen, Manschetten, Decken, Hauben, Schlafsäcken,Tetilien und Stents.

Die Verwendung von den genannten Vorrichtungen zu dem genannten therapeutischen und/oder kosmetischen Zweck ist besonders vorteilhaft gegenüber dem Stand der Technik, da mit Hilfe der erfindungsgemäßen Vorrichtungen unter Verwendung der OLEDs und/oder OLECs homogene Bestrahlungen geringerer Bestrahlungsintensitäten an nahezu jedem Ort und zu jeder Tageszeit möglich sind. Die Bestrahlungen können stationär, ambulant und/oder selbst, d.h., ohne Einleitung durch medizinisches oder kosmetisches Fachpersonal durchgeführt werden. So können, bspw., Pflaster unter der Kleidung getragen werden, so dass eine Bestrahlung auch während der Arbeitszeit, in der Freizeit oder während des Schlafes möglich ist. Auf aufwendige stationäre/ambulante Behandlungen mit kann in vielen Fällen verzichtet bzw. deren Häufigkeit reduziert werden. Die erfindungsgemäßen Vorrichtungen können zum Widergebrauch gedacht sein oder Wegwerfartikel darstellen, die nach ein-, zwei oder dreimaligem Gebrauch entsorgt werden können.

Weitere Vorteile gegenüber dem Stand der Technik sind bspw. eine geringere Wärmeentwicklung und emotionale Aspekte. So werden Neugeborene, die aufgrund einer Gelbsucht (Ikterus) therapiert werden müssen typischerweise mit verbundenen Augen in einem Brutkasten, ohne körperlichen Kontakt zur den Eltern bestrahlt, was eine emotionale Stresssituation für Eltern und Neugeborene darstellt. Mit Hilfe einer erfindungsgemäßen Decke enthaltend die erfindungsgemäßen OLEDs und/oder OLECs kann der emotionale Stress signifikant vermindert werden. Zudem ist eine bessere Temperierung des Kindes durch eine verringerte Wärmeproduktion der erfindungsgemäßen Vorrichtungen gegenüber herkömmlicher Bestrahlungsgeräte möglich.

Auch ohne weitere Ausführungen wird davon ausgegangen, dass ein Fachmann die obige Beschreibung in weitestem Umfang nutzen kann. Die bevorzugten Ausführungsformen und Beispiele sind deswegen lediglich als beschreibende, keinesfalls als in irgendeiner Weise limitierende Offenbarung aufzufassen. Die Gewichtsprozentverhältnisse der einzelnen Inhaltsstoffe in den Zubereitungen der Beispiele gehören ausdrücklich zur Offenbarung der Beschreibung und können daher als Merkmale herangezogen werden.

Weitere wichtige Merkmale und Vorteile der Erfindung ergeben sich aus den Unteransprüchen und aus den Beispielen.

### Beispiele:

### Beispiel 1: Synthese von (4Z,6Z)-2-Ethoxy-4,6-bis[(4-methoxyphenyl)methylen)-2-methyl-1, 3-dioxan-5-on

Es werden 1,96 g Kaliumhydroxid (34,89 mmol; 2,24 Äq.) in 20 ml Ethanol vorgelegt. Das in 10 ml Ethanol gelöste 2-Ethoxy-2-methyl-1,3-dioxan-5-on (2,5 g; 15,61 mmol; 1 Äq.) wird zugegeben und anschließend 4,24 g 4-Methoxybenzaldehyd (34,89 mmol, 2,34 Äq.) innerhalb von 30 min zugetropft. Anschließend wird für 3 Stunden zum Rückfluss erhitzt. Das Ethanol wird abdestilliert und nach abkühlen auf Raumtemperatur und der Zugabe von 50 ml Wasser fällt ein Feststoff aus, der mit 10 ml Wasser gewaschen wird. Der erhaltene Feststoff wird in 85 ml Ethanol in der Siedehitze umkristallisiert. Nach Trocknung erhält man das Produkt als gelbe Kristalle.
¹H-NMR (400 MHz, DMSO-d₆) δ = 7.81 (d, 4x Ar-H), 7.01 (d, 4x Ar-H), 6.82 (s, 2x C=C*H*Ar), 3.81 (s, 2x Ar-C*H*₃), 3.60 (q, O-C*H*₂-), 1.97 (s, C-C*H*₃), 1.04 (t, CH₂-C*H*₃).

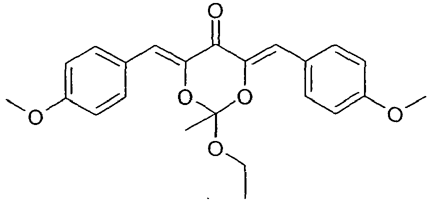

### Beispiel 2:

Analog zu den Reaktionsbedingungen des Beispiels 1 wird 2-Ethoxy-2-methyl-1,3-dioxan-5-on mit 3,4-Dimethoxybenzaldehyd umgesetzt.
Man erhält (4Z,6Z)-4,6-Bis[3,4-dimethoxyphenyl]methylen]-2-ethoxy-2-methyl-1,3-dioxan-5-on als gelbe Kristalle.
¹H-NMR (400 MHz, DMSO-d₆) δ = 7.50 (d, 2x Ar-H), 7.44 (dd, 2x Ar-H), 7.03 (d, 2x Ar-H), 6.93 (s, 2x C=C*H*Ar), 3.81 (s, 4x Ar-C*H*₃), 3.61 (q, O-C*H*₂-), 1.98 (s, C-C*H*₃), 1.03 (t, CH₂-C*H*₃).

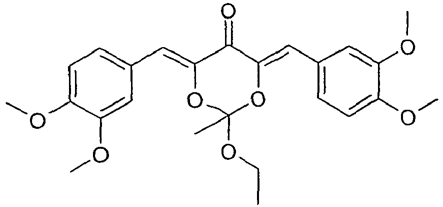

### Beispiel 3:

Analog zu den Reaktionsbedingungen des Beispiels 1 wird 2-Ethoxy-2-methyl-1,3-dioxan-5-on mit 2,4-Dimethoxybenzaldehyd umgesetzt.
Man erhält (4Z,6Z)-4,6-Bis[2,4-dimethoxyphenyl]methylen]-2-ethoxy-2-methyl-1,3-dioxan-5-on als gelbe Kristalle.
¹H-NMR (400 MHz, DMSO-d₆) δ = 8.03 (d, 2x Ar-H), 7.14 (s, 2x Ar-H), 6.63 (m, 2x Ar-H, 2x C=C*H*Ar), 3.87 (s, 2x Ar-C*H*₃), 3.82 (s, 2x Ar-C*H*₃), 3.56 (q, O-C*H*₂-), 1.93 (s, C-C*H*₃), 1.03 (t, CH₂-C*H*₃).

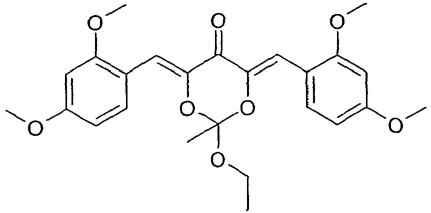

### Beispiel 4:

Analog zu den Reaktionsbedingungen des Beispiels 1 wird 2-Ethoxy-2-methyl-1,3-dioxan-5-on mit 2,4,5-Trimethoxybenzaldehyd umgesetzt.
Man erhält (4Z,6Z)-4,6-Bis[2,4,5-trimethoxyphenyl]methylen]-2-ethoxy-2-methyl-1,3-dioxan-5-on als orangefarbene Kristalle.
¹H-NMR (400 MHz, DMSO-d₆) δ = 7.71 (s, 2x Ar-H), 7.16 (s, 2x Ar-H), 6.74 (s, 2x C=C*H*Ar), 3.87 (s, 2x Ar-C*H*₃), 3.83 (s, 2x Ar-C*H*₃), 3.74 (s, 2x Ar-C*H*₃), 3.59 (q, O-C*H*₂-), 1.94 (s, C-C*H*₃), 1.03 (t, CH₂-C*H*₃).

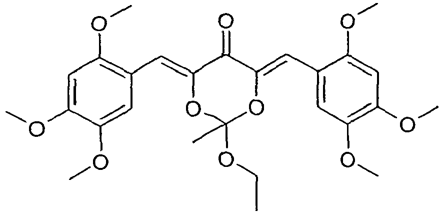

### Beispiel 5:

Analog zu den Reaktionsbedingungen des Beispiels 1 wird 2-Ethoxy-2-methyl-1,3-dioxan-5-on mit 3,4,5-Trimethoxybenzaldehyd umgesetzt. Man erhält (4Z,6Z)-4,6-Bis[3,4,5-trimethoxyphenyl]methylen]-2-ethoxy-2-methyl-1,3-dioxan-5-on als gelbe Kristalle.
¹H-NMR (400 MHz, DMSO-d₆) δ = 7.23 (s, 4x Ar-H), 6.85 (s, 2x C=C*H*Ar), 3.82 (s, 4x mAr-C*H*₃), 3.72 (s, 2x pAr-C*H*₃), 3.62 (q, O-C*H*₂-), 1.99 (s, C-C*H*₃), 1.05 (t, CH₂-C*H*₃).

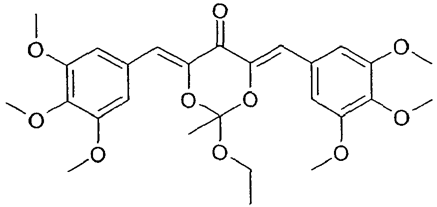

### Beispiel 6:

Analog zu den Reaktionsbedingungen des Beispiels 1 wird 2-Ethoxy-2-methyl-1,3-dioxan-5-on mit 4-Ocyloxybenzaldehyd umgesetzt.
Man erhält (4Z,6Z)-4,6-Bis[(4-Octyloxyphenyl)methylen]-2-ethoxy-2-methyl-1,3-dioxan-5-on als gelbe Kristalle.
¹H-NMR (400 MHz, DMSO-d₆) δ = 7.79 (d, 4x Ar-H), 7.00 (d, 4x Ar-H), 6.81 (s, 2x C=C*H*Ar), 4.02 (t, 2x CH₂), 3.60 (q, O-C*H*₂-), 1.97 (s, C-C*H*₃), 1.73 (m, 2x CH₂), 1.47-1.20 (m, 10x CH₂), 1.05 (t, CH₂-C*H*₃), 0.86 (t, 2x CH₃).

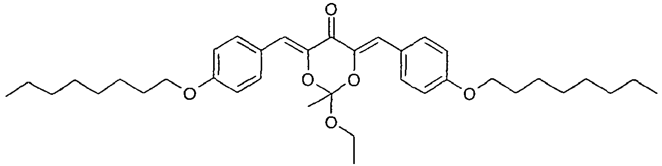

### Beispiel 7:

Analog zu den Reaktionsbedingungen des Beispiels 1 wird 2-Ethoxy-2-methyl-1,3-dioxan-5-on mit 4-Phenoxybenzaldehyd umgesetzt.
Man erhält (4Z,6Z)-4,6-Bis[(4-phenoxyyphenyl)methylen]-2-ethoxy-2-methyl-1,3-dioxan-5-on als gelbe Kristalle.
¹H-NMR (400 MHz, DMSO-d₆) δ = 7.88 (d, 4x r-H), 7.43 (m, 4x Ar-H), 7.19 (m, 2x Ar-H), 7.08 (m, 4x Ar-H), 7.01 (d, 4x Ar-H), 6.85 (s, 2x C=C*H*Ar), 3.61 (q, O-C*H*₂-), 1.96 (s, C-C*H*₃), 1.04 (t, CH₂-C*H*₃).

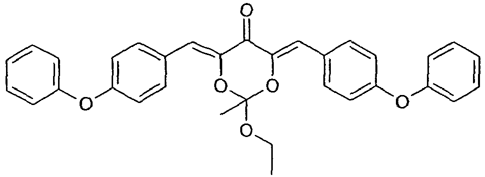

### Beispiel 8:

Analog zu den Reaktionsbedingungen des Beispiels 1 wird 2-Ethoxy-2-methyl-1,3-dioxan-5-on mit 4-Dibutylaminobenzaldehyd umgesetzt.
Man erhält (4Z,6Z)-4,6-Bis[[(4-dibutylamino)phenyl]methylen]-2-ethoxy-2-methyl-1,3-dioxan-5-on als rote Kristalle.
¹H-NMR (400 MHz, DMSO-d₆) δ = 7.64 (d, 4x Ar-H), 6.70 (s, 2x C=C*H*Ar), 6.66 (d, 4x Ar-H), 3.58 (q, O-C*H*₂-), 1.92 (s, C-C*H*₃), 1.52 (m, 4x CH₂), 1.33 (m, 4x CH₂), 1.03 (t, CH₂-C*H*₃), 0.92 (t, 4x CH₃).

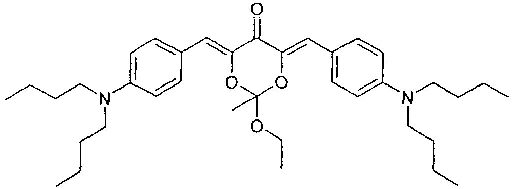

### Beispiel 9:

Analog zu den Reaktionsbedingungen des Beispiels 1 wird 2-Ethoxy-2-methyl-1,3-dioxan-5-on mit 4-Fluorbenzaldehyd umgesetzt.
Man erhält 2-Ethoxy-2,6-bis-[1-(4-fluor-phenyl)-meth-(Z)-yliden]-2-methyl-[1,3]-dioxan-5-on als gelbe Kristalle.
¹H-NMR (300 MHz, DMSO-d₆) δ = 7.90 (dd, 4x Ar-H), 7.27 (t, 4x Ar-H), 6.87 (s, 2x C=C*H*Ar), 3.59 (q, O-C*H*₂-), 1.99 (s, C-C*H*₃), 1.02 (t, CH₂-C*H*₃).

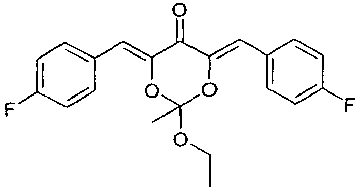

### Beispiel 10:

Analog zu den Reaktionsbedingungen des Beispiels 1 wird 2-Ethoxy-2-methyl-1,3-dioxan-5-on mit 4-Trifluormethylbenzaldehyd umgesetzt.
Man erhält 2-Ethoxy-2,6-bis-[1-(4-trifluormethyl-phenyl)-meth-(Z)-yliden]-2-methyl-[1,3]-dioxan-5-on als gelbe Kristalle.
¹H-NMR (300 MHz, DMSO-d₆) δ = 8.03 (d, 4x Ar-H), 7.79 (t, 4x Ar-H), 6.96 (s, 2x C=C*H*Ar), 3.63 (q, O-C*H*₂-), 2.03 (s, C-C*H*₃), 1.03 (t, CH₂-C*H*₃).

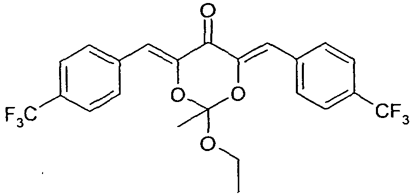

### Beispiel 11:

Analog zu den Reaktionsbedingungen des Beispiels 1 wird 2-Ethoxy-2-methyl-1,3-dioxan-5-on mit 2,4,6-Trimethoxybenzaldehyd umgesetzt.
Man erhält 2-Ethoxy-2,6-bis-[1-(2,4,6-trimethoxy-phenyl)-meth-(Z)-yliden]-2-methyl-[1,3]-dioxan-5-on als gelbe Kristalle.
¹H-NMR (300 MHz, DMSO-d₆) δ = 6.60 (s, 4x Ar-H), 6.26 (s, 2x C=C*H*Ar), 3.81 (s, p-MeO-Ar), 3.78 (s, 2x o-MeO-Ar), 3.62 (q, O-C*H*₂-), 1.60 (s, C-C*H*₃), 1.08 (t, CH₂-C*H*₃).

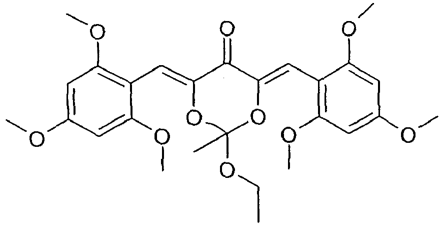

### Beispiel 12:

Analog zu den Reaktionsbedingungen des Beispiels 1 wird 2-Ethoxy-2-methyl-1,3-dioxan-5-on mit 2,3,4-Trimethoxybenzaldehyd umgesetzt.
Man erhält 2-Ethoxy-2,6-bis-[1-(2,3,4,-trimethoxy-phenyl)-meth-(Z)-yliden]-2-methyl-[1,3]-dioxan-5-on als gelbe Kristalle.
¹H-NMR (300 MHz, DMSO-d₆) δ = 7.85 (d, 4x Ar-H), 7.05 (s, 2x C=C*H*Ar), 6.91 (d, 4x Ar-H), 3.85 (s, MeO-Ar), 3.84 (s, MeO-Ar), 3.77 (s, MeO-Ar), 3.59 (q, O-C*H*₂-), 1.94 (s, C-C*H*₃), 1.04 (t, CH₂-C*H*₃).

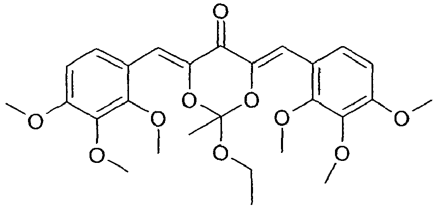

### Beispiel 13:

Analog zu den Reaktionsbedingungen des Beispiels 1 wird 2-Ethoxy-2-methyl-1,3-dioxan-5-on mit Biphenyl-4-carbaldehyd umgesetzt.
Man erhält 2-Ethoxy-2,6-bis-[1-biphenyl-4-yl-meth-(Z)-yliden]-2-methyl-[1,3]-dioxan-5-on als gelbe Kristalle.
¹H-NMR (300 MHz, DMSO-d₆) δ = 7.96 (d, 4x Ar-H), 7.75 (t, 8x Ar-H), 7.49 (t, 4x Ar-H), 7.41 (m, 4x Ar-H), 6.94 (s, 2x C=C*H*Ar), 3.66 (q, O-C*H*₂-), 2.05 (s, C-C*H*₃), 1.06 (t, CH₂-C*H*₃).

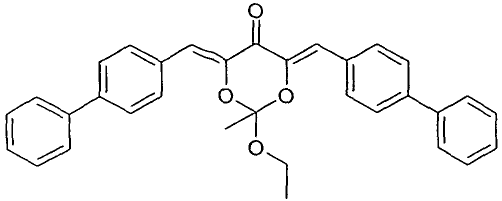

### Beispiel 14:

Analog zu den Reaktionsbedingungen des Beispiels 1 wird 2-Ethoxy-2-methyl-1,3-dioxan-5-on mit Naphthalin-2-carbaldehyd umgesetzt.
Man erhält 2-Ethoxy-2,6-bis-[1-naphthalin-2-yl-meth-(Z)-yliden]-2-methyl-[1,3]-dioxan-5-on als gelbe Kristalle.
¹H-NMR (300 MHz, DMSO-d₆) δ = 8.34 (s, 2x Ar-H), 7.92-8.03 (m, 8x Ar-H), 7.55-7.59 (m, 4x Ar-H), 7.05 (s, 2x C=C*H*Ar), 3.69 (q, O-C*H*₂-), 2.13 (s, C-C*H*₃), 1.05 (t, CH₂-C*H*₃).

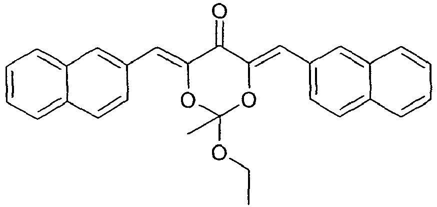

### Beispiel 15:

Analog zu den Reaktionsbedingungen des Beispiels 1 wird 2-Ethoxy-2-methyl-1,3-dioxan-5-on mit 1-Methylindol-3-carbaldehyd umgesetzt.
Man erhält 2-Ethoxy-2,6-bis-[1-(1-methyl-1-H-indol-3-yl)-meth-(Z)-yliden]-2-methyl-[1,3]-dioxan-5-on als orangefarbene Kristalle.
¹H-NMR (300 MHz, DMSO-d₆) δ = 8.02 (s, 2x Ar-H), 7.86 (d, 2x Ar-H), 7.52 (d, 2x Ar-H), 7.16-7.31 (m, 4x Ar-H), 7.22 (s, 2x C=C*H*Ar), 3.91 (s, 2x N-C*H*₃), 3.63 (q, O-C*H*₂-), 2.11 (s, C-C*H*₃), 1.04 (t, CH₂-C*H*₃).

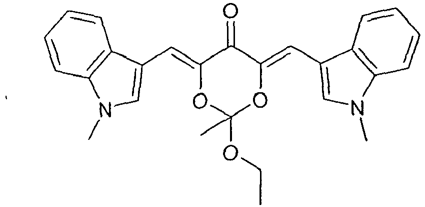

### Beispiel 16:

Analog zu den Reaktionsbedingungen des Beispiels 1 wird 2-Ethoxy-2-methyl-1,3-dioxan-5-on mit 9-Ethyl-carbazol-3-carbaldehyd umgesetzt. Man erhält 2-Ethoxy-2,6-bis-[1-(9-ethyl-9H-carbazol-3-yl)-meth-(Z)-yliden]-2-methyl-[1,3]-dioxan-5-on als orangefarbene Kristalle.
¹H-NMR (300 MHz, DMSO-d₆) δ = 8.56 (d, 2x Ar-H), 8.21 (d, 2x Ar-H), 8.03 (dd, 2x Ar-H), 7.67 (*t*, 2x Ar-H), 7.51 (dt, 2x Ar-H), 7.27 (dt, 2x Ar-H), 7.10 (s, 2x C=C*H*Ar), 4.48 (q, N-C*H*₂-CH₃), 3.71 (q, O-C*H*₂-), 2.16 (s, C-C*H*₃), 1.35 (t, N-CH₂-C*H*₃), 1.06 (t, CH₂-C*H*₃).

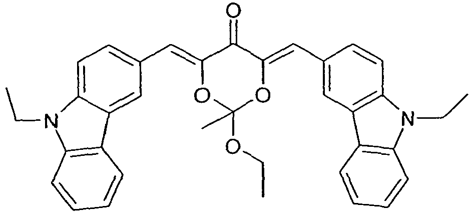

### Beispiel 17:

Analog zu den Reaktionsbedingungen des Beispiels 1 wird 2-Ethoxy-2-methyl-1,3-dioxan-5-on mit 4-Dimethylamino-2-methoxy-benzaldehyd umgesetzt.
Man erhält 2-Ethoxy-2,6-bis-[1-(4-dimethylamino-2-methoxy-phenyl)-meth-(Z)-yliden]-2-methyl-[1,3]-dioxan-5-on als rote Kristalle.
¹H-NMR (300 MHz, DMSO-d₆) δ = 7.95 (d, 2x Ar-H), 7.15 (s, 2x C=C*H*Ar), 6.37 (dd, 2x Ar-H), 6.26 (dd, 2x Ar-H), 3.86 (s, 2x MeO-Ar) 3.55 (q, O-C*H*₂-), 3.00 (s, 2x Me₂N), 1.88 (s, C-C*H*₃), 1.01 (t, CH₂-C*H*₃).

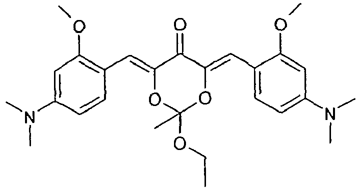

### Beispiel 18:

Analog zu den Reaktionsbedingungen des Beispiels 1 wird 2-Ethoxy-2-methyl-1,3-dioxan-5-on mit 4-Dimethylamino-naphthalin-1-carbaldehyd umgesetzt.
Man erhält 2-Ethoxy-2,6-bis-[1-(4-dimethylamino-naphthalin-1-yl)-meth-(Z)-yliden]-2-methyl-[1,3]-dioxan-5-on als rote Kristalle.
¹H-NMR (300 MHz, DMSO-d₆) δ = 8.24 (d, 2x Ar-H), 8.16-8.22 (m, 4x Ar-H), 7.58 (s, 2x C=C*H*Ar), 7.46.-7.68 (dd, 2x Ar-H), 7.19 (d, 2x Ar-H), 3.65 (q, O-C*H*₂-), 2.91 (s, 2x Me₂N), 1.96 (s, C-C*H*₃), 1.05 (t, CH₂-C*H*₃).

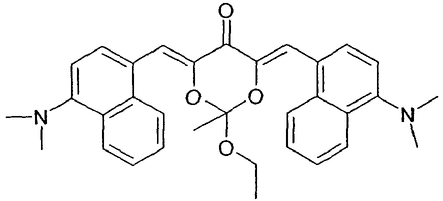

### Beispiel 19:

Analog zu den Reaktionsbedingungen des Beispiels 1 wird 2-Ethoxy-2-methyl-1,3-dioxan-5-on mit 4-Brom-benzaldehyd umgesetzt.
Man erhält 2-Ethoxy-2,6-bis-[1-(4-brom-phenyl)-meth-(Z)-yliden]-2-methyl-[1,3]-dioxan-5-on als gelbe Kristalle.
¹H-NMR (300 MHz, DMSO-d₆) δ = 7.79 (d, 4x Ar-H), 7.63 (d, 4x Ar-H), 6.86 (s, 2x C=C*H*Ar), 3.60 (q, O-C*H*₂-), 2.00 (s, C-C*H*₃), 1.02 (t, CH₂-C*H*₃).

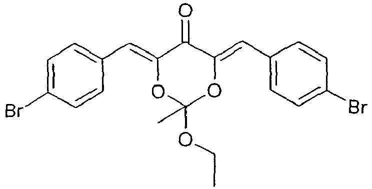

### Beispiel 20:

Analog zu den Reaktionsbedingungen des Beispiels 1 wird 2-Ethoxy-2-methyl-1,3-dioxan-5-on mit Thiophen-2-carbaldehyd umgesetzt.
Man erhält 2-Ethoxy-2-methyl-4,6-bis-[1-thiophen-2-yl-meth-(Z)-yliden]-[1,3] dioxan-5-on.

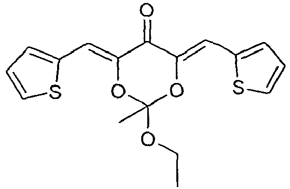

### Beispiel 21:

Analog zu den Reaktionsbedingungen des Beispiels 1 wird 2-ethoxy-2-methyl-1,3-dioxan-5-on mit einem Gemisch aus 4-Methoxy-benzaldehyd und 4-(Dibutylamino)-benzaldehyd umgesetzt. Die erhaltene Produktmischung wird chromatographisch abgetrennt und man erhält insbesondere die Verbindung 4-[1-(4-Dibutylamino-phenyl)-meth-(Z)-ylliden]-2-ethoxy-6-[1-(4-methoxy-phenyl)-meth-(Z)-yliden]-2-methyl-[1,3]dioxan-5-on.
¹H-NMR (300 MHz, DMSO-d₆) δ = 7.78 (d, 2x Ar-H), 7.67 (d, 2x Ar-H), 7.00 (d, 2x Ar-H), 6.67 (s, 2x C=C*H*Ar), 3.79 (s, Ar-OMe), 3.58 (q, O-C*H*₂-), 3.35 (m, 4xCH₂) 1.94 (s, C-C*H*₃), 1.52 (m, 2xCH₂), 1.42 (m, 2xCH₂), 1.03 (t, CH₂-C*H*₃), 0.91 (t, 2x CH₃).

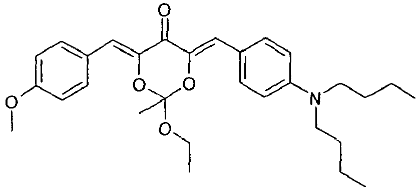

### Beispiel 22:

Analog zu den Reaktionsbedingungen des Beispiels 1 wird 2-Ethoxy-2-methyl-1,3-dioxan-5-on mit 5-Isopropyl-3,8-dimethyl-azulen-1-carbaldehyd umgesetzt.
Man erhält 2-Ethoxy-4,6-bis-[1-(5-isopropyl-3,8-dimethyl-azulen-1-yl)-meth-(Z)-yliden]-2-methyl-[1,3]dioxan-5-on.
¹H-NMR (300 MHz, CDCl₃) δ = 8.41 (s, 2x Ar-H), 8.07 (d, 2x Ar-H), 7.35 (d, 2x Ar-H), 7.04 (s, 2x C=C*H*Ar), 3.72 (q, O-C*H*₂-), 3.15 (s, 2x Ar-Me), 3.01 (m, 2x C*H*(CH₃)₂), 2.59 (s, 2x Ar-Me), 2.05 (s, C-C*H*₃), 1.25 (s, 2xCH(C*H*₃)₂), 1.13 (t, CH₂-C*H*₃).

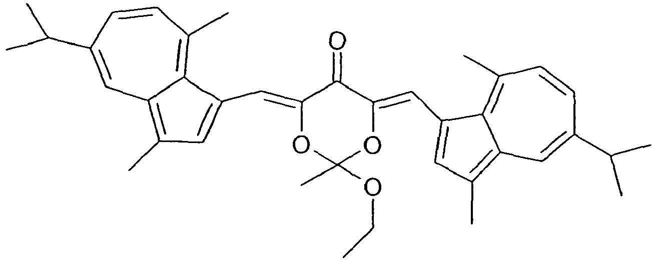

### Beispiel 23:

Analog zu den Reaktionsbedingungen des Beispiels 1 wird 2-Ethoxy-2-methyl-1,3-dioxan-5-on mit 4-Carbazol-9-yl-benzaldehyd umgesetzt.
Man erhält 4,6-Bis-[1-(4-Carbazol-9-yl-phenyl)-meth-(Z)-yliden]-2-ethoxy-2-methyl-[1,3]dioxan-5-on.

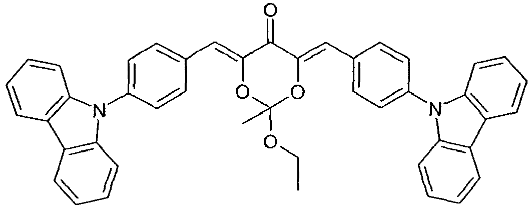

### Beispiel 24:

Analog zu den Reaktionsbedingungen des Beispiels 1 wird 2-Ethoxy-2-methyl-1,3-dioxan-5-on mit [1,1',3',1"]Terphenyl-2'-carbaldehyd umgesetzt. Der Aldehyd kann entsprechend nach der Beschreibung von Bahaaldin Rashidzadeh et al, ARKIVOC 2008 (xvii) 167-172 synthetisiert werden. Man erhält 2-Ethoxy-2-methyl-4,6-bis-[1-[1,1';3',1"]terphenyl-2'-yl-meth-(Z)-yliden]-[1,3]dioxan-5-on. Ph = Phenyl.

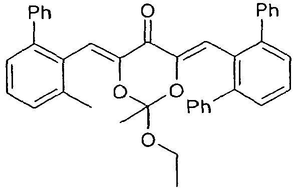

### Beispiel 25:

Analog zu den Reaktionsbedingungen des Beispiels 1 wird 2-Ethoxy-2-methyl-1,3-dioxan-5-on mit 3-(di-p-tolyl-amino)-benzaldehyd umgesetzt. Man erhält 4,6-Bis-[1-[3-(di-p-tolyl-amino)-phenyl]-meth-(Z)-yliden]-2-methoxy-2-methyl-[1,3]dioxan-5-on.

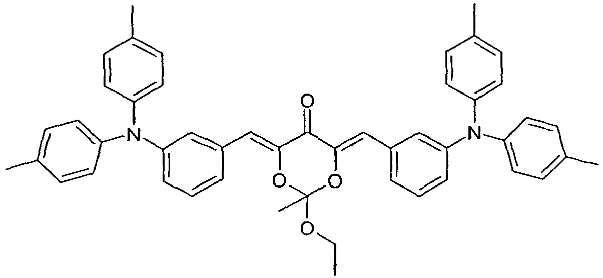

### Beispiel A: Absorptionskraft

Als Maß für die Absorptionskraft wird der so genannte E1%-Wert herangezogen, sowie die Halbwertsbreite der Absorptionsbande in Relation zum angegebenen Absorptionsmaximum λmax. Der E1%-Wert gibt die auf eine Konzentration von 1g/100mL hochgerechnetet Extinktion am Absorprionsmaximum an. Dazu wird in verdünnter Lösung ein Absortionsspektrum der Substanz in Ethanol aufgenommen. Die Werte werden mit dem Referenzspektren von Curcumin und beta-Carotin verglichen.

| Prüfsubstanz | λmax | E1%-Wert | Halbwertsbreite [nm] |
|---|---|---|---|
| nach Beispiel 1 | 402 | 873 | 84 |
| nach Beispiel 2 | 412 | 617 | 85 |
| nach Beispiel 3 | 419 | 688 | 93 |
| nach Beispiel 4 | 444 | 304 | 92 |
| nach Beispiel 5 | 394 | 647 | 85 |
| nach Beispiel 6 | 404 | 557 | 87 |
| nach Beispiel 7 | 389 | 675 | 84 |
| nach Beispiel 8 | 486 | 953 | 105 |
| Curcumin | 426 | 1507 | 78 |
| Beta-carotin | 453 | 408 | 88 |

### Beispiel B: Thermische Stabilität:

Die thermische Stabilität wird mit Hilfe des Thermographimetrieverfahrens bestimmt (Gerät TGA Q5000 V3.10 Build 258, Temperaturbereich RT bis 800°C, 10K/min Aufheizrate).

| Prüfsubstanz | Temperatur, bei der noch 98% der Masse vorlag |
|---|---|
| nach Beispiel 4 | 256°C |
| nach Beispiel 8 | 274°C |
| Curcumin (Vergleich) | 223°C |
| Beta-Carotin (Vergleich) | 84°C |

Die thermischen Stabilitäten sind hervorragend, so dass auch Hochtemperaturverarbeitungen der Farbstoffe der Formel I, beispielsweise der Verbindungen der Beispiele 4 und 8, wie z.B. die Einarbeitung in Kunststoffe, ohne Zersetzung möglich sind.

### Beispiel C: Fluoreszenzmessungen

Die in der Tabelle angegebenen Substanzkonzentrationen in Ethanol werden mit einem Fluoreszenzspektrometer der Marke Aminco Bowman 2 vermessen (Schichtdicke 1 cm; Anregung 220-600nm; Emission 220-800nm; Spektrale Spaltbreite (Anregung) 4nm, (Emission) 8 nm; Registiergeschwindigkeit 10nm/min; Schrittweite (Anregung) 5nm, (Emission) 4nm.

| Substanz | Messkonzentration | Anregung | Emission |
|---|---|---|---|
| nach Beispiel 4 | 0,00436 mg/mL | 440 nm | 596 nm |
| nach Beispiel 8 | 0,00048 mg/mL | 485 nm | 640 nm |
| nach Beispiel 17 | 0,00045 mg/mL | 490 nm | 632 nm |

### Beispiel D: Löslichkeiten

Die Löslichkeitsbestimmung wurde in Phenethylbenzoate (X-Tend 226)durchgeführt:

| Prüfsubstanz | Löslichkeit in X-Tend 226: |
|---|---|
| nach Beispiel 1 | 2,8% |
| nach Beispiel 2 | 1,2% |
| nach Beispiel 3 | 1,0% |
| nach Beispiel 4 | <0,1% |
| nach Beispiel 5 | 1,1% |
| nach Beispiel 6 | 1,0% |
| nach Beispiel 7 | 2,2% |
| nach Beispiel 8 | 2,8% |
| Curcumin | 0,4% |
| Beta-Carotin | 0,2% |

### Beispiel E: Hautfärbetest

Zur Färbung einer Probe humanen Stratum Corneums wird wie folgt vorgegangen: Ein ca. 0,3 cm² großes Stück humanen Stratum Corneums wird in einer 0.6%igen Lösung der Verbindung nach Beispiel 8 für 48h inkubiert (Lösungmittelgemisch:
Tetrahydrofuran/Ethylenglycol/Phosphatpuffer pH6 = 50/47/3). Dabei färbt sich das Hautstück intensiv orange-rot. Im Anschluß wird das Hautpräparat aus der Inkubationslösung entnommen und für eine Woche in Wasser gelagert. Dabei bleibt der visuelle Farbeindruck komplett erhalten. Es tritt kein merkliches Auswaschen der Farbe auf. Im weiteren Testverlauf wird das Hautstück aus dem Wasser entnommen und in zwei Stücke geteilt. Ein Teil wird dunkel gelagert, der andere Teil wird im Solarsimulator (Atlas CPS+) für zweimal eine Stunde mit simuliertem Sonnenlicht bewittert (Geräteeinstellung: 500Wm2). Im Vergleich zur dunkelgelagerten Probe ergibt sich keine visuell wahrnehmbare Farbveränderung.

### Formulierungsbeispiele:

### Beispiel A-1: Haarspülung

| | Gewichtsprozent [%] |
|---|---|
| Cetearyl Alcohol | 10 |
| Sunflowerseedamidopropyl Ethyldimonium | 0,5 |
| Ethosulfate | |
| Ceteareth-20 | 3,0 |
| Panthenol | 0,4 |
| Phenyl Trimethicone | 0,3 |
| Hydroxypropyl Guar Hydroxypropyltrimonium Chloride | 0,8 |
| Verbindung nach Beispiel 8 | 1,0 |
| Passiflora Incarnata Seed Oil | 0,2 |
| Basic red 51 | 0,1 |
| Basic red 76 | 0,2 |
| Parfüm | 1,0 |
| Konservierungsmittel | q.s. |
| Citric Acid / Sodium Hydroxide | q.s. to pH 5.5 |
| Aqua | ad 100 |

### Beispiel B-1: Shampoo

| | Gewichtsprozent [%] |
|---|---|
| Sodium Laureth Sulfate | 5,0 |
| Cocamidopropy Betaine | 5,0 |
| Lauroyl Glutamic Acid | 3,0 |
| Decyl Glucoside | 5,0 |
| Polyquaternium-10 | 0,5 |
| PEG-3 Distearate | 0,8 |
| Verbindung nach Beispiel 22 | 0,5 |
| Nachtkerzenöl | 0,3 |
| Basic Red 51 | 0,1 |
| Ubiquinone | 0,1 |
| Benzyl Alcohol | 0,5 |
| Parfüm | 1,0 |
| Konservierungsmittel | q.s. |
| Sodium Chloride | 0,8 |
| Citric Acid / Sodium Hydroxide | q.s. to pH 5.5 |
| Aqua | ad 100 |

### Beispiel C-1: Haarfärberezepturen

| | 1 | 2 | 3 | 4 | 5 | 6 | 7 |
|---|---|---|---|---|---|---|---|
| Benzyl Alcohol | 2,5 | | | | | | |
| Propylene Carbonate | 10 | | | | | | |
| Ethanol | 5,0 | | | | | | |
| Hydroxyethylcellulose | 2,0 | | | | | | |
| Pirenoxin NatriumCAS 51410-30-1 | 2,0 | | | | | | 2,0 |
| Tramsanguin CAS 34083-17-5 | | 1,0 | | | | 1,0 | |
| Cinnabarin CAS 606-59-7 | | | 1,0 | | | | |
| Cinnabarinsäure CAS 146-90-7 | | | | 1,0 | | | |
| Resorcinblau CAS 71939-12-3 | | | | | 1,0 | | |
| Verbindung nach Beispiel 22 | 1,0 | 1,0 | 2,0 | 1,0 | 1,0 | 0,5 | |
| Verbindung nach Beispiel 4 | | | | | | 1,5 | 2,0 |
| Parfüm | 1,0 | 1,0 | 1,0 | 1,0 | 1,0 | 1,0 | 1,0 |
| Konservierungsmittel | q.s. | q.s. | q.s. | q.s. | q.s. | q.s. | q.s. |
| Citric Acid | q.s. ad pH 5.5 | q.s. ad pH 5.5 | q.s. ad pH 5.5 | q.s. ad pH 5.5 | q.s. ad pH 5.5 | q.s. ad pH 5.5 | q.s. ad pH 5.5 |
| Aqua | ad 100 | ad 100 | ad 100 | ad 100 | ad 100 | ad 100 | ad 100 |

### Beispiel D-1: milde transparente W/O Bräunungslotion

| **Bestandteile / Handelsname** | **INCI** | **[Gew-%]** |
|---|---|---|
| **A** | | |
| Dow Corning 3225 C | CYCLOMETHICONE, DIMETHICONE COPOLYOL | 23.60 |
| Propyl-4-hydroxybenzoate | PROPYLPARABEN | 0.05 |
| Verbindung nach Beispiel 8 | | 0,25 |
| | | |

| **B** | | |
|---|---|---|
| Dihydroxyacetone | DIHYDROXYACETON E | 3.00 |
| Methyl-4-hydroxybenzoate | METHYLPARABEN | 0.15 |
| 1,2-Propanediol | PROPYLENE GLYCOL | 35.90 |
| Water, demineralized | AQUA (WATER) | 35.30 |
| **Gesamt** | | **100.00** |

### Beispiel E-1: Farbiges Duschgel

| Inhaltsstoff | INCI | Konzentration |
|---|---|---|
| Texapon NSO | Sodium Laureth Ether Sulfate | 10 % |
| Dehyton K | Cocamidopropyl Betaine | 3 % |
| Tagat L 2 | PEG-20 Glyceryl Laurate | 1 % |
| Beispiel 22 | | 0,001 % |
| Beispiel 8 | | 0,05% - 0,1% |
| Wasser | Aqua | ad 100 |

### Beispiel F-1: O-in-W-Emulsionen zum Schutz vor sichtbarer Strahlung

Prinzipiell kann alternativ zur Verwendung der in der Tabelle aufgeführten Beispielsubstanzen auch die Verwendung anderer Verbindungen der Formel I erfolgen.
Zahlen in Gew.-%

| | 2-1 | 2-2 | 2-3 | 2-4 | 2-5 | 2-6 | 2-7 | 2-8 | 2-9 | 2-10 |
|---|---|---|---|---|---|---|---|---|---|---|
| Titanium dioxide | | 2 | 5 | | | | | | | 3 |
| Methylene Bis-Benztriazolyl Tetramethylbutylphenol | | | | | | 1 | 2 | 1 | | |
| Beispiel 8 | 0,5 | 1 | 2 | 3 | 5 | | | | 1 | 1 |
| Beispiel 17 | | | | | | 4 | 3 | 2 | 1 | 0,5 |
| Beispiel 22 | 0,5 | 0,5 | 0,5 | | | | | | | |
| Beispiel 4 | | | | | | | | 0,5 | 0,5, | 0,5 |
| 4-Methylbenzylidene Camphor | 2 | | 3 | | 4 | | 3 | | 2 | |
| Butyl Methoxydibenzoylmethane | 1 | 3 | | 3 | 3 | | 3 | 3 | 3 | |
| Stearyl Alcohol (and) Steareth-7 (and) Steareth-10 | 3 | 3 | 3 | 3 | 3 | 3 | 3 | 3 | 3 | 3 |
| Glyceryl Stearate (and) Ceteth-20 | 3 | 3 | 3 | 3 | 3 | 3 | 3 | 3 | 3 | 3 |
| Glyceryl Stearate | 3 | 3 | 3 | 3 | 3 | 3 | 3 | 3 | 3 | 3 |
| Microwax | 1 | 1 | 1 | 1 | 1 | 1 | 1 | 1 | 1 | 1 |
| Cetearyl Octanoate | 11,5 | 11,5 | 11,5 | 11,5 | 11,5 | 11,5 | 11,5 | 11,5 | 11,5 | 11,5 |
| Caprylic/Capric Triglyceride | 6 | 6 | 6 | 6 | 6 | 6 | 6 | 6 | 6 | 6 |
| Oleyl Oleate | 6 | 6 | 6 | 6 | 6 | 6 | 6 | 6 | 6 | 6 |
| Propylene Glycol | 4 | 4 | 4 | 4 | 4 | 4 | 4 | 4 | 4 | 4 |
| Glyceryl Stearate SE | | | | | | | | | | |
| Stearic Acid | | | | | | | | | | |
| Persea Gratissima | | | | | | | | | | |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |
| Water | ad 100 | ad 100 | ad 100 | ad 100 | ad 100 | ad 100 | ad 100 | ad 100 | ad 100 | ad 100 |

### Fortsetzung

| | 2-11 | 2-12 | 2-13 | 2-14 | 2-15 | 2-16 | 2-17 | 2-18 |
|---|---|---|---|---|---|---|---|---|
| Titanium dioxide | 3 | | 2 | | | | 2 | 5 |
| Polysilicone 15 | | 1 | 0,5 | | | | | |
| Beispiel 4 | 0,5 | 1,5 | 0,5 | 1 | | | | |
| Beispiel 22 | | | | | 1 | 0,5 | 1,5 | 0,5 |
| Octocrylene | 5 | 5 | 5 | 5 | 5 | 5 | 5 | 5 |
| Beispiel 17 | 0,5 | 1,5 | 1 | | 3 | | 2 | |
| Zinc oxide | | | 2 | | | | | |
| 4-Methylbenzylidene Camphor | | | | 3 | | | | |
| Butyl Methoxydibenzoylmethane | | | | 2 | | | | |
| Phenylbenzimidazole Sulfonic Acid | | | | | 1 | | | |
| Stearyl Alcohol (and) Steareth-7 (and) Steareth-10 | 3 | 3 | 3 | 3 | | | | |
| Glyceryl Stearate (and) Ceteth-20 | 3 | 3 | 3 | 3 | | | | |
| Glyceryl Stearate | 3 | 3 | 3 | 3 | | | | |
| Microwax | 1 | 1 | 1 | 1 | | | | |
| Cetearyl Octanoate | 11,5 | 11,5 | 11,5 | 11,5 | | | | |
| Caprylic/Capric Triglyceride | 6 | 6 | 6 | 6 | 14 | 14 | 14 | 14 |
| Oleyl Oleate | 6 | 6 | 6 | 6 | | | | |
| Propylene Glycol | 4 | 4 | 4 | 4 | | | | |
| Glyceryl Stearate SE | | | | | 6 | 6 | 6 | 6 |
| Stearic Acid | | | | | 2 | 2 | 2 | 2 |
| Persea Gratissima | | | | | 8 | 8 | 8 | 8 |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| 1-methylhydantoine-2-imide | | | | | 3 | | | |
| Glycerin | | | | | 3 | 3 | 3 | 3 |
| Water | ad 100 | ad 100 | ad 100 | ad 100 | ad 100 | ad 100 | ad 100 | ad 100 |

## Patentansprüche

1. Verwendung von Verbindungen der Formel I 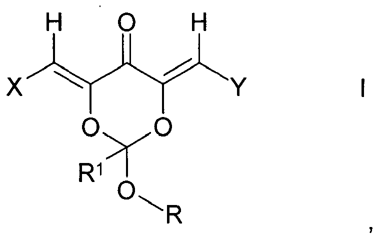 wobei
R eine geradkettige oder verzweigte Alkylgruppe mit 1 bis 20 C-Atomen bedeutet,
R¹ eine geradkettige oder verzweigte Alkylgruppe mit 1 bis 20 C-Atomen bedeutet,
X und Y jeweils unabhängig voneinander
eine unsubstituierte oder einfach- oder mehrfach durch R² substituierte Aryl- oder Heteroarylgruppe mit 5 bis 24 Ringatomen bedeuten oder eine Gruppe von unsubstituierten oder einfach oder mehrfach durch R² substituierten Aryl- und/oder Heteroarylgruppen mit 5 bis 24 Ringatomen bedeuten, wobei die Aryl- und/oder Heteroarylgruppen dieser Gruppe jeweils unabhängig voneinander einfach oder mehrfach durch eine Einfachbindung, eine Doppelbindung, konjugierte Doppelbindungen, ein C-Atom oder durch eine Einheit der Formel (CHR⁴)ₙ-(Het)ₒ-(CHR⁴)ₚ verknüpft sind,
R² jeweils unabhängig voneinander bei jedem Auftreten D, Hal, Alkyl, OH, O-Alkyl, O-Aryl, S-Alkyl, NH₂, NHAlkyl, N(Alkyl)₂, N(Aryl)₂, Cycloalkyl, O-Cycloalkyl, S-Cycloalkyl, NH-Cycloalkyl, N(Cycloalkyl)₂, CN, NO₂ Si(Alkyl)₃, B(OR³)₂, C(O)R³, P(O)(R³)₂, S(O)R³, S(O)₂R³, eine geradkettige oder verzweigte Alkenylgruppe mit 2 bis 20 C-Atomen und einer oder mehreren Doppelbindungen oder eine geradkettige oder verzweigte Alkinylgruppe mit 2 bis 20 C-Atomen und mindestens einer Dreifachbindung und gegebenenfalls einer oder mehrerer Doppelbindungen bedeutet,
R³ jeweils unabhängig voneinander H, D, OH, Alkyl, Aryl, Cycloalkyl, OAlkyl, OAryl oder O-Cycloalkyl bedeutet,
R⁴ jeweils unabhängig voneinander bei jedem Auftreten H, D, Hal, Alkyl, OH, O-Alkyl, O-Aryl, S-Alkyl, NH₂, NHAlkyl, N(Alkyl)₂, N(Aryl)₂, Cycloalkyl, O-Cycloalkyl, S-Cycloalkyl, NH-Cycloalkyl, N(Cycloalkyl)₂, CN, NO₂, Si(Alkyl)₃, B(OR³)₂, C(O)R³, C(O)₂R³, P(O)(R³)₂, S(O)R³, S(O)₂R³, eine geradkettige oder verzweigte Alkenylgruppe mit 2 bis 20 C-Atomen und einer oder mehreren Doppelbindungen oder eine geradkettige oder verzweigte Alkinylgruppe mit 2 bis 20 C-Atomen und mindestens einer Dreifachbindung und gegebenenfalls einer oder mehrerer Doppelbindungen bedeutet,
Alkyl eine geradkettige oder verzweigte Alkylgruppe mit 1 bis 20 C-Atomen bedeutet, die teilweise oder vollständig durch Halogen substituiert sein kann,
Cycloalkyl eine cyclische gesättigte oder teilweise ungesättigte Cycloalkylgruppe mit 3 bis 7 C-Atomen bedeutet,
Aryl eine Arylgruppe mit 6 bis 10 C-Atomen bedeutet, die einfach oder mehrfach durch Alkyl, OAlkyl, N(Alkyl)₂ oder Hal substituiert sein kann,
Hal F, Cl, Br oder I bedeutet,
Het O, S, -N=N-, NH oder NR² bedeutet,
n eine ganze Zahl von 0 bis 5 bedeutet,
o 0 oder 1 bedeutet,
p eine ganze Zanl von 0 bis 5 bedeutet,
n+o+p mindestens die Zahl 1 bedeutet
und deren Salze, Tautomere, Steroisomere, einschließlich deren Mischungen in allen Verhältnissen und/oder Solvate in einer elektronischen Vorrichtung, wobei die Verbindung 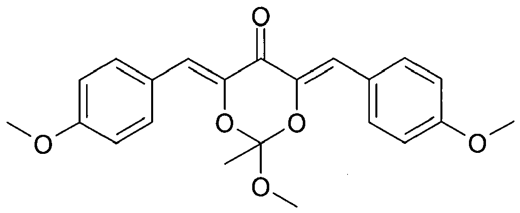 ausgeschlossen wird.

2. Verwendung von Verbindungen der Formel I nach Anspruch 1, **dadurch gekennzeichnet, dass** R¹ eine geradkettige oder verzweigte Alkylgruppe mit 1 bis 4 C-Atomen bedeutet.

3. Verwendung von Verbindungen der Formel I nach Anspruch 1 oder 2, **dadurch gekennzeichnet, dass** R eine geradkettige oder verzweigte Alkylgruppe mit 1 bis 8 C-Atomen bedeutet.

4. Verwendung von Verbindungen der Formel I nach einem oder mehreren der Ansprüche 1 bis 3, **dadurch gekennzeichnet, dass** X und Y jeweils unabhängig voneinander eine unsubstituierte oder einfach oder mehrfach durch R² substituierte Aryl- oder Heteroarylgruppe mit 5 bis 18 Ringatomen bedeuten.

5. Elektronische Vorrichtung enthaltend mindestens eine Verbindung der Formel I nach einem oder mehreren der Ansprüche 1 bis 4.

6. Elektronische Vorrichtung nach Anspruch 5, wobei es sich um eine organische Elektrolumineszenzvorrichtung, eine organisch integrierte Schaltung, einen organischen Feld-Effekt-Transistor, einen organischen Dünnfilmtransistor, einen organischen licht-emittierenden Transistor, eine organische Solarzelle, einen organischen optischen Dektektor, einen organischen Photorezeptor, ein organisches Feld-Quench-Device, eine licht-emittierende elektrochemische Zelle oder einer organischen Laserdiode handelt.

7. Organische Elektrolumineszenzvorrichtung nach Anspruch 6, **dadurch gekennzeichnet, dass** die mindestens eine Verbindung der Formel I nach einem oder mehreren der Ansprüche 1 bis 4 als fluoreszierender Emitter eingesetzt wird.

8. Verbindungen der Formel I 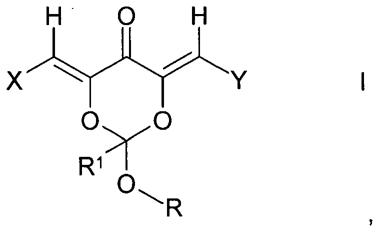 wobei
R eine geradkettige oder verzweigte Alkylgruppe mit 1 bis 20 C-Atomen bedeutet,
R¹ eine geradkettige oder verzweigte Alkylgruppe mit 1 bis 20 C-Atomen bedeutet,
X und Y jeweils unabhängig voneinander
eine unsubstituierte oder einfach- oder mehrfach durch R² substituierte Heteroarylgruppe mit 5 bis 24 Ringatomen ausgewählt aus 2- oder 3-Furyl, 2- oder 3-Thienyl, 1-, 2- oder 3-Pyrrolyl, 1-, 2-, 4- oder 5-Imidazolyl, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -4- oder -5-yl, 1- oder 5-Tetrazolyl, 1,2,3-Oxadiazol-4- oder -5-yl 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-1 H-Indolyl, 1-, 2-, 3-, 4-, 5-, 6- oder 7-2H-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 1-, 2-, 3-, 4-, 5-, 6-, 7- oder 8-Chinolinyl, 1-, 3-, 4-, 5-, 6-, 7- oder 8-Isochinolinyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolinyl, 2-, 4-, 5-, 6-, 7- oder 8-Chinazolinyl oder 1-, 2- oder 3-Pyrrolidinyl, 1-, 2-, 3-, 4-, 6-, 7-, 8- oder 9-Dibenzofuranyl, 1-, 2-, 3-, 4-, 6-, 7-, 8- oder 9-Dibenzothienyl, 1-, 2-, 3-, 5-, 6-, 7- oder 8-Indolizinyl, 1-, 2-, 3-, 4-, 6-, 7-, 8- oder 10-Phenanthridinyl7-1H-Indolyl, 1-, 2-, 3-, 4-, 6-, 7-, 8-, 9-, 10-, 11- oder 12-Benzophenanthridinyl oder 1-, 2-, 3-, 4-, 6-, 7-, 8-, 9-, 10-, 11- oder 12-Benzoacridinyl bedeuten
oder
eine Gruppe von unsubstituierten oder einfach oder mehrfach durch R² substituierten Aryl- und/oder Heteroarylgruppen mit 5 bis 24 Ringatomen bedeuten, ausgewählt aus Biphenyl, Terphenyl, Bipyridin, 9,9'-Spirobifluoren, 9,9-Diphenylfluoren, Diphenylether, Diphenylthioether, Stilben, 1,2-Diphenylethan, 1,1-Diphenylmethan, Biphenylen, Triphenylen oder den Strukturen 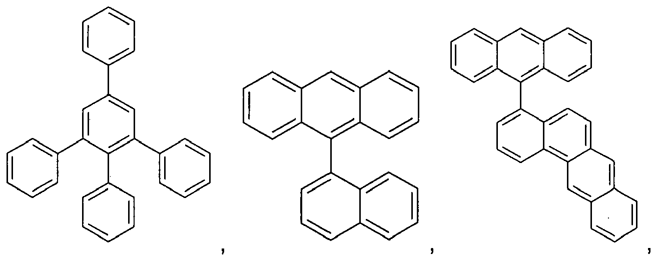 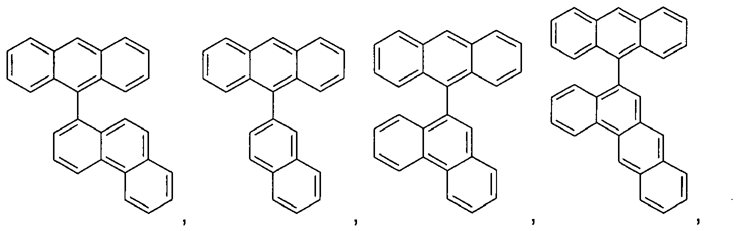 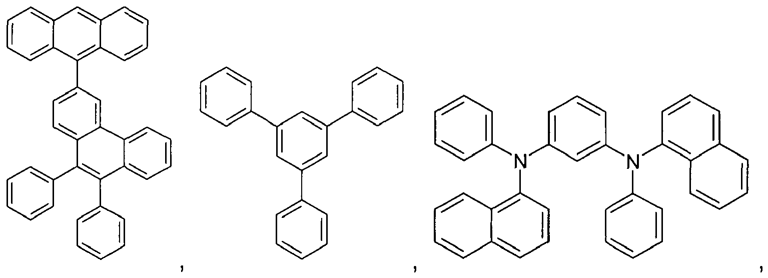 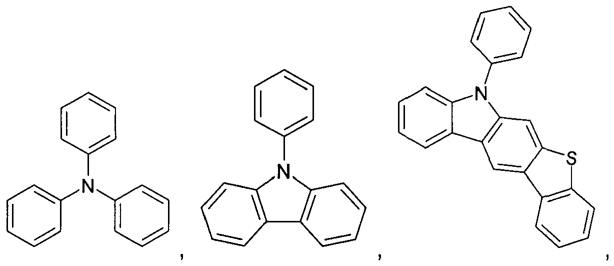 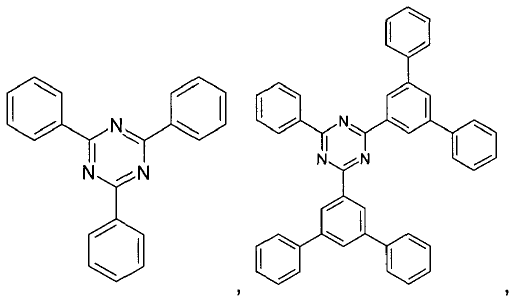 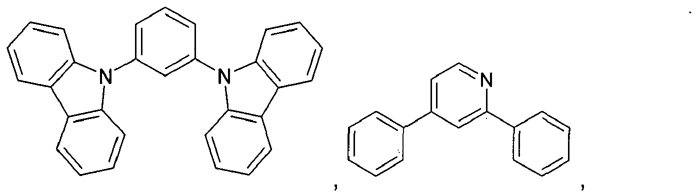 R² jeweils unabhängig voneinander bei jedem Auftreten D, Hal, Alkyl, OH, O-Alkyl, O-Aryl, S-Alkyl, NH₂, NHAlkyl, N(Alkyl)₂, N(Aryl)₂, Cycloalkyl, O-Cycloalkyl, S-Cycloalkyl, NH-Cycloalkyl, N(Cycloalkyl)₂, CN, NO₂, Si(Alkyl)₃, B(OR³)₂, C(O)R³, P(O)(R³)₂, S(O)R³, S(O)₂R³, eine geradkettige oder verzweigte Alkenylgruppe mit 2 bis 20 C-Atomen und einer oder mehreren Doppelbindungen oder eine geradkettige oder verzweigte Alkinylgruppe mit 2 bis 20 C-Atomen und mindestens einer Dreifachbindung und gegebenenfalls einer oder mehrerer Doppelbindungen bedeutet,
R³ jeweils unabhängig voneinander H, D, OH, Alkyl, Aryl, Cycloalkyl, OAlkyl, OAryl oder O-Cycloalkyl bedeutet,
Alkyl eine geradkettige oder verzweigte Alkylgruppe mit 1 bis 20 C-Atomen bedeutet, die teilweise oder vollständig durch Halogen substituiert sein kann,
Cycloalkyl eine cyclische gesättigte oder teilweise ungesättigte Cycloalkylgruppe mit 3 bis 7 C-Atomen bedeutet,
Aryl eine Arylgruppe mit 6 bis 10 C-Atomen bedeutet, die einfach oder mehrfach durch Alkyl, OAlkyl, N(Alkyl)₂ oder Hal substituiert sein kann,
Hal F, Cl, Br oder I bedeutet
und deren Salze, Tautomere, Steroisomere, einschließlich deren Mischungen in allen Verhältnissen und/oder Solvate.

9. Verfahren zur Herstellung von Verbindungen der Formel I nach Anspruch 8, **dadurch gekennzeichnet, dass** eine Verbindung der Formel II 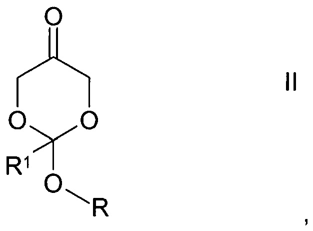 wobei R und R¹ eine Anspruch 8 angegebene Bedeutung hat, mit einer Verbindung der Formel IIIa und/oder IIIb 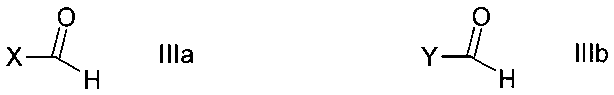 umgesetzt wird, wobei X und Y eine in Anspruch 8 angegebene Bedeutung haben.

10. Konjugierte, teilkonjugierte oder nicht-konjugierte Polymere, Oligomere oder Dendrimere, enthaltend eine oder mehrere Verbindungen der Formel I nach Anspruch 8, wobei die Verknüpfungsstelle zwischen der mindestens einen Verbindung der Formel I und dem Polymer, Oligomer oder Dendrimer an der Position des einen oder der mehreren Reste R² der Verbindung der Formel I liegt.

11. Zubereitung, enthaltend eine oder mehrere Verbindungen der Formel I nach Anspruch 8.

12. Zubereitung nach Anspruch 11 enthaltend mindestens ein weiteres organisch funktionelles Material ausgewählt aus der Gruppe der Host Materialien, Matrix Materialien, Elektronentransportmaterialien, Elektroneninjektionsmaterialien, Lochtransportmaterialien, Lochinjektionsmaterialien, Elektronenblockiermaterielien, Lochblockiermaterialien, Exzitonenblockiermaterialien und/oder Emitter.

13. Verwendung der Verbindungen der Formel I nach Anspruch 8 als Farbstoff.

## Claims

1. Use of compounds of the formula I 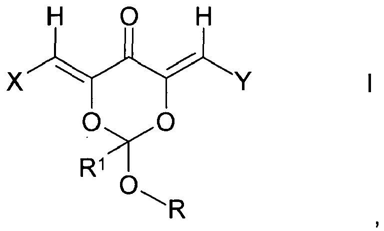 where
R denotes a straight-chain or branched alkyl group having 1 to 20 C atoms,
R¹ denotes a straight-chain or branched alkyl group having 1 to 20 C atoms,
X and Y each, independently of one another, denote an aryl or heteroaryl group having 5 to 24 ring atoms which is unsubstituted or mono- or polysubstituted by R², or a group of aryl and/or heteroaryl groups having 5 to 24 ring atoms which are unsubstituted or mono- or polysubstituted by R², where the aryl and/or heteroaryl groups in this group are each linked, independently of one another, singly or multiply, by a single bond, a double bond, conjugated double bonds, a C atom or by a unit of the formula (CHR⁴)ₙ-(Het)ₒ-(CHR⁴)ₚ,
R² in each case, independently of one another on each occurrence, denotes D, Hal, alkyl, OH, O-alkyl, O-aryl, S-alkyl, NH₂, NH-alkyl, N(alkyl)₂, N(aryl)₂, cycloalkyl, O-cycloalkyl, S-cycloalkyl, NH-cycloalkyl, N(cycloalkyl)₂, CN, NO₂, Si(alkyl)₃, B(OR³)₂, C(O)R³, P(O)(R³)₂, S(O)R³, S(O)₂R³, a straight-chain or branched alkenyl group having 2 to 20 C atoms and one or more double bonds or a straight-chain or branched alkynyl group having 2 to 20 C atoms and at least one triple bond and optionally one or more double bonds,
R³ in each case, independently of one another, denotes H, D, OH, alkyl, aryl, cycloalkyl, O-alkyl, O-aryl or O-cycloalkyl,
R⁴ in each case, independently of one another on each occurrence, denotes H, D, Hal, alkyl, OH, O-alkyl, O-aryl, S-alkyl, NH₂, NH-alkyl, N(alkyl)₂, N(aryl)₂, cycloalkyl, O-cycloalkyl, S-cycloalkyl, NH-cycloalkyl, N(cycloalkyl)₂, CN, NO₂ Si(alkyl)₃, B(OR³)₂, C(O)R³, C(O)₂R³, P(O)(R³)₂, S(O)R³, S(O)₂R³, a straight-chain or branched alkenyl group having 2 to 20 C atoms and one or more double bonds or a straight-chain or branched alkynyl group having 2 to 20 C atoms and at least one triple bond and optionally one or more double bonds,
alkyl denotes a straight-chain or branched alkyl group having 1 to 20 C atoms, which may be partially or fully substituted by halogen, cycloalkyl denotes a cyclic saturated or partially unsaturated cycloalkyl group having 3 to 7 C atoms,
aryl denotes an aryl group having 6 to 10 C atoms, which may be mono- or polysubstituted by alkyl, O-alkyl, N(alkyl)₂ or Hal,
Hal denotes F, Cl, Br or I,
Het denotes O, S, -N=N-, NH or NR²,
n denotes an integer from 0 to 5,
o denotes 0 or 1,
p denotes an integer from 0 to 5,
n+o+p denotes at least the number 1,
and salts, tautomers, stereoisomers thereof, including mixtures thereof in all ratios and/or solvates, in an electronic device, where the compound 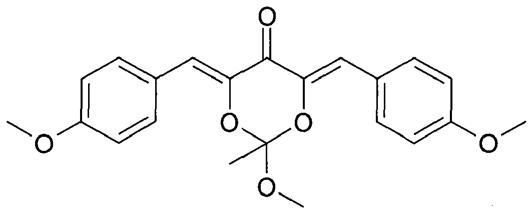 is excluded.

2. Use of compounds of the formula I according to Claim 1, **characterised in that** R¹ denotes a straight-chain or branched alkyl group having 1 to 4 C atoms.

3. Use of compounds of the formula I according to Claim 1 or 2, **characterised in that** R denotes a straight-chain or branched alkyl group having 1 to 8 C atoms.

4. Use of compounds of the formula I according to one or more of Claims 1 to 3, **characterised in that** X and Y each, independently of one another, denote an aryl or heteroaryl group having 5 to 18 ring atoms which is unsubstituted or mono- or polysubstituted by R².

5. Electronic device comprising at least one compound of the formula I according to one or more of Claims 1 to 4.

6. Electronic device according to Claim 5, which is an organic electroluminescent device, an organic integrated circuit, an organic field-effect transistor, an organic thin-film transistor, an organic light-emitting transistor, an organic solar cell, an organic optical detector, an organic photoreceptor, an organic field-quench device, a light-emitting electrochemical cell or an organic laser diode.

7. Organic electroluminescent device according to Claim 6, **characterised in that** the at least one compound of the formula I according to one or more of Claims 1 to 4 is employed as fluorescent emitter.

8. Compounds of the formula I 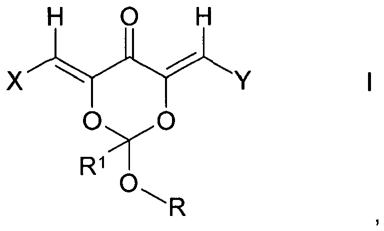 where
R denotes a straight-chain or branched alkyl group having 1 to 20 C atoms,
R¹ denotes a straight-chain or branched alkyl group having 1 to 20 C atoms,
X and Y each, independently of one another, denote a heteroaryl group having 5 to 24 ring atoms which is unsubstituted or mono- or polysubstituted by R², selected from 2- or 3-furyl, 2- or 3-thienyl, 1-, 2- or 3-pyrrolyl, 1-, 2-, 4- or 5-imidazolyl, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, furthermore preferably 1,2,3-triazol-1-, -4- or -5-yl, 1,2,4-triazol-1-, -4- or -5-yl, 1- or 5-tetrazolyl, 1,2,3-oxadiazol-4- or -5-yl, 1,2,4-oxadiazol-3- or -5-yl, 1,3,4-thiadiazol-2- or -5-yl, 1,2,4-thiadiazol-3- or -5-yl, 1,2,3-thiadiazol-4- or -5-yl, 2-, 3-, 4-, 5- or 6-2H-thiopyranyl, 2-, 3- or 4-4H-thiopyranyl, 3- or 4-pyridazinyl, pyrazinyl, 2-, 3-, 4-, 5-, 6- or 7-benzofuryl, 2-, 3-, 4-, 5-, 6- or 7-benzothienyl, 1-, 2-, 3-, 4-, 5-, 6- or 7-1 H-indolyl, 1-, 2-, 3-, 4-, 5-, 6- or 7-2H-indolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5-, 6- or 7-benzisoxazolyl, 2-, 4-, 5-, 6- or 7-benzothiazolyl, 2-, 4-, 5-, 6- or 7-benzisothiazolyl, 4-, 5-, 6- or 7-benz-2,1,3-oxadiazolyl, 1-, 2-, 3-, 4-, 5-, 6-, 7- or 8-quinolinyl, 1-, 3-, 4-, 5-, 6-, 7- or 8-isoquinolinyl, 1-, 2-, 3-, 4- or 9-carbazolyl, 1-, 2-, 3-, 4-, 5-, 6-, 7-, 8- or 9-acridinyl, 3-, 4-, 5-, 6-, 7- or 8-cinnolinyl, 2-, 4-, 5-, 6-, 7- or 8-quinazolinyl or 1-, 2- or 3-pyrrolidinyl, 1-, 2-, 3-, 4-, 6-, 7-, 8- or 9-dibenzofuranyl, 1-, 2-, 3-, 4-, 6-, 7-, 8- or 9-dibenzothienyl, 1-, 2-, 3-, 5-, 6-, 7- or 8-indolizinyl, 1-, 2-, 3-, 4-, 6-, 7-, 8- or 10-phenanthridinyl-7-1H-indolyl, 1-, 2-, 3-, 4-, 6-, 7-, 8-, 9-, 10-, 11- or 12-benzophenanthridinyl or 1-, 2-, 3-, 4-, 6-, 7-, 8-, 9-, 10-, 11- or 12-benzoacridinyl,
or
a group of aryl and/or heteroaryl groups having 5 to 24 ring atoms which are unsubstituted or mono- or polysubstituted by R², selected from biphenyl, terphenyl, bipyridine, 9,9'-spirobifluorene, 9,9-diphenyl-fluorene, diphenyl ether, diphenyl thioether, stilbene, 1,2-diphenylethane, 1,1-diphenylmethane, biphenylene, triphenylene or the structures 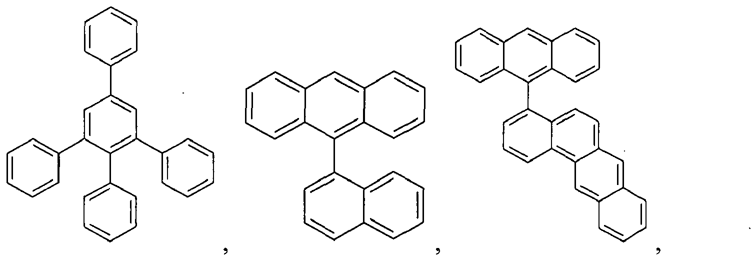 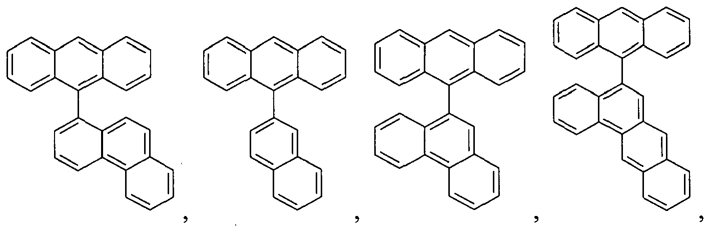 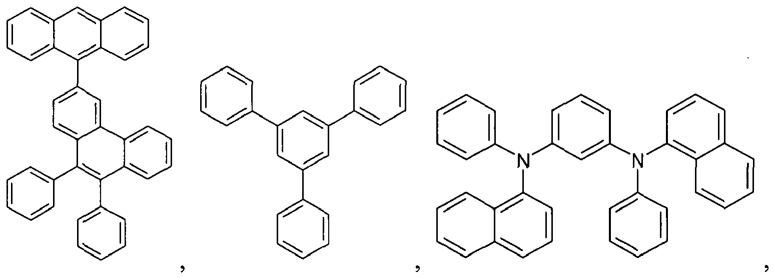 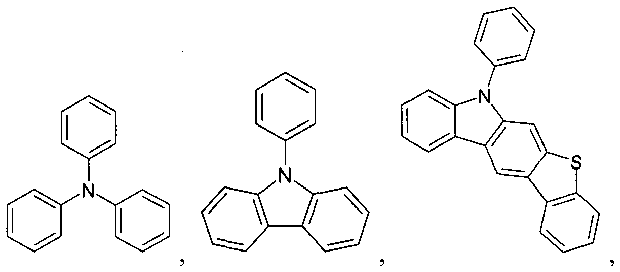 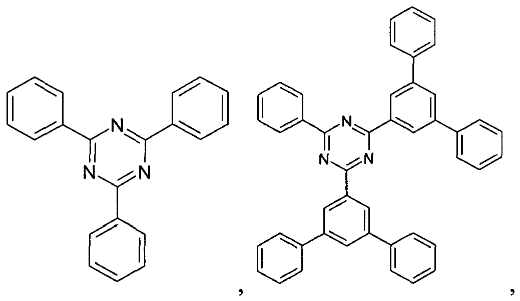 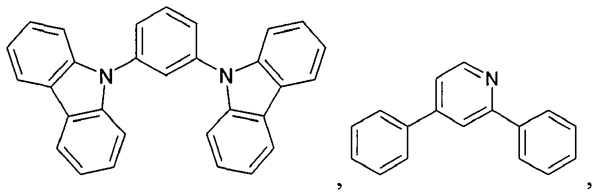
R² in each case, independently of one another on each occurrence, denotes D, Hal, alkyl, OH, O-alkyl, O-aryl, S-alkyl, NH₂, NH-alkyl, N(alkyl)₂, N(aryl)₂, cycloalkyl, O-cycloalkyl, S-cycloalkyl, NH-cycloalkyl, N(cycloalkyl)₂, CN, NO₂, Si(alkyl)₃, B(OR³)₂, C(O)R³, P(O)(R³)₂, S(O)R³, S(O)₂R³, a straight-chain or branched alkenyl group having 2 to 20 C atoms and one or more double bonds or a straight-chain or branched alkynyl group having 2 to 20 C atoms and at least one triple bond and optionally one or more double bonds,
R³ in each case, independently of one another, denotes H, D, OH, alkyl, aryl, cycloalkyl, O-alkyl, O-aryl or O-cycloalkyl,
alkyl denotes a straight-chain or branched alkyl group having 1 to 20 C atoms, which may be partially or fully substituted by halogen, cycloalkyl denotes a cyclic saturated or partially unsaturated cycloalkyl group having 3 to 7 C atoms,
aryl denotes an aryl group having 6 to 10 C atoms, which may be mono- or polysubstituted by alkyl, O-alkyl, N(alkyl)₂ or Hal,
Hal denotes F, Cl, Br or I,
and salts, tautomers, stereoisomers thereof, including mixtures thereof in all ratios and/or solvates.

9. Process for the preparation of compounds of the formula I according to
Claim 8, **characterised in that** a compound of the formula II 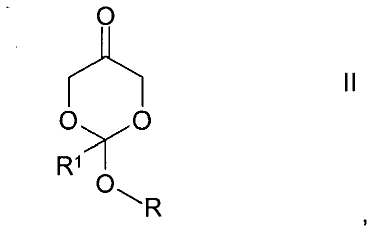 where R and R¹ have a meaning indicated in Claim 8, is reacted with a compound of the formula IIIa and/or IIIb 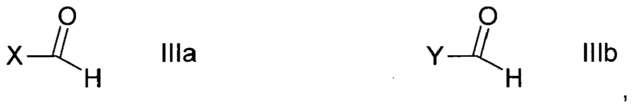 where X and Y have a meaning indicated in Claim 8.

10. Conjugated, partially conjugated or non-conjugated polymers, oligomers or dendrimers containing one or more compounds of the formula I according to Claim 8, where the linking site between the at least one compound of the formula I and the polymer, oligomer or dendrimer is at the position of the one or more radicals R² of the compound of the formula I.

11. Preparation comprising one or more compounds of the formula I according to Claim 8.

12. Preparation according to Claim 11 comprising at least one further organically functional material selected from the group of the host materials, matrix materials, electron-transport materials, electron-injection materials, hole-transport materials, hole-injection materials, electron-blocking materials, hole-blocking materials, exciton-blocking materials and/or emitters.

13. Use of the compounds of the formula I according to Claim 8 as dye.

## Revendications

1. Utilisation de composés de la formule I : 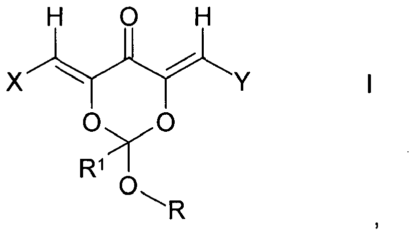 dans laquelle :
R représente un groupe alkyle en chaîne droite ou ramifié comportant de 1 à 20 atome(s) de C,
R¹ représente un groupe alkyle en chaîne droite ou ramifié comportant de 1 à 20 atome(s) de C,
X et Y, chacun indépendamment de l'autre, représentent :
un groupe aryle ou hétéroaryle comportant de 5 à 24 atomes de cycle, lequel est non substitué ou monosusbstitué ou polysubstitué par R², ou un groupe de groupes aryle et/ou hétéroaryle comportant de 5 à 24 atomes de cycle, lesquels sont non substitués ou monosubstitués ou polysubstitués par R², où les groupes aryle et/ou hétéroaryle dans ce groupe sont chacun liés, indépendamment les uns des autres, de manière unique ou multiple, par une liaison simple, une liaison double, des liaison doubles conjuguées, un atome de C ou par une unité de la formule (CHR⁴)ₙ-(Het)ₒ-(CHR⁴)ₚ,
R² dans chaque cas, indépendamment les uns des autres pour chaque occurrence, représente D, Hal, alkyle, OH, O-alkyle, O-aryle, S-alkyle, NH₂, NH-alkyle, N(alkyl)₂, N(aryl)₂, cycloalkyle, O-cycloalkyle, S-cycloalkyle, NH-cycloalkyle, N(cycloalkyl)₂, CN, NO₂, Si(alkyl)₃, B(OR³)₂, C(O)R³, P(O)(R³)₂, S(O)R³, S(O)₂R³, un groupe alkényle en chaîne droite ou ramifié comportant de 2 à 20 atomes de C et une ou plusieurs liaison(s) double(s) ou un groupe alkynyle en chaîne droite ou ramifié comportant de 2 à 20 atomes de C et au moins une liaison triple et en option, une ou plusieurs liaison(s) double(s),
R³ dans chaque cas indépendamment des autres, représente H, D, OH, alkyle, aryle, cycloalkyle, O-alkyle, O-aryle ou O-cycloalkyle,
R⁴ dans chaque cas indépendamment des autres pour chaque occurrence, représente H, D, Hal, alkyle, OH, O-alkyle, O-aryle, S-alkyle, NH₂, NH-alkyle, N(alkyl)₂, N(aryl)₂, cycloalkyle, O-cycloalkyle, S-cycloalkyle, NH-cycloalkyle, N(cycloalkyl)₂, CN, NO₂, Si(alkyl)₃, B(OR³)₂, C(O)R³, C(O)₂R³, P(O)(R³)₂, S(O)R³, S(O)₂R³, un groupe alkényle en chaîne droite ou ramifié comportant de 2 à 20 atomes de C et une ou plusieurs liaison(s) double(s) ou un groupe alkynyle en chaîne droite ou ramifié comportant de 2 à 20 atomes de C et au moins une liaison triple et en option, une ou plusieurs liaison(s) double(s), alkyl représente un groupe alkyle en chaîne droite ou ramifié comportant de 1 à 20 atome(s) de C, lequel peut être partiellement ou totalement substitué par halogène,
cycloalkyl représente un groupe cycloalkyle saturé ou partiellement non saturé cyclique comportant de 3 à 7 atomes de C,
aryl représente un groupe aryle comportant de 6 à 10 atomes de C, lequel peut être monosubstitué ou polysubstitué par alkyle, O-alkyle, N(alkyl)₂ ou Hal,
Hal représente F, CI, Br ou I,
Het représente O, S, -N=N-, NH ou NR²,
n représente un entier de 0 à 5,
o représente 0 ou 1,
p représente un entier de 0 à 5, n+o+p représente au moins le nombre 1,
et leurs sels, tautomères, stéréoisomères, y compris des mélanges afférents selon tous rapports et/ou leurs solvates, dans un dispositif électronique, où le composé 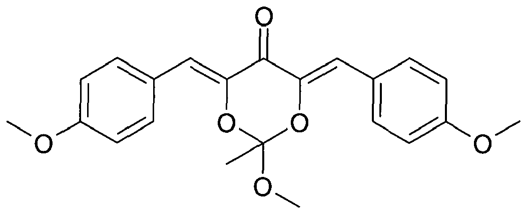 est exclu.

2. Utilisation de composés de la formule I selon la revendication 1, **caractérisée en ce que** R¹ représente un groupe alkyle en chaîne droite ou ramifié comportant de 1 à 4 atome(s) de C.

3. Utilisation de composés de la formule I selon la revendication 1 ou 2, **caractérisée en ce que** R représente un groupe alkyle en chaîne droite ou ramifié comportant de 1 à 8 atome(s) de C.

4. Utilisation de composés de la formule I selon une ou plusieurs des revendications 1 à 3, **caractérisée en ce que** X et Y, chacun indépendamment de l'autre, représentent un groupe aryle ou hétéroaryle comportant de 5 à 18 atomes de cycle, lequel est non substitué ou monosubstitué ou polysubstitué par R².

5. Dispositif électronique comprenant au moins un composé de la formule I selon une ou plusieurs des revendications 1 à 4.

6. Dispositif électronique selon la revendication 5, lequel est un dispositif électroluminescent organique, un circuit intégré organique, un transistor à effet de champ organique, un transistor à film mince organique, un transistor à émission de lumière organique, une cellule solaire organique, un détecteur optique organique, un photorécepteur organique, un dispositif à extinction de champ organique, une cellule électrochimique à émission de lumière organique ou une diode laser organique.

7. Dispositif électroluminescent organique selon la revendication 6, **caractérisé en ce que** l'au moins un composé de la formule I selon une ou plusieurs des revendications 1 à 4 est utilisé en tant qu'émetteur fluorescent.

8. Composés de la formule I : 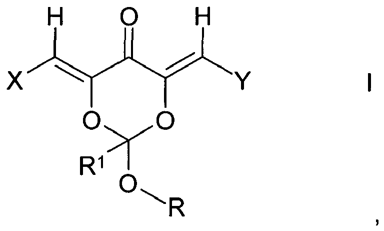 dans laquelle :
R représente un groupe alkyle en chaîne droite ou ramifié comportant de 1 à 20 atome(s) de C,
R¹ représente un groupe alkyle en chaîne droite ou ramifié comportant de 1 à 20 atome(s) de C,
X et Y, chacun indépendamment de l'autre, représentent :
un groupe hétéroaryle comportant de 5 à 24 atomes de cycle, lequel est non substitué ou monosubstitué ou polysubstitué par R², choisi parmi 2- ou 3-furyle, 2- ou 3-thiényle, 1-, 2- ou 3-pyrrolyle, 1-, 2-, 4- ou 5-imidazolyle, 3-, 4- ou 5-pyrazolyle, 2-, 4- ou 5-oxazolyle, 3-, 4- ou 5-isoxazolyle, 2-, 4- ou 5-thiazolyle, 3-, 4- ou 5-isothiazolyle, 2-, 3- ou 4-pyridyle, 2-, 4-, 5- ou 6-pyrimidinyle, en outre de façon préférable 1,2,3-triazol-1-, -4- ou -5-yle, 1,2,4-triazol-1-, -4- ou -5-yle, 1- ou 5-tétrazolyle, 1,2,3-oxadiazol-4- ou -5-yle, 1,2,4-oxadiazol-3- ou -5-yle, 1,3,4-thiadiazol-2- ou -5-yle, 1,2,4-thiadiazol-3- ou -5-yle, 1,2,3-thiadiazol-4- ou -5-yle, 2-, 3-, 4-, 5- ou 6-2H-thiopyranyle, 2-, 3- ou 4-4H-thiopyranyle, 3- ou 4-pyridazinyle, pyrazinyle, 2-, 3-, 4-, 5-, 6- ou 7-benzofuryle, 2-, 3-, 4-, 5-, 6- ou 7-benzothiényle, 1-, 2-, 3-, 4-, 5-, 6- ou 7-1 H-indolyle, 1-, 2-, 3-, 4-, 5-, 6- ou 7-2H-indolyle, 1-, 2-, 4- ou 5-benzimidazolyle, 1-, 3-, 4-, 5-, 6- ou 7-benzopyrazolyle, 2-, 4-, 5-, 6- ou 7-benzoxazolyle, 3-, 4-, 5-, 6- ou 7-benzisoxazolyle, 2-, 4-, 5-, 6- ou 7-benzothiazolyle, 2-, 4-, 5-, 6- ou 7-benzisothiazolyle, 4-, 5-, 6- ou 7-benz-2,1,3-oxadiazolyle, 1-, 2-, 3-, 4-, 5-, 6-, 7- ou 8-quinolinyle, 1-, 3-, 4-, 5-, 6-, 7- ou 8-isoquinolinyle, 1-, 2-, 3-, 4- ou 9-carbazolyle, 1-, 2-, 3-, 4-, 5-, 6-, 7-, 8- ou 9-acridinyle, 3-, 4-, 5-, 6-, 7- ou 8-cinnolinyle, 2-, 4-, 5-, 6-, 7- ou 8-quinazolinyle ou 1-, 2- ou 3-pyrrolidinyle, 1-, 2-, 3-, 4-, 6-, 7-, 8- ou 9-dibenzofuranyle, 1-, 2-, 3-, 4-, 6-, 7-, 8- ou 9-dibenzothiényle, 1-, 2-, 3-, 5-, 6-, 7- ou 8-indolizinyle, 1-, 2-, 3-, 4-, 6-, 7-, 8- ou 10-phénanthridinyl-7-1H-indolyle, 1-, 2-, 3-, 4-, 6-, 7-, 8-, 9-, 10-, 11- ou 12-benzophénanthridinyle ou 1-, 2-, 3-, 4-, 6-, 7-, 8-, 9-, 10-, 11- ou 12-benzoacridinyle,
ou
un groupe de groupes aryle et/ou hétéroaryle comportant de 5 à 24 atomes de cycle, lesquels sont non substitués ou monosubstitués ou polysubstitués par R², choisis parmi biphényle, terphényle, bipyridine, 9,9'-spirobifluorène, 9,9-diphénylfluorène, diphényléther, diphénylthio-éther, stilbène, 1,2-diphényléthane, 1,1-diphénylméthane, biphénylène, triphénylène ou les structures 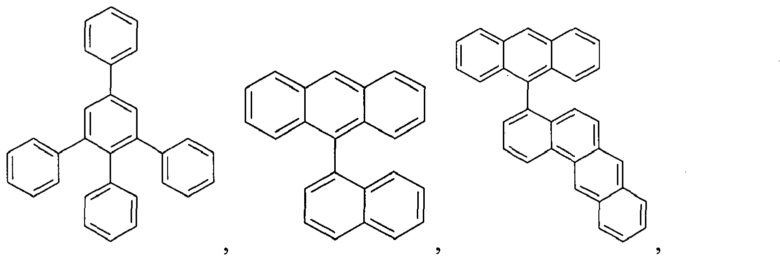 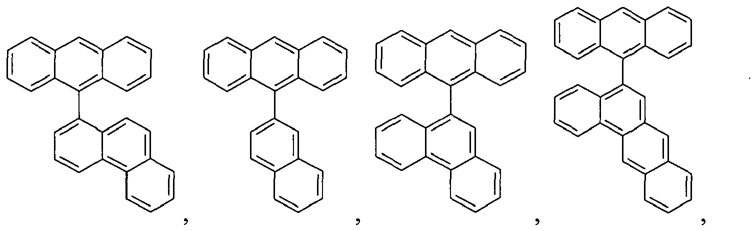 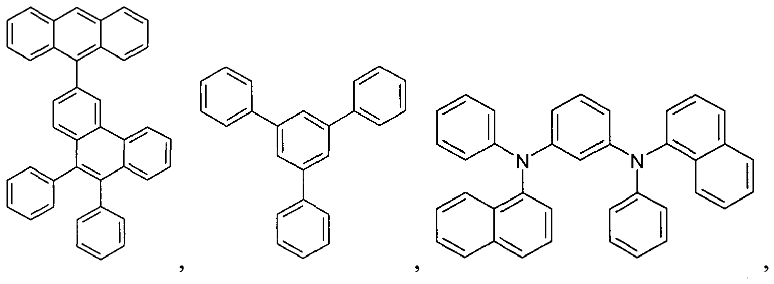 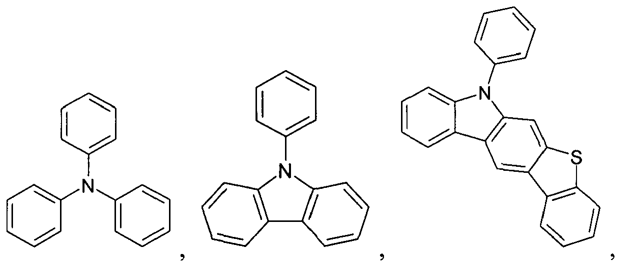 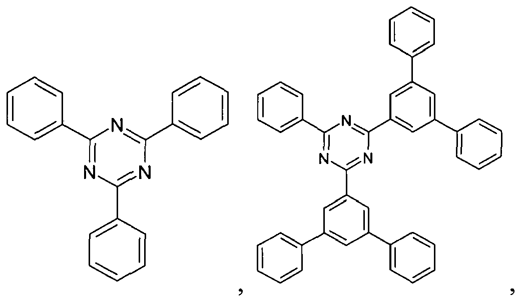 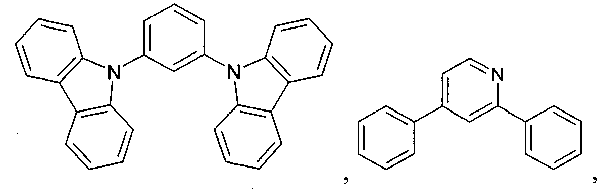
R² dans chaque cas, indépendamment les uns des autres pour chaque occurrence, représente D, Hal, alkyle, OH, O-alkyle, O-aryle, S-alkyle, NH₂, NH-alkyle, N(alkyl)₂, N(aryl)₂, cycloalkyle, O-cycloalkyle, S-cycloalkyle, NH-cycloalkyle, N(cycloalkyl)₂, CN, NO₂, Si(alkyl)₃, B(OR³)₂, C(O)R³, P(O)(R³)₂, S(O)R³, S(O)₂R³, un groupe alkényle en chaîne droite ou ramifié comportant de 2 à 20 atomes de C et une ou plusieurs liaison(s) double(s) ou un groupe alkynyle en chaîne droite ou ramifié comportant de 2 à 20 atomes de C et au moins une liaison triple et en option, une ou plusieurs liaison(s) double(s),
R³ dans chaque cas indépendamment les uns des autres, représente H, D, OH, alkyle, aryle, cycloalkyle, O-alkyle, O-aryle ou O-cycloalkyle, alkyl représente un groupe alkyle en chaîne droite ou ramifié comportant de 1 à 20 atome(s) de C, lequel peut être partiellement ou totalement substitué par halogène,
cycloalkyl représente un groupe cycloalkyle saturé ou partiellement non saturé cyclique comportant de 3 à 7 atomes de C,
aryl représente un groupe aryle comportant de 6 à 10 atomes de C, lequel peut être monosubstitué ou polysubstitué par alkyle, O-alkyle, N(alkyl)₂ ou Hal,
Hal représente F, CI, Br ou I,
et leurs sels, tautomères, stéréoisomères, y compris des mélanges afférents selon tous rapports et/ou leurs solvates.

9. Procédé pour la préparation de composés de la formule I selon la revendication 8, **caractérisé en ce qu'**un composé de la formule II : 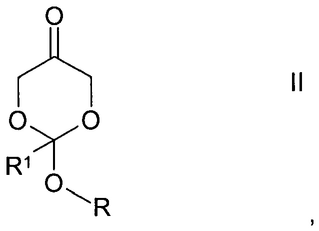 dans laquelle R et R¹ présentent une signification indiquée selon la revendication 8, est amené à réagir avec un composé de la formule IIIa et/ou IIIb : 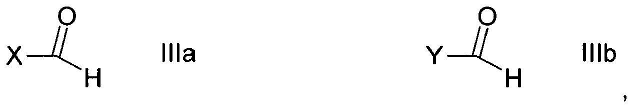 dans lesquelles X et Y présentent une signification indiquée selon la revendication 8.

10. Polymères, oligomères ou dendrimères conjugués, partiellement conjugués ou non conjugués contenant un ou plusieurs composé(s) de la formule I selon la revendication 8, où le site de liaison entre l'au moins un composé de la formule I et le polymère, l'oligomère ou le dendrimère est à la position des un ou plusieurs radical/radicaux R² du composé de la formule I.

11. Préparation comprenant un ou plusieurs composé(s) de la formule I selon la revendication 8.

12. Préparation selon la revendication 11 comprenant au moins un autre matériau organiquement fonctionnel choisi parmi le groupe des matériaux hôtes, des matériaux de matrice, des matériaux de transport d'électrons, des matériaux d'injection d'électrons, des matériaux de transport de trous, des matériaux d'injection de trous, des matériaux de blocage d'électrons, des matériaux de blocage de trous, des matériaux de blocage d'excitons et/ou des émetteurs.

13. Utilisation des composés de la formule I selon la revendication 8 en tant que colorant.
